# EUROPEAN PATENT APPLICATION

(11) **EP 2 105 511 A1**
(43) Date of publication of application: **30.09.2009**
(21) Application number: 08075243.9
(22) Date of filing: 28.03.2008
(51) Int. Cl.: C12Q 1/68, G01N 33/574, A61K 48/00, C12N 15/11, A61K 39/395

(54) **Medicament compositions and substances for treatment and identifying adenocarcinoma of the lung**

(71) Applicant: Fraunhofer-Gesellschaft zur Förderung der Angewandten Forschung e.V., 80686 München (DE)
(72) Inventor: Borlak, Jürgen, Prof. Dr., 31275 Lehrte/OT Immensen (DE); Meier, Tatiana, Dr., 30455 Hannover (DE); Halter, Roman, Dr., 30851 Langenhagen (DE)
(74) Representative: Baumbach, Friedrich

(57) **Abstract**

The invention is based on the surprising finding that in mammalian lungs c-myc acts as a molecular switch, specifically inducing an expression pattern *in vivo*, which results in prototypical mammalian adenocarcinoma of the lung and liver metastasis. A set of factors essential for the processes of tumorigenesis and tumor progression, i.e. cell cycle and apoptosis, cell growth, extracellular signaling, angiogenesis and invasion, is identified, whose expression is significantly changed. In particular, the expression pattern found uncovers the network of molecules leading to mammalian papillary adenocarcinomas of the lung.

The aim of the present invention is therefore to make available a medicament, compositions, and substances directed to novel c-myc modulated factors, and the use of said compositions and substances for labelling, identifying, and treating adenocarcinoma of the lung, in particular papillary adenocarcinoma of the lung.

The invention concerns a medicament for the treatment of lung carcinoma comprising a composition that decreases the expression or activity of a c-myc modulated gene selected from the group consisting of Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn
and/or
increases the expression or activity of a c-myc modulated gene selected from the group consisting of Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2, Satb1, Anp32a, Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1.

## Description

The invention relates to a medicament, compositions, and substances directed to novel c-myc modulated factors, and the use of said compositions and substances for labelling, identifying, and treating adenocarcinoma of the lung, in particular papillary adenocarcinoma of the lung. Areas of application are the life sciences: biology, biochemistry, biotechnology, medicine and medical technology.

The most common cause of cancer-related death in industrial countries is due to lung cancer. In the year 2000 the European mortality from lung cancer was estimated to be 347,000 cases (Tyczynski et al., 2003), and an approximately 158,000 deaths are predicted annually for the United States alone (Greenlee et al., 2001). In the majority of lung cancer cases (87%) tobacco smoke is the primary cause, but environmental (asbestos, radon) and genetic factors are additional causes for disease (Russo et al., 2005).

Lung carcinoma is a leading cause of cancer morbidity with tumor entities being classified as small-cell (SCLC) or non-small-cell lung carcinomas (NSCLC). In fact, NSCLC account for approx. 75% of all lung tumors (Batura-Gabryel and Foremskalciek, 2005) and are divided into adenocarcinomas, squamous cell and large-cell carcinomas. Notably, adenocarcinomas are on the raise and account for approximately 30-50% of all lung carcinomas (Charloux et al., 1997; Bhattacharjee et al., 2001). These tumors are derived from epithelium of the peripheral small airways. It is generally accepted that several genetic changes appear to be necessary for malignant transformation and these include, amongst others, overexpression of protooncogenes and loss of tumor-suppressor functions. However, despite intense research the molecular causes of lung adenocarcinoma are poorly understood.

Nonetheless, there is evidence for the c-Myc protooncogene to play a central role in various types of human tumors (Oster et al., 2002). Its overexpression was reported for lung carcinomas (Geng et al., 2003). The c-Myc protein is a key regulator of cell proliferation and cell fate decision. In normal cells its activity is tightly controlled by mitogens (Sears, 2004). By contrast, in tumor cells its expression is usually altered and no longer dependent on external signals. In fact, c-Myc exerts its biological effect through transcriptional activation of numerous genes through recognition of E-box sequences of targeted genes (Grandori and Eisenman, 1997). This transactivation ability of c-Myc is essential for its oncogenic capacity and dependent on a heterocomplex with the MAX protein. Transcriptional repression has also been observed in c-Myc transformed cells (Oster et al., 2002), by as yet unknown mechanism. The c-Myc transcription factor is one of the proto-oncogenes with a central role in pathogenesis of human tumors (Adhikary and Eilers, 2005). Its overexpression has been reported for various types of human and animal malignancies including lung cancer (Mitsuuchi and Testa, 2002). The incidence of lung carcinomas is high in humans (Travis et al., 1999). Notably, c-Myc is part of the normal cell proliferation machinery and regulates cell cycle, cell growth, apoptosis and differentiation in response to mitogenic stimuli through modulation of gene expression (Henriksson and Luscher, 1996). The precise mechanism of c-Myc-induced transformation capacity remain unresolved and require further investigation. Furthermore, the function and activity of c-Myc in normal cell biology and in neoplastic conditions may differ dramatically.

Notably, exaggerated expression of MYC as a result of undue gene amplification was observed in SCLCs and NSCLCs (Mitsuuchi and Testa, 2002). Moreover, altered c-Myc expression was detected in a variety of other human and animal tumors which included breast, colon, and cervical cancers in addition to osteosarcomas, glioblastomas, myeloid leukemias and lymphomas (Hurlin and Dezfouli, 2004; Oster et al., 2002; Spencer and Groudine, 1991). This suggests a central role of c-Myc in carcinogenesis. Specifically, c-Myc is an immediate early transcription factor and plays a key role in cell cycle regulation, apoptosis and differentiation. In normal cells c-Myc gene expression is tightly regulated and correlates closely with the proliferation status of a cell; it is undetectable in quiescent cells but gene transcription is rapidly induced upon mitogenic stimulation. This protein functions as a transcriptional activator through heterodimerization with Max and recognition of a specific hexanucleotide core sequences, termed E-box (CACGTG). Furthermore, c-Myc's ability to act as a negative regulator of transcription may be unrelated to E-box binding and transactivation function, possibly through dimerization with protein partner other than Max (e.g., Miz-1). Hence, c-Myc's biological effects may result in positive or negative regulation of gene transcription thereby inducing a cascade of direct and indirect transcriptional alterations (Zeller et al., 2003). Specifically, a number of c-Myc- regulated genes have been identified *in vitro,* in established cell lines or primary human cells with conditional c-Myc alleles (Marinkovic et al., 2004) as well as tumor cells expressing various c-Myc levels (Schuldiner and Benvenisty, 2001). Similarly, primary endothelial cells were investigated immediately after transfer of an ectopic c-Myc allele (Menssen and Hermeking, 2002). Previously, the development of a transgenic mouse model with targeted overexpression of c-Myc under the control of the SP-C (surfactant protein C) promoter has been reported to result in lung adenocarcinomas of mice (Ehrhardt et al., 2001). However, the molecular and cellular mechanisms of carcinogenesis induced by c-myc *in vivo* are poorly understood, so far. In this regard, the identification of downstream target genes, which distinguish between physiologic and tumorigenic functions of c-Myc *in vivo* would allow novel approaches for the treatment, diagnosis, and study of cancer.

The aim of the present invention is therefore to make available a medicament and medication, compositions, and substances directed to novel c-myc modulated factors, and the use of said compositions and substances for labelling, identifying, and treating adenocarcinoma of the lung, in particular papillary adenocarcinoma of the lung.

To this end, the implementation of the actions and embodiments as described in the claims provides appropriate means to fulfill these demands in a satisfying manner.

Thus, the invention in its different aspects and embodiments is implemented according to the claims.

The invention is based on the surprising finding that in mammalian lungs c-myc acts as a molecular switch, specifically inducing an expression pattern *in vivo,* which results in prototypical mammalian adenocarcinoma of the lung and liver metastasis. A set of factors essential for the processes of tumorigenesis and tumor progression, i.e. cell cycle and apoptosis, cell growth, extracellular signaling, angiogenesis and invasion, is identified, whose expression is significantly changed. In particular, the expression pattern found uncovers the network of molecules leading to mammalian papillary adenocarcinomas of the lung.

In one aspect, the invention is directed to the use of at least one biomarker selected from the group consisting of Satb1, Hspa9a, Hey1, Gas1, Bnip2,Capn2,Anp32a, Ddit3,Ccnb2, Cdkn2d (p19), Prc1, Uck2, Srm, Shmt1, Slc19a1, Npm1, Npm3, Nol5, Lamr1/Rpsa, Arhu(Rhou), Traf4, Adam19, Bmp6, Rbp1, Reck, Ect2
in the diagnosis of cancer and/or the prognosis of cancer and/or the treatment monitoring of cancer, in particular of lung cancer such as lung adenocarcinoma(s) may be.

For different purposes, it is preferred if the at least one biomarker is a combination of
(a) at least one biomarker selected from the group consisting of Satb1, Hspa9a, Hey1, Gas1, Bnip2,Capn2,Anp32a, Ddit3,Ccnb2, Cdkn2d (p19), Prc1, Uck2, Srm, Shmt1, Slc19a1, Npm1, Npm3, Nol5, Lamr1/Rpsa, Arhu(Rhou), Traf4, Adam19, Bmp6, Rbp1, Reck, Ect2, and
(b) the epithelial cell adhesion molecule (EpCAM).

Preferentially, a combination of Satb1 and at least one further biomarker selected from the group of Hspa9a, Hey1, Gas1, Bnip2,Capn2,Anp32a, Ddit3,Ccnb2, Cdkn2d (p19), Prc1, Uck2, Srm, Shmt1, Slc19a1, Npm1, Npm3, Nol5, Lamr1/Rpsa, Arhu(Rhou), Traf4, Adam19, Bmp6, Rbp1, Reck, Ect2, EpCAM is used as the at least one biomarker. Preferably, an appropriate amount of the at least one biomarker is used, in particular an amount for manufacturing a reference, more particular for manufacturing a reference comprising a reference level of said at least one biomarker, such as the level of said at least one biomarker in a sample of a normal healthy individual or the level of a said at least one biomarker in a sample of a patient suffering from cancer may be.

In particular, the at least one biomarker selected from the group consisting of Satb1, Hspa9a, Hey1, Gas1, Bnip2,Capn2,Anp32a, Ddit3,Ccnb2, Cdkn2d (p19), Prc1, Uck2, Srm, Shmt1, Slc19a1, Npm1, Npm3, Nol5, Lamr1/Rpsa, Arhu(Rhou), Traf4, Adam19, Bmp6, Rbp1, Reck, Ect2 , or the combination of (a) at least one biomarker selected from the group consisting of Satb1, Hspa9a, Hey1, Gas1, Bnip2,Capn2,Anp32a, Ddit3,Ccnb2, Cdkn2d (p19), Prc1, Uck2, Srm, Shmt1, Slc19a1, Npm1, Npm3, Nol5, Lamr1/Rpsa, Arhu(Rhou), Traf4, Adam19, Bmp6, Rbp1, Reck, Ect2 and (b) the epithelial cell adhesion molecule (EpCAM), is used for monitoring the therapeutic treatment of a patient, wherein the patient is preferably a human being or a transgenic c-myc mouse, suffering from lung cancer, in particular for monitoring the treatment of said patient with irinotecan, paclitaxel and/or 5-fluorouracil and/or the treatment with a drug binding to the epithelial cell adhesion molecule (EpCAM), such as an anti EpCAM antibody may be.

Preferably, at least one biomarker selected from the group consisting of Ccnb2, Slc19a1, Uck2, Srm1, Nol5a, Arhu, Adam19, Ect2, Shmt1 or from the group consisting of Gas1, Bmp6, Bnip2, Capn2, Ddit3 , Hey1, and wherein the at least one biomarker preferably further comprises EpCAM, is used, in particular for the diagnosis, prognosis and/or treatment monitoring of PLAC.

More preferably, at least one biomarker selected from the group consisting of Ccnb2, Slc19a1, Uck2, Srm1, Nol5a, Arhu, Adam19, Ect2, Shmt1 and at least one biomarker selected from the group consisiting of Gas1, Bmp6, Bnip2, Capn2, Ddit3 , Hey1 are used, and wherein said at least two biomarkers preferably further comprise EpCAM.

Another aspect of the invention is directed to a method for diagnosing cancer and/or prognosing cancer and/or staging cancer and/or monitoring the treatment of cancer, in particular lung cancer, such as lung adenocarcinoma(s) may be, comprising the steps of
(a) measuring the level of at least one biomarker selected from the group consisting of Satb1, Hspa9a, Hey1,Gas1, Bnip2,Capn2,Anp32a, Ddit3,Ccnb2, Cdkn2d (p19), Prc1, Uck2, Srm, Shmt1, SIc19a1, Npm1, Npm3, Nol5, Lamr1/Rpsa, Arhu(Rhou), Traf4, Adam19, Bmp6, Rbp1, Reck, Ect2, and wherein said at least one biomarker preferably further comprises EpCAM, in a patient or in a sample of a patient, wherein the patient is preferably a human being or a transgenic c-myc mouse, suffering from or being susceptible to cancer, and
(b) comparing the level of said at least one biomarker in said patient or in said sample to a reference level of said at least one biomarker, in particular by the use according to one of the claims 1-5.

Preferably, the method for diagnosing, prognosing and/or staging cancer and/or monitoring the treatment of cancer, is implemented for monitoring the therapeutic treatment of a patient suffering from lung cancer, in particular the treatment with irinotecan, paclitaxel and/or 5-fluorouracil and/or the treatment with a drug binding to the epithelial cell adhesion molecule (EpCAM), such as an anti EpCAM antibody may be.

In a particularly preferred embodiment, at least one biomarker is selected from the group consisting of Ccnb2, SIc19a1, Uck2, Srm1, Nol5a, Arhu, Adam19, Ect2, Shmt1 or from the group consisting of Gas1, Bmp6, Bnip2, Capn2, Ddit3 , Hey1, and wherein said at least one biomarker preferably further comprises EpCAM, in particular for the diagnosis, prognosis, staging and/or treatment monitoring of PLAC.

In particular, the method is preferably implemented to distinguish between different subtypes of lung cancer, such as (but not limited to) lung adenocarcinomas as defined by BAC or PLAC, wherein a significantly increased level of Ccnb2, SIc19a1, Uck2, Srm1, Nol5a, Arhu, Adam19, Ect2, Shmt1 in comparison with the level of a normal individual or a significantly decreased level of Gas1, Bmp6, Bnip2, Capn2, Ddit3 , Hey1 in comparison with the level of a normal individual is indicative of PLAC.

More particular, at least one biomarker selected from the group consisting of Ccnb2, SIc19a1, Uck2, Srm1, Nol5a, Arhu, Adam19, Ect2, Shmt1 and at least one biomarker selected from the group consisting of Gas1, Bmp6, Bnip2, Capn2, Ddit3 , Hey1 are measured to distinguish between different subtypes of lung cancer, such as (but not limited to) lung adenocarcinomas as defined by BAC or PLAC.

A significantly increased level of Ccnb2, Slc19a1, Uck2, Srm1, Nol5a, Arhu, Adam19, Ect2, Shmt1 in comparison with the level of a normal individual and a significantly decreased level of Gas1, Bmp6, Bnip2, Capn2, Ddit3, Hey1 in comparison with the level of a normal individual is then indicative of PLAC.

According to the invention, the medicament or medication for the treatment of lung carcinoma, in particular of papillary adenocarcinomas of the lung, comprises a pharmaceutically effective amount of a composition that decreases the expression or activity of a c-myc modulated gene selected from the group consisting of Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, lpo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn and/or
increases the expression or activity of a c-myc modulated gene selected from the group consisting of
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2, Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1.
Preferably, the medicament or medication further comprises a pharmaceutically acceptable carrier and/or recipient and/or diluent.

In particular, the medicament or medication for the treatment of lung carcinoma comprises a composition that increases and/or decreases the expression or activity a c-myc modulated gene in a mammal, preferably in a human being or a mouse, more preferably in a human being.

The genes according to the invention concern genes of mammalia, preferably genes of the genome of *mus musculus* or *homo sapiens,* in particular the respective genes of *homo sapiens* are preferred. Within the context of the invention, it is in particular preferred if the c-myc modulated gene is selected from the group of genes being grayscaled in the Tables 2, 4 and 6 or preferably Table 6, in particular according to claim 20 or preferably claim 9.

Preferably, the medicament or medication according to the invention comprises a pharmaceutically effective amount of a composition as detailed below.

Within the context of the present invention an antisense composition is provided, wherein the antisense composition comprises a nucleotide sequence complementary to a coding sequence of a c-myc modulated gene selected from the group consisting of Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Co115a1, Dsg2, Ect2, Lcn2, Kng, Hgfac, Adora2b, Spint1, Hpn.

In this regard, the term "coding sequence" is directed to the portion of an mRNA which actually codes for a protein. The term "nucleotide sequence complementary to a coding sequence" in particular is directed to an oligonucleotide compound, preferably RNA or DNA, more preferably DNA, which is complementary to a portion of an mRNA, and which hybridizes to and prevents translation of the mRNA. Preferably, the antisense DNA is complementary to the 5' regulatory sequence or the 5' portion of the coding sequence of said mRNA.

It is preferred that the antisense composition comprises a nucleotide sequence containing between 10-40 nucleotides , preferably 12 to 25 nucleotides, and having a base sequence effective to hybridize to a region of processed or preprocessed mammalian, preferably human or murine, more preferably human mRNA.

In particular, the composition comprises a nucleotide sequence effective to form a base-paired heteroduplex structure composed of mammalian, preferably human or murine, more preferably human RNA transcript and the oligonucleotide compound, whereby this structure is characterized by a Tm of dissociation of at least 45°C.

Further, a siRNA composition is provided, wherein the siRNA composition comprises an siRNA reducing or preferably inhibiting the expression of a c-myc modulated gene selected from the group consisting of Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, lpo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn.

The present invention employs siRNA for use in modulating the level of protein presence in the cell. SiRNA oligonucleotides directed to said genes specifically hybridize nucleic acids encoding the gene products of said genes and interfere with gene expression of said genes.

Preferably, the siRNA composition comprises siRNA (double stranded RNA) that corresponds to the nucleic acid ORF sequence of the gene product coded by one of said mammalian, preferably human or murine, more preferably human genes or a subsequence thereof; wherein the subsequence is 19, 20, 21, 22, 23, 24, or 25 contiguous RNA nucleotides in length and contains sequences that are complementary and non-complementary to at least a portion of the mRNA coding sequence.

The nucleotide sequences and siRNA according to the invention may be prepared by any standard method for producing a nucleotide sequence or siRNA, such as by recombinant methods, in particular synthetic nucleotide sequences and siRNA is preferred.

Furthermore, an antibody composition is provided, wherein the antibody composition comprises a pharmaceutically effective amount of an antibody or fragment thereof that binds to a polypeptide encoded by a gene selected from the group consisting of Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rp144, Eif2b, Tomm40, Slc15a2, Sic4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Co115a1, Dsg2, Ect2, Lcn2, Kng, Hgfac, Adora2b, Spint1, Adam19, Hpn.

Within the inventive context, antibodies are understood to include monoclonal antibodies and polyclonal antibodies and antibody fragments (e.g., Fab, and F(ab')₂) specific for one of said polypeptides. Polyclonal antibodies against selected antigens may be readily generated by one of ordinary skill in the art from a variety of warm-blooded animals such as horses, cows, various fowl, rabbits, mice, or rats.

Preferably, monoclonal antibodies are used in the antibody compositions of the invention which may be readily generated using conventional techniques (see Monoclonal Antibodies, Hybridomas: A New Dimension in Biological Analyses, Plenum Press, Kennett, McKearn, and Bechtol (eds.), 1980, and Antibodies: A Laboratory Manual, Harlow and Lane (eds.), Cold Spring Harbor Laboratory Press, 1988, which are incorporated herein by reference).

In particular, antibodies or fragments thereof specific for the gene products involved in extracellular signaling, angiogenesis and invasion, in particular specific for the gene products of Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Hpn, more particular for said gene products highlighted in Table 3, are preferred.

Further, a polypeptide composition (also termed as polypeptide composition (1)) is provided, wherein the polypeptide composition comprises a polypeptide coded by the sequence of a c-myc modulated gene selected from the group consisting of Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2, Satb1, Anp32a, Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1.

Preferably, the polypeptide composition comprises a peptide coded by the entire ORF/coding sequence of one of said genes. Such peptides include isolated polypeptides comprising an amino acid sequence which has at least 70% identity, preferably at least 80% identity, more preferably at least 90% identity, yet more preferably at least 95% identity, most preferably at least 97-99% identity, in particular 100 % identity, to the entire amino acid sequence of the gene product of one of said genes.

In particular, a polypeptide composition comprising a polypeptide involved in extracellular signaling, angiogenesis and invasion is preferred.

Polypeptides of the present invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

Furthermore, a vaccine composition is provided comprising a polypeptide coded by the sequence of a c-myc modulated gene selected from the group consisting of Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, lpo4, Mlp, Stk39, Rbp1, Reck, Areg, Ros1, Arhu, Frat2, Traf4, Myc, Frat2, Cldn2, Gjb3, Gja1, Krt1-18, Co115a1, Dsg2, Ect2, Lcn2, Kng, Hgfac, Adora2b, Spint1, Adam19, Hpn.

The vaccine composition for immunogenic and vaccine purposes in eliciting lung adenocarcinoma-specific immune responses preferably provides polypeptides comprising antigenic determinants from said gene products. An antigenic determinant of the gene product of one of said genes can be provided either by the full sequence of the gene product concerned, or by such subsequences as may be desired, e.g. a sequence fragment comprising at least 25%, e.g. at least 50% or 75% of the full sequence of the gene product concerned, e.g. a N-terminal or C-terminal sequence fragment.

In particular, a vaccine composition is preferred that comprises an antigenic determinant of a polypeptide involved in extracellular signaling, angiogenesis and invasion.

A further aspect of the invention concerns the use of a composition that decreases the expression or activity of a c-myc modulated gene selected from the group consisting of Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, lpo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn.
and/or
increases the expression or activity of a c-myc modulated gene selected from the group consisting of
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1
for the preparation of a medicament or medication, preferably for the preparation of a medicament or medication for preventing, treating, or ameliorating lung carcinoma, more preferably for preventing, treating, or ameliorating adenocarcinoma of the lung, in particular papillary adenocarcinomas of the lung.

In particular, an antisense composition, an siRNA composition, an antibody composition or the polypeptide composition (1) as detailed herein, or a combination thereof, is used for the preparation of the medicament or medication.

In one embodiment, a nucleotide composition (also termed as nucleotide composition (1)) is used for the preparation of said medicament or medication, wherein the nucleotide composition comprises a nucleotide sequence of a c-myc modulated gene selected from the group consisting of
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1.

The nucleotide composition particularly comprises a nucleic acid being from about 20 base pairs to about 100,000 base pairs in length, wherein the nucleotide sequence is included. Preferably the nucleic acid is from about 50 base pairs to about 50,000 base pairs in length. More preferably the nucleic acid is from about 50 base pairs to about 10,000 base pairs in length. Most preferred is a nucleic acid from about 50 pairs to about 4,000 base pairs in length. The nucleotide sequence can be a gene or gene fragment that encodes a protein, an oligopeptide or a peptide. Preferably, the nucleotide sequence of the present invention may comprise a DNA construct capable of generating a gene product coded by one of said genes and may further include an active constitutive or inducible promoter sequence.

In particular the nucleotide composition comprises a nucleotide sequence encoding a polypeptide which has at least 70% identity, preferably at least 80% identity, more preferably at least 90% identity, yet more preferably at least 95% identity, to the amino acid sequence of the gene product of one of said genes. In this regard, nucleotide sequences coding for polypeptides which have at least 97% identity are highly preferred, whilst those with at least 98-99% identity are more preferred, and those with at least 99% identity are most preferred. In particular, it is preferred if the nucleotide sequence encodes a polypeptide with 100 % identity to the entire amino acid sequence of the gene product of one of said genes.

In particular, the nucleotide composition comprises a DNA sequence that has at least 70% identity, preferably at least 80% identity, more preferably at least 90% identity, yet more preferably at least 95% identity, to the ORF (or coding sequence, respectively) of one of said genes over the entire coding region. In this regard, nucleoetide sequences which have at least 97% identity are highly preferred, whilst those with at least 98-99% identity are more highly preferred, and those with at least 99% identity are most highly preferred. In particular, it is preferred if the nucleotide sequence encodes a DNA sequence that has 100 % identity to the entire ORF of one of said genes over the entire coding region.

The nucleotide composition may further comprise an enhancer element and/or a promoter located 5' to and controlling the expression of said therapeutic nucleotide sequence or gene. The promoter is a DNA segment that contains a DNA sequence that controls the expression of a gene located 3' or downstream of the promoter. The promoter is the DNA sequence to which RNA polymerase specifically binds and initiates RNA synthesis (transcription) of that gene, typically located 3' of the promoter.

Preferably, a pharmaceutically effective amount of one of the compositions specified above or a pharmaceutically effective amount of a combination thereof is used, in particular furthermore a pharmaceutically acceptable carrier and/or recipient and/or diluent is used for the preparation of the medicament or medication.

In a further aspect of the invention, an immunogenically effective amount of the vaccine composition as detailed herein is used for the preparation of a vaccine, wherein preferably furthermore an adjuvant is used. In particular, it is preferred if the vaccine composition and the adjuvant are admixed with a pharmaceutically effective vehicle (excipient).

Yet another aspect of the invention concerns the use of novel c-myc modulated genes and/or gene products of thereof to screen for and to identify drugs against lung carcinoma, preferably against adenocarcinoma of the lung, more preferably against papillary adenocarcinoma of the lung, wherein at least one or more genes and/or one or more gene products of thereof selected from the group of Prc1, Kit4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn, Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2, Satb1, Anp32a, Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1
is used.

In one embodiment, a nucleotide composition (termed as nucleotide composition (2), respectively) is used as the one or more genes, wherein the nucleotide composition comprises a nucleotide sequence of a c-myc modulated gene selected from the group consisting of Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Hpn.

Said nucleotide composition particularly comprises a nucleic acid being from about 20 base pairs to about 100,000 base pairs in length, wherein the nucleotide sequence is included. Preferably the nucleic acid is from about 50 base pairs to about 50,000 base pairs in length. More preferably the nucleic acid is from about 50 base pairs to about 10,000 base pairs in length. Most preferred is a nucleic acid from about 50 pairs to about 4,000 base pairs in length. The nucleotide sequence can be a gene or gene fragment that encodes a protein, an oligopeptide or a peptide. Preferably, the nucleotide sequence of the present invention may comprise a DNA construct capable of generating a gene product coded by one of said genes and may further include an active constitutive or inducible promoter sequence.

Preferably, this nucleotide composition comprises a nucleotide sequence encoding a polypeptide which has at least 70% identity, preferably at least 80% identity, more preferably at least 90% identity, yet more preferably at least 95% identity, to the amino acid sequence of the gene product of one of said genes. In this regard, nucleotide sequences coding for polypeptides which have at least 97% identity are highly preferred, whilst those with at least 98-99% identity are more preferred, and those with at least 99% identity are most preferred. In particular, it is preferred if the nucleotide sequence encodes a polypeptide with 100 % identity to the entire amino acid sequence of the gene product of one of said genes.

In particular, the nucleotide composition (2) comprises a DNA sequence that has at least 70% identity, preferably at least 80% identity, more preferably at least 90% identity, yet more preferably at least 95% identity, to the ORF (or coding sequence, respectively) of one of said genes over the entire coding region. In this regard, nucleoetide sequences which have at least 97% identity are highly preferred, whilst those with at least 98-99% identity are more highly preferred, and those with at least 99% identity are most highly preferred. In particular, it is preferred if the nucleotide sequence encodes a DNA sequence that has 100 % identity to the entire ORF of one of said genes over the entire coding region.

In another embodiment, a polypeptide composition (also termed as polypeptide composition (2)) is used as the one or more gene products, wherein the polypeptide composition comprises a polypeptide coded by the sequence of a c-myc modulated gene selected from the group consisting of Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Co115a1, Dsg2, Ect2, Lcn2, Kng, Hgfac, Adora2b, Spint1, Adam19, Hpn.

Such peptides include isolated polypeptides comprising an amino acid sequence which has at least 70% identity, preferably at least 80% identity, more preferably at least 90% identity, yet more preferably at least 95% identity, most preferably at least 97-99% identity, to the entire amino acid sequence of the gene product of one of said genes. Preferably, the polypeptide composition comprises a peptide coded by the entire ORF/coding sequence of one of said genes. In particular, a polypeptide composition comprising a polypeptide involved in extracellular signaling, angiogenesis and invasion, as coded by Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn is preferred.

Preferably, one or more of said genes and/or their gene products, in particular the nucleotide composition (2) and/or the polypeptide composition (2), is incubated with a compound to be tested and changes in the expression of said genes and/or derived sequences and/or the function of said gene products are determined. In particular, the antisense composition, the siRNA composition, the antibody composition, the nucleotide composition (1) or the polypeptide composition (1), as detailed herein, or a combination thereof, is used as test compound.

More preferably, said use for screening and identifying drugs comprises the steps of: (1) contacting a test cell expressing, preferably overexpressing, at least one of said genes with a test compound; (2) detecting the expression level of said gene; and (3) determining the compound that suppresses said expression level compared to a normal control level of said gene as an inhibitor of said gene.

In particular, a compound that enhances the expression or activity of one of said genes is identified by the inventive use comprising the steps of : (1) contacting a test cell expressing, in particular overexpressing, said non-small cell lung cancer-associated gene with a test compound; (2) detecting the expression level of said non-small cell lung cancer-associated gene; and (3) determining the compound that increases said expression level compared to a normal control level of said gene as an enhancer of said non-small cell lung cancer-associated gene.

Within this context, it is particularly preferred that the use comprises the steps of : (1) contacting a test compound with a polypeptide encoded by the selected gene; (2) detecting the binding activity between the polypeptide and the test compound; and (3) selecting a compound that binds to the polypeptide.

More specifically, said use comprises the steps of (a) contacting a test compound with a polypeptide encoded by the selected gene;
(b) detecting the biological activity of the polypeptide of step (a); and (c) selecting a compound that suppresses the biological activity of the polypeptide encoded by the gene selected from the group consisting of Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Sicl5a2, Sic4a7, Slc4a4, Rangnrf, Kpnb3, lpo4, Mlp, Stk39, Rbp1, Reck, Areg, Ros1, Arhu, Frat2, Traf4, Myc, Frat2, Cldn2, Gjb3, Gja1, Krt1-18, Co115a1, Dsg2, Ect2, Lcn2, Kng, Hgfac, Adora2b, Spint1, Hpn.
in comparison with the biological activity detected in the absence of the test compound, and/or enhances the biological activity of the polypeptide encoded by the polynucleotide selected from the group consisting of
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1,
in comparison with the biological activity detected in the absence of the test compound. Advantegeously, cell proliferation is detected as biological activity, or any other biological activity of the cell related to carcinogenesis or tumorigenesis, in particular the presence of tumor markers known in the art, is detected.

In a further preferred embodiment the use comprises the steps of : (1) contacting a test compound with a cell into which a vector comprising the transcriptional regulatory region of one or more of the selected genes and a reporter gene that is expressed under the control of the transcriptional regulatory region has been introduced; (2) measuring the activity of said reporter gene; and (3) selecting a compound that reduces the expression level of said reporter gene when the selected gene is an up-regulated gene selected from the group consisting of
Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, lpo4, Mlp, Stk39,
Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn,
and/or that enhances the expression level of said reporter gene when the selected gene is a down-regulated gene selected from the group consisting of
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1, as compared to a control.

More particular, the use comprises drugs, in particular a test compound and/or a medicament or medication as specified above, wherein the drugs regulate the expression of one or more of said genes and/or the function of one or more of said gene products and/or their derived molecules, and said drugs are used for the production of means for treating, identifying or labeling adenocarcinoma, in particular papillary adenocarcinoma, of the lung.

In one embodiment of the use for screening and identifying drugs DNA and/or related molecules encoding one or more of said gene products and/or derived structures are used, and/or one or more polypeptides, peptides and/or derived molecules having the function of one or more of said gene products, are used.

Yet another aspect of the invention concerns a procedure for identifying, labelling and/or treating lung carcinoma, in particular adencarcinoma of the lung, more particular papillary adenocarcinoma of the lung, wherein a biological and/or biotechnological system is contacted with a soluble substance, such as an oligonucleotide sequence or antibody may be, having affinity with at least one of the genes selected from the group of
Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, lpo4, Mlp, Stk39,
Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn,
and/or mRNA encoded thereby and/or their gene products and/or parts thereof and wherein the soluble substance is linked with a marker.

Preferably, the biological or biotechnological system is an organism, a tissue, a cell, a part of a cell, a DNA, a RNA, a cDNA, a mRNA, a cRNA, a protein and/or a peptide and/or a derived structure and/or contains the same. In particular, the biological system is a cell derived from a tumor or of a tumor metastasis, preferably a cell derived from a lung tumor or of a liver metastasis thereof or the biotechnological system is an oligonucleotide library derived thereof.

Particularily, the biological or biotechnological system employed for the procedure comprises cells of a tumor malignancy of the lung and/or an oligonucleotide library and/or a protein library and/or a peptide library derived thereof, such as a transgenic animal, in particular a c-myc mouse as described herein, or biological material received thereof or biological material received from a human lung cancer patient may be.

As the, at least partially, soluble substance in particular oligonucleotides, proteins, peptides or structures derived of thereof are suitable. These have the advantage that they can recognize specifically two or three-dimensional target structures on the molecular level. Beyond that, they provide the favourable characteristic that the recognition usually takes place in aqueous physiologically buffered solutions and leads to a specific association/binding with the targeted structure.

In one embodiment of the procedure, a nucleotide sequence according to the antisense composition, an siRNA according to the siRNA composition, an antibody according to the antibody composition, a nucleotide sequence according to the nucleotide composition (1) or a polypeptide according to the polypeptide composition (1), as described herein, or a combination thereof, is used as the soluble substance. In particular, complementary oligonucleotides, such as a primer or primer pairs described herein, is used as the substance having specific affinity.

Thereby, monoclonal and/or polyclonal antibodies and/or antibody fragments are particularly suitable, since they are formed as stable highly specific structures, which are, in principle, producible against all possible molecular target structures. In a favourable embodiment of the procedure human and/or bispecific antibodies or human and/or bispecific antibody fragments are used.

In particular, monoclonal antibodies and/or antibody fragments are thereby suitable.

For implementing the invention it is preferred, if the marker according to invention is selected as an element, an isotope, a molecule and/or an ion or is composed of thereof, such as a dye, contrast means, chemotherapeutic agent, radionuclide, toxin, lipid, carbohydrate, biotin, peptid, protein, microparticle, vesicle, polymer, hydrogel, cellular organelle, virus and/or whole cell, in particular if the marker is formed as dye labeled and/or enzyme-labeled secondary antibodies and/or as protein A and/or as protein G or structures derived of thereof.

The linkage between the marker and the substance is favourably chemically, electrostatically and/or over via hydrophobic interactions, such as there is sufficient connection stability for the use of the marked substance for identifying, labelling and treating of metastatic cells, preferably if the linkage is covalent.

For the increase of the sensitivity of the procedure according to invention also the simultaneous use of several substances is in particular suitable, in particular if they comprise two or more substances having each affinity with one of the targets or their mRNA sequences or their gene products.

In particular, for a specific recognition/identification substances are preferred, which bind with an affinity above the association constant Ka = 1000 M-1 to the target structure.

Preferably, the procedure according to the invention comprises contacting the biological or biotechnological system with a plurality of said soluble substances.

For implementing the procedure according to the invention it is favourable to use one of the following methods - PCR, in vitro translation, RT-PCR, gel electrophoresis, Western Blot, Northern Blot, Southern Blot, ELISA, FACS measurement, chromatographic isolation, UV microscopy, immunohistochemistry, screening of solid phase bound molecules or tissues and/or biosensory investigation - whereby by amplification, isolation, immobilization and/or detection and/or by combinations of thereof a particularly simple conversion of the procedure according to invention is made possible for the examined sample, in particular if furthermore a statistic analysis is accomplished.

The procedure according to invention is preferably implemented by using molecules, cells and/or tissue, in particular being immobilized or synthesized on a planar surface, e.g. spatially addressed. on a nitrocellulose or PVDF membrane, or is linked to the cavity of a microtiter/ELISA plate or on the bottom of a cell culture container, in particular on a glass or plastic chip (biochip).

Favourably, as solid phase bound molecules, substances are used, having affinity for at least one, in particular two, preferably three, more preferably four, most preferably five of the selected target genes and/or their variants and/or parts thereof and/or their mRNA and/or their gene products and/or cleavage products derived thereof, polypeptides or peptides. The indentification of the target structures, e.g. of factors in an immobilized section of tissue or of cells of a cell culture, can take place thereby with arbitrarily marked molecules, which are brought on the surface in solution, e.g. (fluorescence marked/labeled) antibodies.

If a RT-PCR is accomplished, then favourably appropriate oligonucleotide probes and/or primers are used. For implementing the procedure according to invention immobilized molecule libraries, e.g. DNA or antibody libraries are used, which associate with the target structure(s) in solution, e.g. with a cDNA library, a PCR product library or with cells of a secondary tumor.

Substances bound to the molecule libraries are particularly identified by the use of appropriately marked probes, e.g. dye labeled oligonucleotides. If unabeled primary antibodies are used, preferably labeled secondary antibodies are used for detection. Furthermore, the use of other proteins, e.g. enzymes, or streptavidin or parts of thereof is suitable. For the diagnosis of a tumor, in particular by *in vitro* and/or *in vivo* diagnostics, with the help of the procedure according to invention preferably optically (or radiographically) sensitive equipment, is used, e.g. an UV microscope, scanner or ELISA reader, photometer, or szintigrafic equipment, e.g. X-ray gadget are appropriate.

A further aspect of the invention concerns the procedure according to the invention for diagnozing lung cancer, in particular adenocarcinoma of the lung, more particular papillary adenocarcinoma of the lung, or a predisposition to developing lung cancer in a subject, comprising determining an expression level of a c-myc modulated gene selected from the group consisting of
Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39,
Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn, in a biological sample derived from the subject, wherein an increase of said level compared to a normal control level of said gene indicates that said subject suffers from or is at risk of developing lung cancer, in particular if said increase is at least 200% greater than said normal control level, and whereby preferably said level is determined for a plurality of said genes.

Preferably, this procedure further comprises determining an expression level of a c-myc modulated gene selected from the group consisting of
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1,
in a biological sample derived from the subject, and wherein a decrease in said level compared to a normal control level indicates said subject suffers from or is at risk of developing non-small cell lung cancer, in particular if said decrease is at least 200% lower than said normal control level, and whereby preferably said level is determined for a plurality of said genes.

In practice, the procedure according to the invention preferably comprises any one of the methods: detecting the mRNA of the selected genes, detecting the protein encoded by the selected gene or detecting the biological activity of the protein encoded by the selected gene.

In one embodiment of the procedure it is preferred if the hybridization of a selected gene probe to a gene transcript of a biological sample is determined, in particular if a primer or primer pair, as described herein, is used as gene probe and/or if the hybridization step is carried out on a DNA array.

Another aspect of the invention concerns a method appropriate for the use and/or the procedure according to the invention, in particular for identifying adenocarcinoma of the lung, comprising the steps of
- isolating a biological sample from biological material received from an organism, in particular from a human patient, suffering from lung cancer or from an organism to be tested for its susceptibility to lung cancer, and
- determining the level of
   Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn
   and/or
   Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
   Satb1, Anp32a,
   Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1.
   or of fragments of thereof or of a selection of thereof in the biological sample by screening the presence of said proteins or of fragments of thereof or of mRNA coding for the same.

In one embodiment it is preferred, if the method comprises the steps of
- isolating a biological sample from biological material received from a healthy organism, in particular from a human being and pairwisely comparing the gene expression profiles determined for the isolated biological samples by correlating the levels measured; and/or
- a standard is used for determining the changes of expression, in particular the level of gene expression of at least one of the genes to be screened derived from a gene expression profile for a healthy tissue of the organ, wherein the tumor is formed, is used.

In particular, it is preferred, if
- the isolated biological sample is a tissue, a cell, a cellular compartment, total RNA, total protein or a body fluid; and/or
- the standard comprises values for the expression levels of the targeted genes and/or values for β-Actin, in particular being determined parallely to the gene expression profile (2); and/or
- the levels of expression of the at least two genes coding for the factors screened are normalized with respect to the levels of expression of the respective genes in healthy tissue wherein the tumor is formed, in particular by using the standard for normalizing.

Moreover, the gene expression profiling preferably comprises the steps of
- synthesizing a cDNA library derived of the isolated RNA by RT-PCR,
- synthesizing a cRNA library, derived of the cDNA library by second strand cDNA synthesis and *in vitro* transcription of the double stranded cDNA,
- producing a RNA fragment library derived of the cRNA library by hydrolytic cleavage into RNA fragments,
- performing a hybridization assay by incubating an oligonucleotide array including spatially addressed solid phase bound oligonucleotide sequences coding for the at least two oligonucleotide sequences to be screened or for parts of thereof or for sequences being complementary of the same, with the cRNA fragment library,
- scanning the hybridization pattern of the oligonucleotide array.

Particularily, the use of labelled ribonucleotides, such as biotin labelled ribonucleotides may be, is preferred for the *in vitro* transcription or the method wherein antibodies specific for one or more of said targets are used..

In particular, it is preferred according to the invention, if microarrays are used for performing the hybridization assays.

Yet another preferable aspect of the invention concerns a testkit for identifying and/or determining mammalian, in particular human and/or murine, preferably human, tumor malignancies, in particular adenocarcinoma of the lung, more particular papillary adenocarcinoma of the lung, comprising a soluble substance as specified above, in particular comprising two or more detection reagents which bind to one or more genes selected from the group consisting of
Prc1, Klt4, Ect2, Cdc20, Stk6, Nek6, Ect2, Birc5, Hspa9a, Cideb Pglyrp, Zfp239, Elf5, Uck2, Smarcc1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39,
Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn,
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1,
and/or mRNA of thereof and/or polypeptides encoded thereby, for a fast and easy implementation of the invention.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

To investigate the consequences of oncogenic activation of c-Myc in lung adenocarcinomas the surfactant protein C (SPC)- promoter was used to target c-Myc expression to alveolar epithelium (Ehrhardt et al., 2001). The SPC/c-Myc-transgenic mice are indeed useful to study early events in lung carcinogenesis and to identify molecular mechanism of c-Myc-induced transformation in vivo. Gene expression profiling of well-defined lung adenocarcinomas in this transgenic disease model was performed as to identify the genetic program induced by c-Myc over expression in lung epithelial cells leading to neoplasia. Therefore, genome wide expression signatures of solid lung tumors were compared with normal non-transgenic lung tissues as controls. 162 genes were found up regulated and 305 down regulated which were further analyzed with regard to their biological functions, known responsiveness to c-Myc and the presence of c-Myc -binding sites in their promoters (putative c-Myc targets). The study of coded biological functions of either induced or repressed genes in c-Myc - induced lung adenocarcinoma evidenced a broad spectrum of pivotal metabolic and signaling pathways to be regulated in the tumors.

In Part 1 and Part 2 on the study c-Myc regulated genes linked to cell cycle promotion and stimulation of cell growth and incapacitating of the intrinsic programms for cell cycle arrest / cell death were reported.

It was first aimed to identify new c-Myc target genes and report the genome-wide response to c-Myc overexpression associated with growth of lung adenocarcinoma. For this purpose the Affymetrix platform was used to identify genes regulated in solid lung tumors of c-Myc transgenic mice and compared findings with lung tissue from non-transgenic healthy animals. To the best of our knowledge this is the first report on a genome-wide search of new c-Myc target genes in malignantly transformed respiratory epithelium. Overall, identifying the genetic events associated with c-Myc-transforming capacity was aimed. In this first part of the investigation the regulation of genes involved in promotion of cell cycle progression and inhibition of cell death is reported. It is proposed that their regulation is responsible, at least in part, for exaggerated cell proliferation in papillary lung adenocarcinomas induced by overexpressed c-Myc as reported herein.

In the second part of the study on c-Myc induced lung cancer it was aimed at identifying genes which are regulated by c-Myc in vivo and play a specific role in tumor growth. By use of the surfactant protein C promoter it was possible to selectively target alveolar epithelium of mice and to induce nearly 50-fold c-Myc transcriptional activation. This led to malignant cell transformation resulting in invasive PLACs as reported in the first part of the study. Based on oncogenomic profiling more than 400 genes were differently expressed in lung tumors and associated with diverse cellular processes. While in the first part transcriptional events linked to activation of cell cycle and suppression of cell death were examined, it was now aimed at identifying genes coding for proteins in cell growth. It is of considerable importance that some of the genes identified in c-Myc-induced lung adenocarcinomas were shown previously to be c- Myc responsive or interact directly with the c-Myc protein (c-Myc gene targets). These genes are disease candidate genes and are most likely selectively regulated by c-Myc in tumorigenesis. Furthermore, bioinformatics were applied to search for c-Myc binding sites in the promoters of regulated genes and identified novel c-Myc candidate targets linked to lung carcinogenesis worthy for exploration to combat this highly malignant disease. Overall, the second part of the oncogenomics study on c-Myc induced lung adenocarcinoma, as described herein, focused on tumor growth by investigating regulation of gene networks linked to DNA and RNA metabolism and processing, ribosomal biosynthesis and protein biosynthesis, nuclear and cellular transport, glycolysis, lipid metabolism and chromatin remodeling.

In the third part the efforts were focused onto regulation of genes whose expression was altered in c-Myc-induced lung tumors and may lead to of modulation of extracellular signaling in favour of cell proliferation, rearrangement of cytoskeleton, changes in adhesion and acquirement of invasive and angiogenic capacities.

### Histopathology of lung tumors.

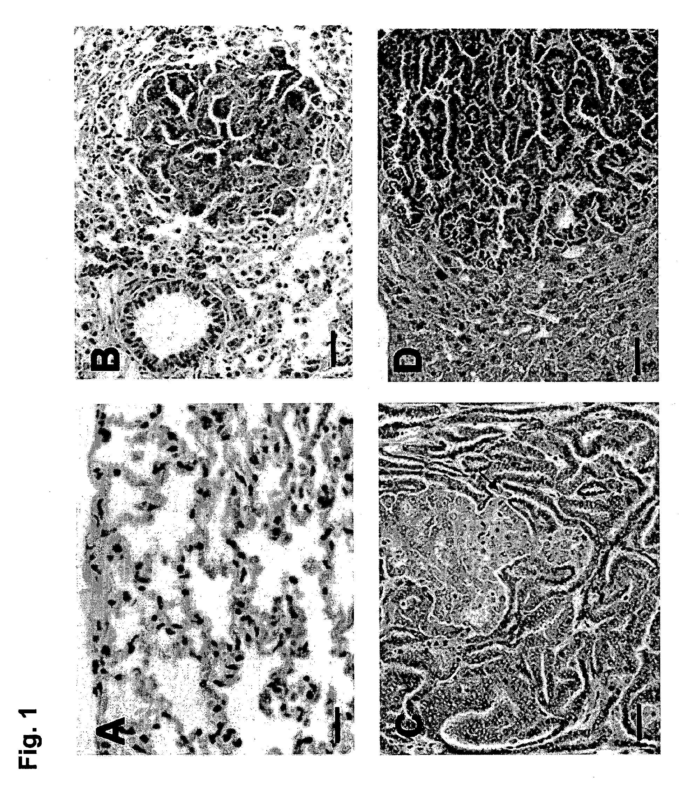

Lung specimens of non-transgenic and transgenic mice were examined at the age of 9-13 months. Mostly a medium sized or large solid tumor nodule was found in transgenic lung and such nodule may filled an entire lobe, while the remaining parenchyma appeared to be macroscopically unaffected. Histologically a ubiquitous proliferation of the peripheral pulmonary epithelium was evident, and the solid tumors were only part of it (Fig. 1 band 1 c). The alveoli and parts of the terminal bronchioli were lined by hyperchromatic low columnar cells, exhibiting classic lepidic growth pattern. These cells were dispersed throughout the whole lung and eventually formed a confluent monolayer in the alveoli. The lesional cells were all quite similar and showed only slight nuclear polymorphism, but severe nuclear atypia, as defined by vesicular enlargement and prominent nucleoli. Mitosis was repeatedly seen, e.g. at least 1 mitosis/ high power field (=HPF) in confluent lesions. There was lepidic growth and severe cellular atypia typical for bronchiolar alveolar carcinoma (BAC). Unlike BAC the macrosopically visible solid tumors were invasive growing carcinomas, all invariably with papillary growth pattern. While initial papillary lung adenocarcinomas (PLAC) were minimal invasive (lesions), advanced PLAC developed the whole range of malignancy as gross bronchial and vascular invasion and finally liver metastasis (Fig. 1d). A lung lobe might contain up to five tumor foci. By extrapolation one might expect up to 25 multicentric invasive tumors for an entire lung. Note, solid invasive tumors, i.e. PLACs, were taken for gene expression analysis.

### Genome wide transcript profiling

To better understand the transforming capacity of over expressed c- Myc, gene expression profiles in c-Myc-induced PLACS of transgenic mice with histologically confirmed normal lung of non transgenic animals were compared. To identify key transcriptional events associated with malignant growth RNA was prepared from tumor sets of small, middle and large size (see Methods) and analyzed by oligonucleotide microarrays.

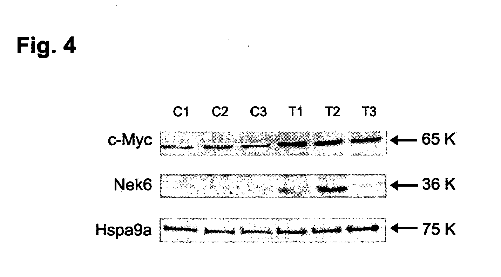

Specifically, c-Myc transcript level was more than 50 fold induced in tumor tissues of transgenic mice when compared with lung tissues of normal non-transgenic mice Induction of c-Myc protein expression was confirmed by Western blot analysis (Fig. 4). The expression of c-Myc heterodimeric partner Max was, however, only slightly increased (FC 1,21-1,55 in various tumor samples). The gene coding for Mad4, which competes with c-Myc for Max binding to represses c-Myc activity was expressed in all lung tissues at the same level. Furthermore, Miz-1, which is one of the possible mediator of c-Myc induced gene (Oster et al., 2002), was abundantly expressed in all lung tissues without differences in expression between tumor and control samples. Thus, c-Myc partners were not substantially regulated in response to c-Myc over expression.

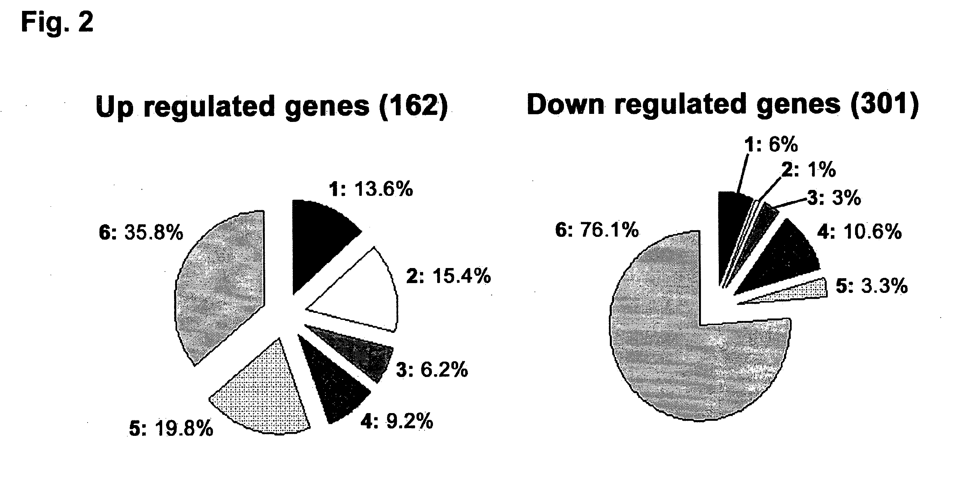

Statistical analysis of replicates with T-test and more stringent comparative ranking was performed to identify significantly induced and repressed genes in tumor sets in comparison with non transgenic lungs (see Methods). Array analyses yielded 162 genes (not functionally characterized ESTs were not taken into consideration) with significantly increased expression levels (mean FC> 3, p-value in T-test < 0,05 and 100% of "Increase" calls in comparative ranking analyses in one or more sets of tumors - see Methods for more details) and about twice as many genes (301) were identified with repressed expression (FC<- 3, p-value in T-test < 0,05 and 100% "Decrease" accordingly) in c-Myc-induced lung tumors (Table 1). Then, genes differently expressed in lung tumors with published data using the Myc Target Gene database were compared (www.myc-cancer-gene.org). This database provides valuable information on Myc-responsive genes for various cell types and species (Zeller et al., 2003; Zeller et al., 2003; Zeller et al., 2003). Known c-Myc-responsive genes were identified, i.e. genes whose expression was changed in response to c-Myc activation as well as known c-Myc targets, e.g. genes known to bind c-Myc directly. The results are included in Table 1 and Table 2. From 162 up regulated genes in tumors 29% proved to be previously reported as c-Myc-targets (designated as "T" in the Table 2) or their relatives ("rT"), i.e. these belong to gene families, which include known c-Myc-target (Fig. 2). As expected, many of these genes coded for proteins involved in cell cycle regulation (Cdk4, Ccnb1, Cks2) and apoptosis (Hspa9a, Trp53). Additional 14,8% were known c-Myc responsive genes ("R") or their relatives ("rR") which included, amongst others, Tfdp1 and cdc2a, involved in cell cycle regulation (Table 2a). Among the 301 repressed genes identified in tumors it only 7% were found to be known c-Myc- targets or their relatives and additional 13,6 % reported as c-Myc responsive genes or the relatives (Table 1). Thus, much more up regulated genes found in the study, have been previously reported to interact directly with c-Myc when compared with repressed genes. About half of the genes with increased expression in papillary lung adenocarcinomas (56,2%) and the majority of down regulated genes (79,4%) were not previously described as c-Myc-responsive. Thus, by use of the genetic disease model it was possible to identify novel c-Myc-responsive genes and these included over expressed Stk6, Nek6, Prc1, Ect2, Birc5 and the repressed Cdkn2d, Lats2, Bnip2, Hey1, all of which are highly interesting disease candidate genes for their essential role in the regulation of cell cycle and apoptosis. Some genes known as c-Myc responsive genes (cEBPalpa, cyclinD1 etc.) were altered in lung adenocarcinomas in opposite direction when compared with previously reported studies which points to cell-type specific responses to c-Myc. These genes are annotated in the Table 2a,b as *cursive.*

To identify genes which may be directly regulated by c- Myc a computational tool was applied to search for potential c-Myc-binding sites in promoter regions of differently regulated genes. A specific set of mononunucleotide weight matrices with E-box as a core sequence available in TRANSFAC® professional database (see Methods) was applied. The results of this search are included in Table 2a,b and summarized in Table 1 and Fig. 2. With the stringent cut-offs used much more potential c-Myc binding sites in promoters of genes with enhanced expression were found compared with repressed genes (Table 1), with the highest score in the known c-Myc targets (e.g. Cdk4, Kif11, Hspa9a, Fasn, Shtm1, Srm, Apex1 and others). A number of new putative gene targets containing c-Myc-binding sites in their promoters like Prc1, Elf5, Klf7 and others (Table 2a) was also identified Notable, in lung adenocarcinomas repression of several transcription factors was detected, some of them carrying potential c-Myc-binding sites in their promoters, e.g. Foxf1, Tbx3, KLf7 (Table 2b).

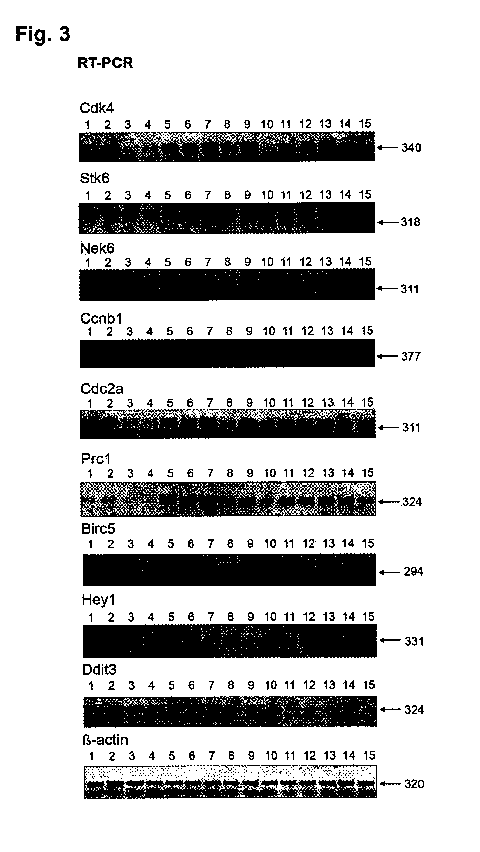

The functional analysis of regulated genes in c-Myc induced lung adenocarcinomas indicated that they code for proteins involved in a broad spectrum of cell metabolic and signalling pathways. In the first part of the study genes were described which are associated with cell cycle regulation and intracellular control for cell division as well as those coding for transcription factors. The genes are compiled in Table 2a,b and known c-Myc responsive genes and c-Myc targets are indicated (see above for definition). In addition the number of identified c-Myc-binding sites in promoter regions is also included. Additionally, RT-PCR was performed for 9 genes involved in cell cycle regulation and apoptosis to verify their regulation in tumors by an alternative method The changes in gene expression found in lung tumors by both methods were in good agreement (Fig. 3, Table 3). Moreover, Western blot analysis for two selected genes Nek6 and Hspa9a (Hsp70) confirmed their over expression in tumors at the protein level (Fig. 4).

No qualitative differences in gene expression profiles in relation to tumor size were observed. This agrees with the histological examination where all tumors were classified as papillary adenocarcinomas of various sizes. However, some quantitative differences (up to 2-3 fold) in expression level of several genes between small and large tumors were observed. Among those genes whose expression vel was substantially higher in small size tumors were members involved in cell cycle regulation and included the proto-oncogene ect2. Evidently, this underlines their important role in the onset of tumor growth. Furthermore, the expression of some genes was not detected in tumor samples while their expression was abundant in non-transgenic healthy control lungs (highlighted by grey row accordingly in Table 2b). These included, among others, pro-apoptotic Ddit3 and transcription factor Nr2f1 which exerts anti-AP-1 activity and is a mediator of anti-cancer effect of retinoic acid (Lin et al., 2002).

Thus, in this first study on the oncogenomics of c-Myc-induced lung tumours activation of Myc-target genes was identified greatly contributing to the promotion of cell cycle as well as activation of genes involved in inhibition of apoptosis, therefore, providing a selective advantage for tumor growth, as discussed below

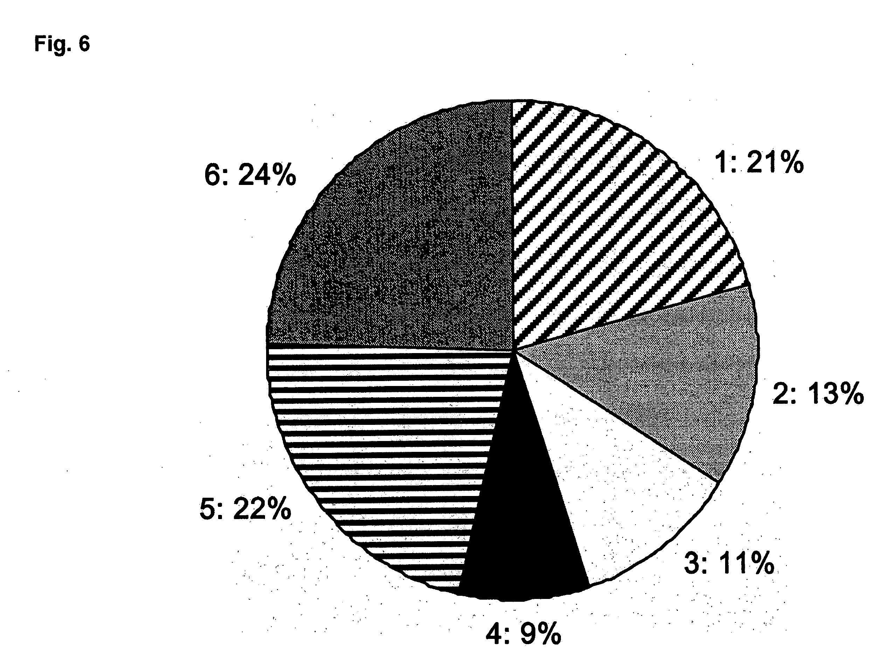

Genome-wide expression of transcripts in solid tumors induced by targeted c-Myc-expression in alveolar epithelial cells was studied. Note, these tumors were classified as papillary lung adenocarcinomas. To follow the dynamic changes in the gene expression during carcinogenesis, sets of small-, middle- and large-sized tumors were examined separately. By use of the mouse whole genome microarrays it was possible to investigate expression of more than 12480 genes in c-Myc-induced lung tumors and compared findings with normal non-transgenic lungs. Stringent criteria were applied to determine significantly regulated genes in tumors (see "Material and Methods"). As shown in Table 4, many of these genes coded for cell metabolism, DNA and RNA synthesis, ribosomal biogenesis, protein synthesis and transport. Additionally, regulated genes were subjected to bioinformatic analysis and for comparison with findings deposited in a publicly available database termed the Myc Target Gene (www.myc-cancer-gene.org). The results are included in the Table 4 and summarized in Fig. 6. Remarkably, about one third of the genes involved in meolism and growth proved to be known as c-Myc-targets (designated as "T" in the Table 4) or their relatives ("rT"), i.e. belong to gene families, whose members have been shown in the past to bind c-Myc directly. Amongst them thymidilate synthase, thymidine kinase, fatty acid synthase, lactate dehydrogenase1A, spermidine synthase, nucleolin, nucleophosmin, polyA binding protein 4, several ribosomal proteins, eukaryotic translation initiation factor 3, chaperonin subunit 5, solute carrier family 19a1 and others were identified. Furthermore, 20% of regulated genes were known c-Myc responsive genes ("R") or their relatives ("rR"). Examples include hexokinase 2, Gpi1, helicase, RNA polymerase 1-3, replication factor C (activator1) 4, Nol5a, Impdh2 and Gart (Table 4). Since the induction of these genes had been repeatedly reported for different tumor cell lines in response to c-Myc activation, they can be considered as likely candidate genes in c-Myc-induced carcinogenesis. It is of considerable importance that the genetic disease model, as described herein, enabled identification of novel c- Myc-responsive genes involved in cell growth and proliferation. For instance, arginase1, ribonucleotide reductase M1 and M2, uridine-cytidine kinase2, topoisomerase (DNA) 2a, meiotic recombination 11 a, nucleoplasmin 3, nucleolar proteins and importin4 are novel c-Myc responsive genes whose altered expression is linked to lung adenocarcinomas. Remarkably, several genes which are involved in regulation of transcription through chromatin remodeling were detected. Specifically, up regulated helicase, Smarcc1, histone H1fX, IaminB1 as well as the repressed histone1, H2bc and negative regulators of histone acetylation Satb1 and Anp32a were observed. Additionally, in tumors significant increase of the SNRP transcript was identified, which belongs to the dsRNA binding protein family, involved in translation, RNA editing and stability. These findings suggest that chromatin remodeling and posttranscriptional RNA-editing may be additionally mechanisms by which c-Myc augment expression of numerous responsive genes. A further search for c-Myc-recognition sites in promoter regions (2000 bp upstream and 100 bp downstream of the tentative transcription start) of regulated genes was carried out. For this purpose, a specific set of mononucleotide weight matrices with E-box as a core sequence and other computational tools available in the TRANSFAC® professional database (Kel et al., 2003) was used. The results are depicted in Fig.6 and are also included in Table 4. With the stringent cut-offs applied c-Myc binding sites were identified in promoters of known c-Myc target genes, e.g., Fasn, Shtm1, Srm, Apex1; notably, recognition sites in putative gene targets for c-Myc like Uck2, Hk1, Gapd, Smarcc1, Npm3, Nol5, Ppan, Rnac, Lamr1 and others (Table 4) were identified. Importantly, no qualitative differences could be determined in the gene expression profiles of papillary adenocarcinomas of various sizes. However, quantitative differences in expression of Tk1, Top2a, Hmgb2, Rrm2, Kpna2, H1fx were observed which were found to be most strongly induced in small-sized adenocarcinomas. This suggests a specific role of these genes at the onset of tumor growth. Other genes, i.e. arginase1, hexokinase2 and anion exchanger Slc4a4 were expressed at higher level in large tumors (more than 2 fold) when compared with small sized tumors pointing to their essential function in malignant growth (Table 4).

It is also of considerable importance that induction of genes in tumors was identified whose expression was absent in non transgenic control lungs (marked by a gray background for the gene title in Table 4); some were consistently increased in all 10 tumors studied ( marked by grey rows accordingly in Table 4). Likely, these uniquely expressed genes such as arginase1 and serine hydroxymethyl transferase, are good candidates for novel tumor therapy.

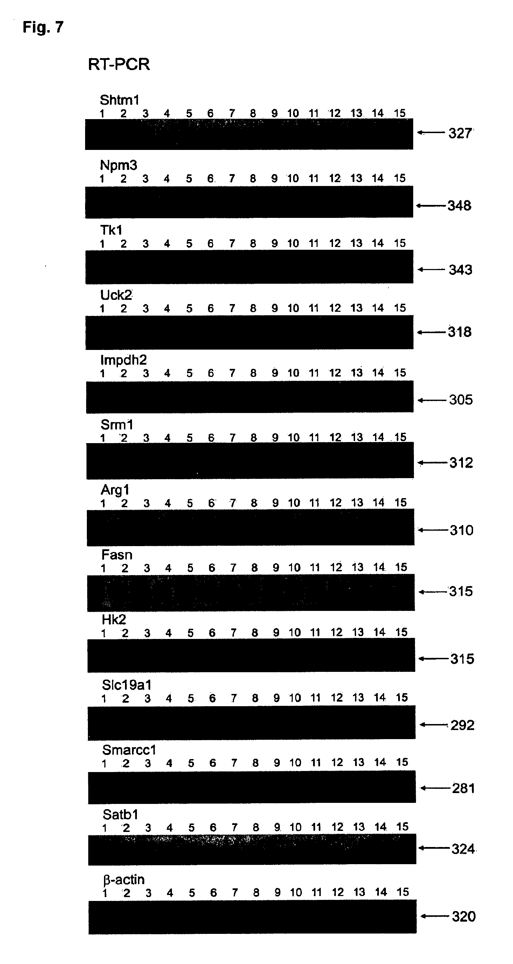

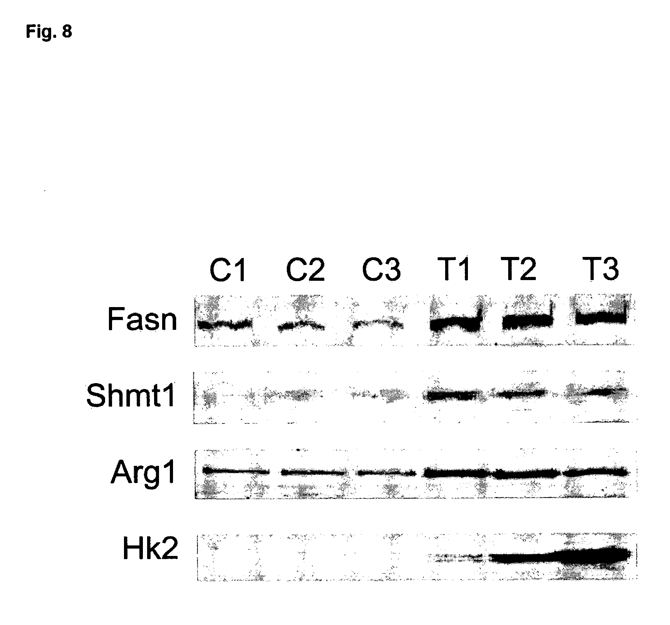

Also, reverse transcription-polymerase chain reaction (RT-PCR) for 12 genes was performed to confirm their regulation in c-Myc induced lung tumors by an alternative method. Notably, the results from both methods agreed well (Fig.7 , Table 5). Additionally, expression of coded protein for Fasn, Shtm1, Arg1 and Hk2 with important functions in cell proliferation and growth was studied. Western blot analysis confirmed the coded proteins (see Fig. 8) to be overexpressed in lung adenocarcinomas.

The solid tumors isolated from the lungs of c-Myc-transgenic mice were identified as papillary lung adenocarcinomas arising from lung epithelial cells, as discussed in part 1 of the study. To follow gene expression changes during lung tumor progression RNA was prepared from tumor sets of small, middle and large size (see Methods) and analyzed using the oligonucleotide microarrays MG u74Av2 to study simultaneously nearly 12500 genes. Statistical analysis of replicates with T-test and stringent comparative ranking was performed to identify regulated genes in tumors as compared with non transgenic healthy lungs (see Methods). The analysis was confined to up regulated genes which were expressed in all tumor samples of at least one set of tumors and down regulated genes which were expressed in all non-transgenic control lungs. The thresholds for significance testing was defined as mean FC≥ 3, p-value in T-test ≤ 0,05 and 100% "Increase" call in comparative ranking analysis at least in one set of tumors for up regulated genes and FC≤- 3, p-value in T-test ≤ 0,05 and 100% "Decrease" for down regulated genes (see Methods). 162 genes with significantly increased expression and 301 genes with significantly decreased expression in c-Myc-expressing lung adenocarcinomas were further analyzed in regard to their biological functions and known c-Myc responsiveness based on publically available databases and the presence of in silico determined c-Myc binding sites in their promotors. Here the focus was directed on regulated genes involved in extracellular signaling, adhesion, angiogenesis and invasion. In Table 6a, b regulated genes are divided into functional groups. Also, the number of c-Myc-binding sites and known c-Myc responsiveness for each gene analysed is reported. Remarkably, in tumors regulation of many genes was observed implicated in the main extracellular signaling pathways thereby contributing to cell proliferation. Thus, some genes coding for proteins involved in the Ras/Raf/MEK/ERK signaling cascade like the autocrine growth factor amphiregulin, Map2k1 (MEK) were specifically up regulated, and several genes that negatively influence Erk signaling like phosphatases Dusp1, Ptpre and Rassf5, a proapoptic Raseffector, were found to be repressed. Elevated gene expression of Arhu and Frat2 as well as repressed expression of Tcf7 and Sox, involved in Wnt/beta-catenin signaling was detected. Importantly, in lung adenocarcinomas multiple gene expression changes were found which can lead to excess of_survival signals and inhibition of antigrowth signaling and, in such a way, contribute to the emerging of a malignant phenotype. These changes included up regulation of Tnf receptor associated factor 4 (Traf 4) and down regulation of the death receptor protein Tnfrsf6 to result in reduction of signaling through cell death receptor. Remarkably, the numerous gene transcript repressed in c-Myc- over expressing lung tumors included many coding for growth-inhibiting functions. Obviously disabling growth inhibitor function contributed to malignant transformation in the lung. Importantly, among repressed genes several genes coding for proteins interfering with survival signaling pathway were detected. These included lgfbp 4, lgfbp5, lgfbp 6 and inhibitor of NFk-beta Nfkbia with reduced expression leads to excessive survival signaling through IGFR and in such a way to suppression of the apoptic capacity of c-Myc. Furthermore, in lung adenocarcinomas of transgenic mice a considerable decline in expression of genes involved in anti-growth TGFbeta signaling like Bmp6 and Tgfβ1 conveying antigrowth signals, Acvrl1, a member of Tgfβ receptor family, Gadd45b and others was found. Genes whose expression was decreased in lung adenocarcinomas also included inhibitors of proliferation like Gas3, Ndr2, Lox, Meox2, Ptprb and Ptprg as well as transformation repressors like Lats2, scaffolding proteins Akap 12 and caveolin-1.

In c-Myc -over expressing lung tumors repression of genes coding for cell-cell and cell-ECM junctions like laminin alpha 3, integrin alpha 8, cadherin 5, protocadherin alpha 4 and 6, and others was detected. Up regulation of Ect2 and down regulation of Vsnl1 and calpain2 in lung tumors demonstrates a role for c-Myc in regulation of cytoskeleton dynamics of transformed cells.

Increased expression of several proteases like HGF activator, Adam 19, lipocalin, hepsin, thimet oligopeptidase 1 and down regulation of Reck, visinine-like1 and semaphorins may well contribute to invasion and angiogenesis in c-Myc-induced lung tumors.

Some of the differently regulated genes were uniquely expressed in tumors_(expressed in all tumor samples, not detected in healthy non-transgenic control lungs) and coded for extracellular proteins, for example, HGF activator, thimet oligopeptidase and kininogen, which makes them good candidates as tumor markers. Inversely, expression of some other genes detected in all normal lungs was lost in all tumor samples. These included, among others, genes coding for cell adhesion proteins protocadherin alpha 4 and 6, Lama3, procollagen XIIIalpha1, dermatopontin, claudin5 as well as antiproliferative proteins like Bmp6, Ndr2 and lgfbp4, Sema3f, fibulin1, the loss of which in lung epithelial tissues may be essential for the development towards malignancy. These genes are highlighted in the Table 6 a,b.

Quantitative differences in gene expression from small to large size tumors included 2-3 fold changes in expression level of several genes. Among those genes whose expression level was substantially higher in small size tumors were adenosine A2b receptor and kininogen presumably involved in angiogenic signaling. In large size tumors expression levels of proto-oncogene Ros1, protease Hgfac, procollagen type XV and claudin2, were significantly increased, while gene expression of Tgfb1, Gadd45b, Igfbp6, Akap12, Ptpns1 coding for proteins involved in inhibition of growth were significantly decreased when compared with small size tumors that infers an important role for tumor progression. Among differently regulated genes in lung adenocarcinomas some known c-Myc-responsive genes as Tnfrsf6 and Hoxa5, involved in Tnf-signaling, scaffolding proteins Akap12 and Cav, phosphatase Dusp1, ECM enzyme Lox as well as established c-Myc targets which included both up regulated (for example, mucin1, Icam1) and down regulated genes (for example, Tcf7, Notch4) were found. The remaining regulated genes represent newly identified c- Myc responsive genes and are of great interest for their role in cancer development.

Search of the putative c-Myc-binding sites in the promoter region yielded new putative c-Myc -targets which may play a role in tumor development like Arhu involved in Wnt-signaling, anti-apoptic Traf4, membrane -anchored peptidase Adam19, anti-growth Bmp6 and Rbp1, anti-invasive Reck and previously unsuspected classes of c-Myc-responsive proteins- connexins (Gjb3, Gja1) and semaphorines such as Sema3c.

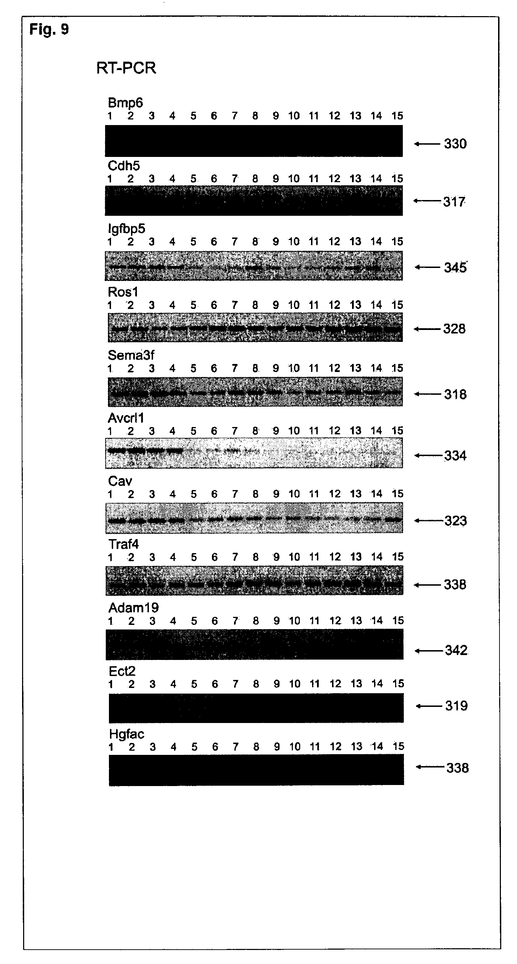

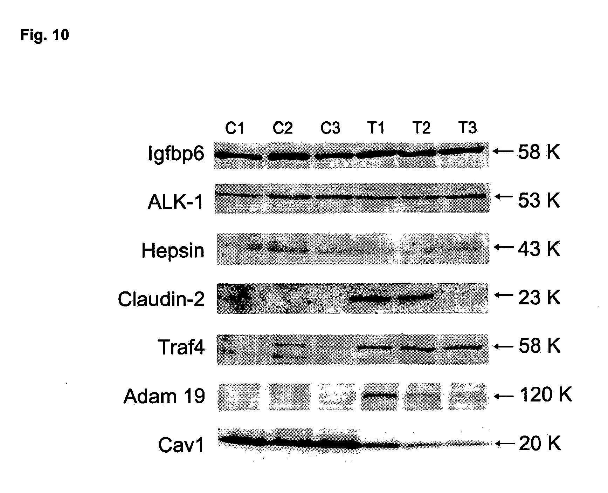

To confirm the microarray data RT-PCR analysis for 5 up regulated and 6 repressed genes in 11 individual lung tumors and 4 control lungs (Fig. 9) was performed. Gene expression identified by both methods were in good agreement (Table 7). Western blotting (Fig. 10) provided further evidence for regulation at the protein level, most notably for claudin2, Traf4 and Adam19 while proteins Hepsin, Alk1(Acvrl1) and Igfbp6 were equally expressed in tumors and control lungs.

### Hispopatology

By use of the surfactant protein C promoter it was possible to selectively target alveolar epithelium and achieved > 50-fold c-Myc expression. This led to an atypical proliferation with malignant cell transformation resulting ultimately in invasive PLACs. The WHO-Classification of human lung tumors (Travis et al., 1999) was used because it fitted well with the observed alterations in c-Myc-induced lung cancer. The resulting hyperchromatic monolayers of bronchiolar alveolar cells were typical for a non-mucinous BAC. The growth of the invasive PLAC was not hindered by the surrounding non-invasive BAC-formations. All invasive tumors of the c-Myc-transgenic model were invariably pure PLAC from the very onset of invasion. Likely, the non-mucinous BAC and pure PLAC observed in the study are derived from the same ancestral tumor progenitor, as suggested by others (Shimosato et al., 1982). Based on histopathology, the disease model as described herein is highly relevant for the study of human lung malignancies. This is of considerable importance as primary lung adenocarcinoma (LAC) is on the rise. Furthermore, the c-Myc-transgenic disease model mimics subtypes of LAC and, therefore, enables novel insight into the molecular pathology of lung cancer. Indeed, a central role of Myc proteins in human tumorigenesis has been reported (Henriksson and Luscher, 1996; Spencer and Groudine, 1991) Despite intense research, the mechanism of c-Myc induced tumorigenesis is still not fully understood and many aspects are still enigmatic. Identifying the genetic program induced by c-Myc in vivo can help to improve an understanding of the molecular basis of c-Myc action and define its role in neoplasia as detailed below. Initially the focus was directed on regulators of cell cycle progression and apoptosis.

### c-Myc activates cell cycle

In normal cells, expression of c-Myc is tightly regulated (Sears and Nevins, 2002). The resting cells express little c-Myc, whereas cells stimulated by growth factors dramatically increase c-Myc expression that persist into the cell cycle, but returns to its basal quiescent state in resultant resting daughter cells. Thus, the role of c-Myc in normal cells consists of coordination of cell physiology with extracellular stimuli. Experimental activation of c-Myc often allows cells to enter S-phase and undergo mitosis in the absence of external factors (Eilers et al., 1991). In c-Myc overexpressing lung epithelial tumor cells an altered expression of some key cell cycle regulators was found that can be responsible for the cell cycle reentry as discussed below.

### G1/S promotion

The progression of cell cycle is catalyzed by cyclin-dependent kinases which are activated by cyclins. In mammals, the cell cycle is initiated by forming a Cyclin D1-CdK4 complex which inactivates Rb protein by phosphorylation. Phosphorylated Rb protein, in turn, liberates E2F transcription factors to activate gene expression of many proteins required for S-phase entry and DNA replication. In c-Myc-induced lung tumors Cdk4 was up regulated, and gene expression of its inhibitor, Cdkn2d (p19), was repressed. Additionally, the expression of cyclin D1 was also increased in tumors. Further, expression of the heterodimer partner of E2Fs, transcription factor Dp1 (Tfdp1), was significantly elevated. These changes are highly suggestive for c-Myc-over expression in the lung tumors to abrogate Rb protein/E2F regulated pathway which controls G1 progression and S-phase initiation and therefore allow inappropriate reinititiation of DNA synthesis. These transcriptional events induced by c-Myc may possibly explain the typical observation of DNA endoreplication and polyploidy in cells that were activated to overexpress c-Myc but then exited mitosis in G2 (due to the activation of intrinsic cell cycle checkpoints or following the treatment with mitotic inhibitors (Gandarillas et al., 2000).

### G2/M promotion

The final M-phase of cell cycle is triggered by Cdk1 (Cdc2a) associated with mitotic cyclin B. In constitutively c-Myc expressing lung tumor cells, significant up regulation of genes coding for both cyclin B1, cyclin B2 and associated kinase cdc2a (cdk1), the complex of which promotes the transition G2/ M, was observed. Besides, over expression of several other genes essential for progression of mitosis like serine/threonine kinases Nek6 and Stk6 (Aurora-A kinase), Cks1 (cdc28 protein kinase), Cks2 (cdc28 protein kinase regulator subunit 2), cdc20, regulators of cytokinesis Prk1 (protein 1), and three kinesin family members coding for molecular motors involved in various kinds of spindle dynamics was detected in lung tumors. Most of these mitotic genes were shown the first time to be activated in response to c-Myc. Transcriptional activation of Aurora-A kinase, observed at the highest level in small size lung tumors, may additionally contribute to the genomic instability induced by c-Myc. The elevated expression of the gene was shown to override the checkpoint mechanism that monitors mitotic spindle formation, resulting in an inappropriate enter of anaphase, leading to mitotic abnormalities. (Anand et al., 2003). Furthermore, up regulation in lung tumors of the proto-oncogene ect2 which plays a critical role in cytokinesis (Saito et al., 2004) as well as repression of tumor suppressor Lats2, which negatively regulates the cell cycle by controlling G1/S and/or G2/M transition (Li et al., 2003), are important additional changes induced by c-Myc and likely contributed to an advanced cell cycle.

An altered transcription of genes coding key cell cycle regulator proteins may lead to an accelerated cell cycle and when combined with abrogation of checkpoints may account for genomic instability, including karyotypic abnormalities, characteristic for c-Myc overexpressing cells (Felsher and Bishop, 1999; Yin et al., 1999)

### Loss of intracellular control of cell division

*Inactivation of p53-mediated apoptosis:* C-Myc was found to be a powerful inducer of apoptosis. This phenomenon can be observed by experimental hyperactivation of c-Myc alone in most normal cells which undergo cell death following cell proliferation (Pelengaris et al., 2002). The apoptotic function of c-Myc protein is related to its other function (Evan et al., 1992), and the innate apoptotic potential of c-Myc serves likely as a restriction for its oncogenic capacity. Besides, excessive proliferation and growth, per se, are detected in normal cells, and the activation of intracellular check point mechanisms causes cells to undergo cell cycle arrest or apoptosis, thereby preventing their inappropriate proliferation and survival. How do tumorous lung epithelial cells inactivate cell cycle check points and cell death machinery? By gene expression profiling of c-Myc over expressing lung tumors multiple alterations were identified that may have negatively affected apoptosis and cell cycle arrest programs. A central intersection of pathways governing a cell's responses to DNA damage and other disorders is the tumor suppressor protein p53 (Trp53) with triple role in cell-cycle arrest, apoptosis and DNA repair. Moderate elevation of Trp53 gene transcription level in lung tumors of transgenic mice was observed that was expected because the gene is a known direct target for c-Myc. Its expression may be induced by c-Myc directly or indirectly, as a response to signaling imbalance provoked by the oncogene. Importantly, in lung adenocarcinomas deregulation of several genes that interfere with the p53 response was identified. Among these a dramatic decrease of gene expression of epithelially enriched transcription factor Klf-4, an essential mediator of Trp53 in controlling cell cycle progression following DNA damage (Yoon et al., 2003) was detected. The transcription factor regulates expression of numerous cell-cycle regulatory genes in a concerted manner, activating cell-cycle inhibitors and repressing cell-cycle promoters (Chen et al., 2003b). Mdm-2 is the main regulator of Trp53 which can trigger its degradation by the ubiquitin system. Moreover, in papillary lung adenocarcinoma the loss of expression of transcription factor Hey-1 was detected, which was recently shown to activate Trp53 protein through repression of Mdm2 transcription and to induce apoptosis in vivo (Huang et al., 2004). Another transcriptional repressor of Mdm-2 suppressed in lung adenocarcinomas was transcription factor Stat-1 which also binds to p53 and acts as a coactivator for induction of Trp53- responsive genes (Townsend et al., 2004). Furthermore, a significant decline in Gas-1 expression (detected at extremely low level in 3 of 10 tumors) was found which blocks cell proliferation in a p53-dependent manner (Del Sal et al., 1995). At last, up regulated genes in tumors included a known target of c-Myc Hspa9a (homologous to human mortalin-2). The gene codes for a heat-shock protein 70 family member involved in cytoplasmic sequestration and inactivation of Trp53 through direct binding (Wadhwa et al., 2002), and its over expression was reported for human lung adenocarcinomas (Volm et al., 1995). Likely, the identification of two binding sites of c-Myc in the promoter region of Hspa9a indicates direct regulation of the gene by c-Myc. Altered expression of the discussed genes points to an escape mechanism of cell cycle arrest and apoptosis in c-Myc-overexpressing lung tumor epithelial cells.

### Repression of intrinsic apoptotic machinery:

In addition to genes linked to the silencing of Trp53 activity, changes in expression of several genes interfering with the cell apoptotic machinery were observed in tumor tissues. One of these was up regulation of Birc5 (survivine), which is a member of the inhibitor of apoptosis protein (IAP) family and interferes directly with the function of caspases (Chiou et al., 2003). Several down regulated genes in tumors coded for proteins interfering with the anti-apoptotic Bcl-2 protein. This is of particular importance because Bcl-2 inhibits almost all types of cell death. One of these repressed genes was Bnip-2, coding for a protein which interacts directly with death-inhibiting Bcl-2 and induced apoptosis via a caspase-dependent mechanism (Zhang et al., 2002). The other repressor calpain- 2 is an intracellular protease that was demonstrated to promote decrease of Bcl-2 proteins by cleavage and thereby triggers the intrinsic apoptotic pathway (Gil-Parrado et al., 2002). Proapoptotic effect of Ddit3 (=Gadd153) was linked to down regulation of Bcl-2 and enhanced oxidant injury (McCullough et al., 2001). Furthermore, the tumor suppressor Lats2 was down regulated which was reported to induce apoptosis in lung cancer cells through decrease of the protein level of anti-apoptotic proteins BCL-2 and BCL-x(L) and activation of caspase 9- cascade (Ke et al., 2004). At last, expression of the tumor suppressor Anp32a (=PHAP) was reduced which promotes caspase-9 activation after apoptosome formation (Jiang et al., 2003). Markedly, some of these proteins with regulatory role in apoptosis, have other functions that can also contibute to cell proliferation and motility. For example, Birc5 is implicated not only in anti-apoptosis but also in cytokinesis by stimulating Aurora-B kinase activity (Chen et al., 2003a), Lats2 inhibits cell cycle by controlling different check points (s. above). Anp-32 is involved in repression of transcription as part of the INHAT (inhibitor of histone acetyltransferases) complex (Seo et al., 2002) and calpain-2 plays a role in cell migration through regulation of membrane activity and morphology (Franco et al., 2004).

In summary, the gene expression changes discussed above indicate essential molecular events that allowed c-Myc-overexpressing tumor cells to suppress intracellular control for cell division.

### The presence of c-Myc-binding sites in promoters of deregulated gene

C-Myc binding sites were not always identified in genes whose expression was altered in response to c-Myc over expression. Likely, some genes changed their expression as a result of regulation by other transcriptions factors targeted by c-Myc (see Table 2a,b). It was of no surprise that most of the down regulated genes did not contain c-Myc-binding sites in their promoters. Indeed repression of gene transcription by c-Myc is thought to occur independently of binding to the E-box, but is driven by interaction of c-Myc with other transcription factors, notably Miz-1, which recognizes other regulatory sequences (Wanzel et al., 2003). Besides, the repression of several transcription factors by c-Myc, some of them, E-boxes containing, may be one of the reasons for the remarkable large number of down regulated genes in c-Myc-induced lung tumors.

Unexpectedly, a small number of genes known to bind c-Myc directly, e.g. cyclin B1, Cdc2a, Cks2, did not contain c-Myc recognition sites in the bioinformatic analyses. This requires further consideration. First, the selected promoter region was restricted to 2000 bp upstream and 100 bp downstream of the putative transcription start site. It is possible that some c-Myc binding sites are positioned outside this region. Second, the matrices similarity cut-offs were set stringently so that false positive matches would be less than 1 per 10,000 bp. On the other side, such cut-off enables recognition of only 10 -30% of putative sites. Finally, for human E-box containing genes a distribution into two groups with low and high affinity binding for c-Myc has been reported (Fernandez et al., 2003). Remarkably, overexpression of c-Myc enhanced its association with low affinity E-boxes and at very high level to non targeted chromatin. Possible, c-Myc overexpression of > 50-fold, as achieved in the study, resulted in perturbation of transcriptional control and extended activity of c-Myc beyond E-box containing promoters. The consequence may be up regulation of such genes as Birc5, Ect2 and others found to be uniquely expressed in lung tumors.

### Orthologs regulated in human malignancies.

A number of genes regulated in c-Myc-induced papillary lung adenocarcinomas that contribute to cell cycle progression, growth and block of apoptosis, i.e. Ccnd1, Stk6, Klf-4, Gas1, Hey1 and Stat-1 were similarly regulated in various human malignancies (He et al., 2004; Anand et al., 2003; Chen et al., 2003a). Apart from remarkable agreement in histological phenotypes the disease model mimic closely genetic events in human lung cancer. Therefore, its significance in the study of lung tumor development is emphasized. Specifically, altered expression of some genes in lung tumors of the mouse model, were identified in human NSCLC as well. This included overexpression of CDK4, CDC2A, TFDP1, CCNB1, CDC20 (Singhal et al., 2003), and contributed to promotion of cell cycle. In addition, overexpression of anti-apoptotic BIRC5 (Escuin and Rosell, 1999) and heat shock protein HSP70 (Volm et al., 1995) as well as repression of the tumor suppressor LATS2 (Li et al., 2003) were also found in human lung cancer. Up regulation of Cdk4 and down regulation of pro-apoptotic Bnip2 were likewise detected in chemically induced murine lung tumors (Bonner et al., 2004). These changes in gene expression point to similarities in the development of lung adenocarcinoma in mouse and human malignancies and demonstrate the relevance of the c-Myc-induced lung tumor model for investigating human NSCLCs.

To the best of our knowledge, some genes identified in the study were not previously associated with human lung carcinogenesis like the newly identified c-Myc responsive genes Hey-1, Anp32a, Bnip-2, calpain-2, amongst others. Their altered expression may lead to inhibition of apoptosis and these genes represent interesting candidates for further validation as therapeutic targets.

### Central role of c-Myc in activation of cell metabolism

Previously, changes in gene expression induced by targeted c-Myc-overexpression in lung epithelium were reported to result in an activation of genes coding for cell cycle promotion and inhibition of cell cycle arrest and death. Here the oncogenomics associated with different metabolic pathways are presented to enable rapid growth and proliferation of tumor cells. Notably, induction of genes coding for DNA and RNA metabolism, protein synthesis, ribosomal biogenesis, glycolysis and transport in c-Myc induced lung tumors was observed ( see Table 4). The ability of c-Myc to control transcription of genes involved in both cell cycle progression and growth is in agreement with other studies (Lutz et al., 2002). A significant number (53,7%) of identified genes are known to be c-Myc responsive in other cell types (Fig. 6). This supported the notion that c-Myc is a key activator for cell metabolism and that the set of regulated genes is, at least in part, common in various cell types. Moreover, the presence of c-Myc recognition sites in more than one half of regulated genes (Nonetheless Nonetheless.1) argues for the ability of c-Myc to directly activate the transcription of genes directly involved in cell growth.

### Metabolic genes activated by c-Myc contributes to regulation of cell proliferation

Recent data provided evidence that several c-Myc target genes which were found to be upregulated in lung adenocarcinoma code for metabolic enzymes and may contribute otherwise to cell proliferation. For example, thymidilate synthase which maintains the dTMP pool critical for DNA replication functions also as an RNA binding protein. It forms a complex with a number of cellular mRNAs, including the RNA corresponding p53 tumor suppressor gene, to cause translational repression (Liu et al., 2002), and, in such a way, may regulate cell cycle and apoptosis. Furthermore, fatty acid synthase (Fasn) is a key metabolic enzyme catalyzing the synthesis of long-chain saturated fatty acids and plays a central role in the production of surfactant in lung. On the other hand overexpression of Fasn has been linked to dysregulation of membrane composition and modulation of lipid rafts functioning in tumor cells. Lipid rafts are membrane microdomains involved in signal transduction, intracellular trafficing and cell migration (Swinnen et al., 2003). The Fasn is expressed at high level in numerous human malignancies (Evert et al., 2005), and it was found that the ability of flavonoids to induce apoptosis in cancer cells is strongly associated with their properties to inhibit Fasn (Brusselmans et al., 2005). In addition, nucleophosmin (Npm1) is a nucleolar phosphoprotein which functions as a molecular chaperone and plays an important role in ribosomal protein assembly and transport through prevention of proteins from aggregating in the nucleolus. Besides, it inhibits apoptosis by suppression of the protein kinase PKR. This kinase effects apoptosis in normal cells in response to various extra and intracellular cues (Pang et al., 2003). Up regulation by c-Myc of spermidine synthase (Srm) in lung tumor cells could lead to elevated intracellular level of polyamines which are substantial for cell proliferation. Polyamines are not only of critical importance for protein biosynthesis, membrane stability, chromatin structure or modulation of ion channels but they have been shown in breast cancer cells to regulate the interaction of transcription factors such as NFkB with their responsive elements, thereby leading to an up regulation of genes involved in proliferation (Shah et al., 2001). In the case of Fas, Npm1 and Srm multiple (2,3 and 5, respectively) c- Myc -binding sites were found in the promoter region (Table 5). This suggests direct up regulation of these genes by c-Myc in lung adenocarcinomas. Unlike normal cells many tumors rely entirely on glycolysis for ATP production even in a not hypoxic environment, known as the Warburg effect (Garber, 2004). Though being less efficient, glycolysis provides ATP quickly which is needed for the many metabolic processes associated with rapid tumor growth. Note, several glycolytic enzymes were over expressed in lung adenocarcinoma and contained c-Myc -recognition sites in their promoters or were previously shown to bind c-Myc directly (see Table 1). This clearly demonstrates a direct role of c-Myc in the activation of glycolysis (Table 4). It is highly interesting that glycolytic enzymes, similarly to other metabolic enzymes discussed above, have been shown to have additional functions in cell proliferation. For example, activity of hexokinase 2 associated with the outer membrane of mitochondria, has been implicated in protection from apoptosis (Garber, 2004; Majewski et al., 2004). Gpi1/AMP (glucose phosphate isomerase 1/autocrine motility factor), a ubiquitous cytosolic enzyme that plays a key role in glycolysis, is a multifunctional protein that acts in the extracellular milieu as a potent mitogen/cytokine. Indeed, its over expression contributes to motility, invasion and metastasis (Garber, 2004; Tsutsumi et al., 2004) and was correlated with aggressive tumor growth and poor prognosis in human pulmonary adenocarcinomas (Garber, 2004; Takanami et al., 1998). These examples demonstrate how expression of metabolic genes induced by c-Myc contribute to promotion of cell proliferation in lung tumorigenesis.

Remarkably, 9 genes coding for enzymes involved in purine and pyrimidine nucleotide synthesis pathways (Tk1, Tyms, Shtm1, Srm, Impdh2, Gart, Uck2, Rrm2, Rrm1) were identified as well as folate transporter Slc19a1 which were found to be over expressed in lung adenocarcinomas and are essential for rapid cell proliferation. Indeed, inhibition of thymidilate syntase by 5-fluorouracil is an established therapy against certain tumors. Notably, Shmt1, both mRNA (Table 4) and protein (Fig. 8), was expressed in all c-Myc-induced lung adenocarcinomas but not in healthy non-transgenic lung. Its role was examined in human breast cancer cells and it was shown that cSHTM1 is a metabolic switch that, when activated, gives dTMP synthesis higher metabolic priority than competing synthesis of SAM (S-adenosylmethionine), a cofactor that methylates DNA, RNA, proteins and many metabolites (Herbig et al., 2002). Furthermore, among other genes which were uniquely expressed in lung adenocarcinomas five genes coded for enzymes involved in DNA metabolism - e.g. thymidine kinase 1, topoisomerase (DNA) II alpha, helicase, ribonucleotide reductase M1 and meiotic recombination 11 homolog A. These genes were expressed at the highest level in small tumors. Again, this points to their essential role in emerging tumors. Up regulation of Apex and Xrcc5, involved in DNA repair, indicate that c-Myc couples DNA replication to the maintenance of DNA integrity.

### Altered expression of several genes coding for proteins important in chromatin remodeling.

The increased expression of linker histone H1fx and repression of core histone H2b1 could change the structure of nucleosome and access to nucleosomal DNA for gene expression, DNA replication and repair. Besides, increased expression of genes coding for helicase and Smarcc1 was found. Both have c-Myc-binding sites in their promoters and are involved in all processes including DNA strand separation like gene transcription, DNA replication, recombination and repair. Satb1 and Anp32a, which are found to be repressed, are involved in repression of gene transcription through interfering with histone acetylation (Seo et al., 2002). Altered expression of such genes could lead to remodeling of large chromatin domains and initiate undue-expression of numerous genes in c-Myc- overexpressing cells. In addition, elevated level of the SNRP transcript, the gene coding for a protein involved in RNA editing ( the evolutionary conserved mechanism for regulation of gene expression at the posttranscriptional level that plays an essential role in development) may prove to be of great importance for lung carcinogenesis. These genes were identified as new c-Myc responsive genes and first demonstrate their regulation in cancer (except for helicase which was linked to cancer previously).

### Orthologues regulated in human malignancies.

Several genes regulated in c-Myc-induced lung adenocarcinomas contribute to cell growth e.g. Hk2, Fasn, Uck2, Impdh2, Tk1 and were shown previously to be similarly regulated in human malignancies (Shen et al., 1998; Swinnen et al., 2003; Goel et al., 2003; Natsumeda et al., 1990). Strikingly, altered expression of some of these orthologues were specifically identified in human NSCLC. This included induction of RRM2 and TOP2A transcript expression with defined roles in DNA synthesis and transcription (Wikman et al., 2002), as well as overexpression of ARG1 which fosters polyamine synthesis (Suer et al., 1999). High activity of TYMS activity and GPI1 expression was associated with poor prognosis of patients with NSCLC (Nakagawa et al., 2004; Takanami et al., 1998) as was exaggerated activity of LDH with tumor stage (Rotenberg et al., 1988). Clearly, this demonstrates the relevance of the findings obtained in transgenic mice for human NSCLCs, as described herein. To the best of our knowledge, certain genes identified in the study were not previously associated with human lung carcinogenesis and these included the c-Myc targets Srm, Shmt1, Npm1, Sic19a1 as well as the newly identified c-Myc responsive genes Satb1 amongst others. Their specific regulation represents new and important therapeutic targets worthy for further exploration to combat this highly malignant disease.

In the effort to better understand the transforming capacity of c-Myc in lung adenocarcinomas firstly gene networks directly involved in cell cycle progression and cell growth as well as regulation of cell cycle arrest and programmed cell death were examined. As intrinsic programs to regulate cell proliferation, growth and cell death are also governed by extracellular signaling, regulation of genes coding for proteins involved in the main pro- and anti-growth extracellular signaling pathways was studied. In the following, the consequences of c-myc overexpression on extracellular signaling, angiogenesis and invasion are discussed.

### Incapacitating of extrinsic pathway of growth-inhibitory signaling

### Decrease of cell death receptor signaling

In lung tumors gene expression changes were identified that could have a negative impact on the death receptor signaling , for example, decrease in expression of the death receptor protein Tnfrsf6 (FAS) and up regulation of uniquely expressed gene transcripts in tumors, such as Traf 4 which codes for a putative signal-transducing protein and was shown to inhibit Fas-induced apoptosis in transformed embryonic kidney cells (Fleckenstein et al., 2003). Both Tnfrsf6 and Traf 4 possess c-Myc-binding site in their promoters, and presumably are directly regulated by c-Myc. Besides, in lung tumors decreased expression of proapoptic transcription factor Hoxa5 was found which was shown to sensitize cells more than 100-fold to TNF-alpha-induced death mediated by caspase 2 and 8 (Chen et al., 2004). Expression of this transcription factor led to apoptotic death in breast tumor cells and was lost in the majority of breast tumors (Raman et al., 2000). The expression of these genes could make lung epithelial cells resistant to apoptosis induced by cytokines like FasL and TNFalpha.

### Evading the Tgf-beta growth- suppression signaling

Gene expression of several components of the Tgf- beta (TGFb) signaling pathway was significantly repressed in c-Myc- induced lung adenocarcinomas. Members of the TGFb superfamily, including TGFb and BMPs are important extracellular signaling proteins which inhibit proliferation of several cell types, either by blocking cell cycle progression through G1 or by stimulating apoptosis. Down regulated genes in lung tumors of transgenic mice included genes coding for type I receptor for TGFb superfamily of ligands Acvrl1 and a Tgfb coreceptor endoglin as well as the ligand molecules Tgfb1 and Bmp6. Notable, gene expression of Bmp6, a new putative c-Myc target, was lost in all lung tumor tissues of transgenic mice. Additionally, expression of dermatopontin was completely abrogated in lung tumors. This gene codes for a component of extracellular matrix that increases the cellular response to TGF-beta (Okamoto et al., 1999). Besides, significant decrease in expression of transcription factor Zfhx1 a that is crucial regulator of TGFb/BMP signaling through synergizing with SMAD to induce growth arrest (Postigo et al., 2003) was detected. Additionally, an immediate early response gene for TGFb inhibitory growth factors Gadd45b, which functions as a positive effector of Tgfb-induced apoptosis (Yoo et al., 2003), was found to be repressed in tumors of c-Myc transgenic mice. Gadd45b is a likely c-Myc target gene based on the c-Myc recognition site which was identified in its promoter. A strong repression of Gadd45 was also observed in RAT-1 overexpressing c-Myc cells (Amundson et al., 1998) and therefore agrees well with the finding as described herein. The inhibited expression of discussed genes could likely disrupt growth-inhibitory signaling by Tgfb family proteins in lung tumor tissues.

### Promotion of cell survival through IGFR-signaling pathway

Apoptosis can be antagonized by exogenous survival factors like insulin-like growth factors (IGF) I/II which bind to cell-surface receptors and activate Pl3 kinase- Akt/PKB signaling pathway that keeps the cell death program suppressed. Gene expression changes in c-Myc induced lung adenocarcinomas indicated significant activation of the cell survival signaling pathway. The prominent feature in lung adenocarcinomas was the significant decrease of gene expression levels of three Igf-binding proteins - lgfbp 5, 6, 4 which were expressed at high level in control lungs. These IGFBPS bind to IGFs and regulate its biological availability and function. Besides, they exert also IGF-independent growth - inhibitory effects (Clemmons, 1998). Thus, decline of the level of the IGFBPs could provide for a local excess of available IGFs in tumor tissue and thereby contribute to antiapoptotic signaling through IGFR. It is of considerable importance that amongst several growth factors tested only IGFs proved to be survival factors for c-Myc-over expressing fibroblasts (Harrington et al., 1994). Furthermore, repression of additional genes involved in negative regulation of IGF1R signaling pathway was found. These were Socs2 coding for a protein which interacts with IGF1 R and suppresses IGF1-induced growth (Dey et al., 1998) and Nfkbia, which inhibited NFkB (a transcription factor activated by P13K-Akt signaling) by trapping it in the cytoplasm. Activation of IGFR-mediated survival signaling through inhibition of expression of several negative regulators of the pathway could antagonize c-Myc-mediated apoptosis and allow proliferation and growth of c-Myc-overexpressing lung epithelial cells without concomitant apoptosis.

### Activation of growth factor signaling through tyrosine kinase receptors and related genes

Many of the regulated genes identified in c-Myc-induced lung adenocarcinomas coded for molecules involved in the main cell signaling pathways with a positive effect on cell division and growth. A key role in extracellular stimulation of cell growth and proliferation belongs to the signaling through EGFR (epidermal growth factor receptor). One of the up regulated genes in lung adenocarcinomas of c-Myc transgenic mice coded for amphiregulin (AR), an autocrine growth factor of the EGF family. AR was shown to stimulate proliferation of human lung epithelial cells through binding to EGFR (Lemjabbar et al., 2003) as well as survival of the human NSCLC cells through activation of IGF1 R-dependent pathway (Hurbin et al., 2002). Besides, AR was demonstrated to be an essential mediator of G-protein-coupled receptor (GPCR)-induced transactivation of EGFR signal and down stream cell response in squamous cell carcinoma. In turn, the secretion of AR could be induced through an activation of the EGFR - signaling pathway in bronchiolar epithelial cells (Blanchet et al., 2004) and IGF1R in NSCLC (Hurbin et al., 2002). These findings suggest that AR growth factor may play an important role in lung cancer development and, like in human cells, increased AR expression in c-Myc -induced lung tumors may resulted in stimulation of autocrine growth and suppression of apoptosis.

One further component of the EGFR/Ras/Raf/Mek/Erk -signaling cascade found to be up regulated in lung tumors was Map2k1 (Mek) while Rassf5, the pro-apoptic Ras effector, was down regulated, therefore evidencing the suppression of Ras-mediated apoptotic signaling. Notable, Rassf5 was not expressed in 80% of human lung adenocarcinomas and was specifically repressed in lung epithelial tumor cells (Vos et al., 2003). Additionally, among down regulated genes in lung adenocarcinomas several phosphatases like Dusp1, Ptpre, Ptpns1, Ptprb and Ptprg were identified. Likely, specific dephosphorylation of signaling proteins such as MAPKs negatively regulate receptor tyrosine kinase -coupled signaling processes. Notably, the phosphatases Ptprb and Ptprg excert growth inhibitory effect (Gaits et al., 1995); (Liu et al., 2004), and expression of Ptprb was dramatically decreased expression of Ptprb in human lung adenocarcinomas (Gaits et al., 1994).

Among uniquely expressed genes in c-Myc-induced tumors the proto-oncogene Ros1 coding for a transmembrane tyrosine kinase which may function as a growth factor receptor (Keilhack et al., 2001 was identified).

Another gene uniquely expressed in tumors coded for the HGF activator, a secreted protease that converts precursor of HGF in its active form which functions as a potent mitogenic factor for epithelial cells and was found to induce angiogenesis (Sengupta et al., 2003). Up regulation of HGFac was also observed in human lung adenocarcinomas (McDoniels-Silvers et al., 2002). The unique expression of these genes which in tumorous lungs may be essential for promotion of lung carcinogenesis. Besides, up regulation of Adam19, a member of a disintegrin and metalloprotease family that is involved in signal transduction through processing of transmembrane growth factor precursors (Fischer et al., 2003) may foster activation of EGFR-signaling in lung tumors of transgenic mice. Presence of c-Myc-binding sites in the promoters of Adam19, Dusp 1 and Map2k1 likely indicates their direct regulation by c-Myc.

### Activation of Wnt / beta-catenin signaling

The study of differently expressed genes in lung tumors of transgenic mice provide evidence for Wnt signaling to be affected in c-Myc-induced lung adenocarcinomas. In tumor tissues enhanced expression of Arhu, a member of the Rho family that mediates in part the effects of Wnt1 signaling in the regulation of cell morphology, cytoskeleton organization and cell proliferation (Tao et al., 2001) was found. Also, unique expression of Frat2, a positive regulator of Wnt signaling and stabilizer of beta-catenin (Saitoh and Katoh, 2001) was observed. In contrast, repressed expression of the transcription factors Tcf7 and Sox7, which are negative regulators of Wnt/beta-catenin -induced gene transcription, was observed. Down regulation of these genes was shown to be associated with malignant transformations (Roose et al., 1999). Modulation of Wnt signaling in c-Myc transgenic mice contributed to promotion of cell proliferation, cytoskeleton dynamic and motility of tumor cells. It is of considerable importance that c-Myc-binding sites were found in promoters of Arhu and Tcf7, the latter was shown to be a c-Myc target gene, therefore linking c-Myc directly to the activation of Wnt-signaling pathway.

### Regulation of cytoskeleton contributing to transformation, increased motility and invasiveness

The ability of malignant cells to migrate and invade surrounding tissues is associated with changed motility based on modifications of the cytoskeleton. A central role in rearrangement of the actin cytoskeleton in response to diverse external signals is ascribed to members of the Rho protein family. Many genes involved in regulation of cytoskeleton and transformation were found to be differentially expressed. These included the strongly up regulated proto-oncogene Ect2, a growth-regulatory molecule, which, in addition to its ability to regulate cell cycle, activates as a guanine nucleotide exchange factor the Rho family of Ras-related GTPases to bring about remodelling of actin cytoskeleton and to contribute to cell transformation (Saito et al., 2004); (Westwick et al., 1998).

In lung tumors the loss of expression of invasion suppressor Vsnl1, which was shown to decrease RhoA activity and MMP-9 level through cAMP-mediated signaling (Mahloogi et al., 2003), may enhance the malignant phenotype. Down regulation of calpain-2, an intracellular protease required for proteolysis of the cytoskeleton and focal adhesion proteins was shown to restrict membrane protrusions and lamellipodia dynamics (Franco et al., 2004), to further strengthen promigratoric capacity of lung tumor cells.

### Changes in cell junctions and adhesion: increased motility and invasiveness

Cell - cell and cell - extracellular matrix (ECM) junctions do not only bind cells mechanically through adhesion but also transmit signals to the cell interior thereby playing an important role in regulation of cell survival, proliferation and migration.

For example, up regulation of the c-Myc target gene mucin1, a transmembrane glycoprotein defined as a tumor antigen (VanLith et al., 2002) that has been implicated in activation of Wnt/beta-catenin -signaling (Huang et al., 2003) was observed in lung tumors of transgenic mice. Over expression of mucin1 was shown to be involved in invasive tumorigenesis in the breast (Schroeder et al., 2003) and colon (Baldus et al., 2004). The other c-Myc target gene Icam1 (intercellular adhesion molecule1) which is the only known direct ligand of the mucin extracellular domain was also over expressed in c-Myc-induced lung tumors. It was shown that mucin1, on the contact with Icam1 produced calcium-based signals that might affect the cytoskeleton and increased cell motility (Rahn et al., 2004). In contrast, strong decrease in expression of genes coding for cell-cell junction proteins like cadherin-5, protocadherins alpha 4 and 6 may contribute to cell proliferation and migration in lung tumor. Additionally, in lung adenocarcinomas of transgenic mice repression of several genes coding for proteins implicated in cell- ECM interactions was observed. These included fibulin-1, an extracellular matrix (ECM) protein which affect the Erk- mediated signaling transduction cascades to inhibit motility and invasiveness of cancer cells (Twal et al., 2001), and Lama3 (laminin 5), a ECM glycoprotein shown to be repressed in various human cancers (Sathyanarayana et al., 2003). The decreased expression of matrix receptor alpha8integrin which negatively affects cell migration and proliferation (Bieritz et al., 2003) could therefore contribute to advanced malignant phenotype of lung tumors.

Tumor specific regulation of genes involved in cell adhesion may impact control of cell growth through changes in cell-cell and cell-matrix attachment to foster increased cell proliferation and motility in lung tumor tissue.

### Degradation of ECM

Based on the histopathology of PLACs an invasive nature of the lung tumor was evidenced. Note, proteolysis of matrix proteins is essential for cell migration. Matrix components are degraded by extracellular proteolytic enzymes, most of which are matrix metalloproteinases while others are serine proteases. In lung tumors up regulation of gelatinase-associated protein lipocalin 2, a regulator of activity of matrix metalloproteinase (MMP) was found, which was suggested to be involved in invasion of tumor cells (Liu et al., 2003; Huang et al., 2003) Additionally, repressed expression of the metastasis suppressor Reck was observed (Liu et al., 2003) which suppresses invasiveness of carcinoma cells through inhibition of the release of MMP. Likewise, upregulation of the metalloproteinase Adam 19 impacts EGFR signaling as discussed above. Furthermore, the known c-Myc responsive gene Thop1 was significantly overexpressed in tumor tissues of transgenic mice. This gene codes for a member of the metalloendopeptidase family but was not as yet linked to cancer. Among serine proteases over expressed in c-Myc-induced lung tumors the cell surface protease hepsin was found over expressed. This protein was demonstrated to cause disorganization of basement membrane and promote progression of metastasis in a mouse prostate cancer model (Klezovitch et al., 2004). The other serine protease, i.e. HGF activator, a serine protease specific for activation of HGF (Tjin et al., 2004) with key roles in invasion and metastasis of cancer (Hurle et al., 2005), was upregulated as well. At last, gene transcription of extracellular enzyme lysyl oxidase (Lox) indicated an inhibition of cross-linking of collagen and elastin in ECM that makes the matrix fragile and prone to tear. Mutations or reduction of the LOX transcript level in human cancer suggests this protein may excert tumor suppressive activity (Rost et al., 2003).

### Gap junctions

Over expression of connexins 43 and 31 in lung tumors revealed an unsuspected role for c-Myc in cell-cell communication.

Note, there connexins contain c-Myc binding sites in their promoters as evidenced by in silico annotations. These proteins form intercellular channels, which allow the exchange of small molecules (such as inorganic ions, sugars, nucleotides, intracellular mediators like cyclic AMP and IP3) among coupled cells. Connexin43 is essential for survival, and its expression was altered in malignant tissues and in the presence of carcinogenic factors. Besides, it was reported that connexins can be induced through Ras-signaling (Carystinos et al., 2003). The consequences of perturbed connexin 31 expression on cell proliferation (Kibschull et al., 2004) and cell death (Di et al., 2002) were discussed elsewhere.

### Tight junctions

Tight junctions seal epithelial cells together and function as selective permeability barriers. They are formed exclusively by integral membrane proteins of the claudins family. Their role in cancer is not well understood. High level of claudin-5 gene expression was observed in control lung which was progressively suppressed and eventually absent in large tumors. As a consequence loosening of intercellular adhesion and increased motility can be expected. Inversely, gene expression of claudin 2, absent in normal lungs, was strongly induced in tumors, probably, due to the activation of Wnt signaling (Mankertz et al., 2004). Claudin 2 and some other claudin family members were shown to be involved in activation of proMMP through recruitment of enzymes on the cell surface (Miyamori et al., 2001) and so may contribute to invasion.

### Proangiogenic signaling

Some of the differently expressed genes in lung adenocarcinomas of transgenic mice can be linked to angiogenesis. Indeed, a remarkable up regulation of kininogen, a haemostatic factor that exerts its angiogenic effect (Colman et al., 2003) through signaling of its cleaved product form bradykinin through the B (2) receptor, was observed (Yang et al., 2003). Increased transcription level of G-protein-coupled receptor Adora2b detected in tumor tissues might additionally contribute to a stimulation of angiogenesis because it was shown to mediate secretion of angiogenic factors like VEGF and IL-8 (Feoktistov et al., 2003). Furthermore, in all lung tumors the loss of expression of Sema3f, a secreted signaling molecule from the immunoglobulin family with antagonistic action towards Vegf due to competitive binding to neuropilin1/2 receptors (Nasarre et al., 2003), was observed. Sema3f was demonstrated to inhibit angiogenesis (Kessler et al., 2004), metastasis (Bielenberg et al., 2004) and motility of primary tumor cells (Nasarre et al., 2003). Decreased expression of Sema3f correlated with advanced stage and more aggressive type of human NSCLC (Roche and Drabkin, 2001) that indicates its important role in lung carcinogenesis. At last, up regulation of Hgfac in c-Myc-induced tumors could result in activation of HGF, whose multiple functions include also a potent angiogenic capacity.

From the study the inferred link of c-Myc to angiogenesis is in agreement with an observation that - c-Myc had a potent angiogenic capacity. This was shown in skin (Pelengaris et al., 1999), pancreatic β-cells (Pelengaris et al., 2002) and lymphoma (Brandvold et al., 2000). The reversible activation of conditional alleles of c-Myc in these studies induced hyperproliferation, dedifferentiation and angiogenesis in a reversible manner, suggesting that these complex events may be directly affected by c-Myc.

### Deregulation of signaling pathways through scaffold proteins

Gene expression changes discussed above link c-Myc to the regulation of the main intra- und extracellular signaling pathways. To this question, in lung adenocarcinomas of c-Myc-transgenic mice significantly decreased expression of several scaffolding proteins involved in spatiotemporal regulation of signaling pathways was found. They included two known c-Myc responsive genes caveolin 1 and Akap12 (gravin), both proteins being frequently absent in a number of carcinomas (Williams et al., 2003), including lung cancer (Wikman et al., 2002). Caveolin-1 is the principal structural component of caveolae - the sites of membrane responsible for concentrating an array of signaling molecules including growth factor receptors and G-proteins as well as calcium channels and pumps, and thought to be a major regulator of signaling complexes in caveolae. The loss of caveolin-1 was demonstrated to confer a significant growth advantage to mammary epithelial cells in vitro and in vivo (Williams et al., 2004). Decreased expression of the c-Myc responsive genes caveolin-1 together with overexpression of FAS , points to an important role of derangement of the membrane rafts in the malignant transformation induced by c- Myc. The transformation suppressor potential of Akap 12 seems to be based on its ability to reorganize actin-based cytoskeleton architecture and control G1 phase signaling proteins (Gelman, 2002). Scaffold proteins guide the interactions between successive components of signaling complexes to relay the signal with precision, speed and efficiency and to avoid an unwanted cross-talk between signaling pathways therefore providing for a specificity of cell response to many different signals. The loss of such spatiotemporal regulators of signaling processes in response to c-Myc could likely resulted in an amplification and spreading of proliferative signals in lung epithelial cells thereby accelerating malignant transformation.

### Conclusions

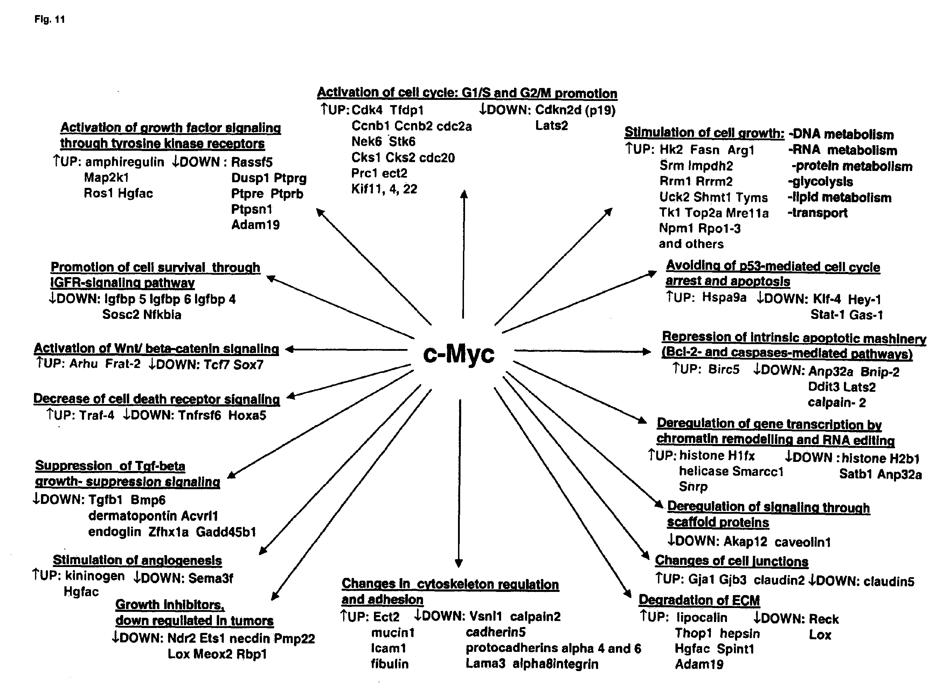

1. As reported in Part 3 and in Part 1 and 2 of the c-Myc oncogenomic study according to the invention, inappropriate expression of a variety of genes involved in the several pathways critical for the development of lung tumor was identified: cell cycle, cell growth, cell death, adhesion, cytoskeleton organization, invasive and angiogenic capacity (see Fig. 11 for a summary).
2. Some genes identified in this study were known to be similarly changed in human malignancies and specifically in lung tumors which makes the SPC/c-Myc-transgenic mouse model useful for investigating human lung cancer.
3. Many other genes differently expressed in lung tumors are novel targets for mechanism based therapies and serve as useful tumor markers.
4. New c- Myc-responsive genes and putative c-Myc -targets identified in this study contributed to the collective examination of distinct c-Myc transcriptomes that are partly cell type- and species- specific and therefore represent one more important result of this study.

c-Myc is a transcription factor and frequently overexpressed in diverse malignancies. It's targeted overexpression (>50-fold) in mouse alveolar epithelium induced lung cancer. To identify new myc target genes and to explore the molecular basis of c-Myc transforming capacity were investigated genome wide regulation of genes in histologically well defined tumors. Essentially, expression of 463 genes differed significantly when compared with healthy non-transgenic lung. Then promoters of regulated genes for c-Myc recognition sites to link c-Myc binding to gene expression were annotated and interrogated. Notably, many of the genes could be traced back to regulators of cell cycle and included c-Myc targets, e.g. Cdk4, Ccnb1, Cks2 in addition to c-Myc responsive genes, e.g. Tfdp1, Cdc2a, Stk6, Nek 6, Prc1, Ect2. Furthermore, Several genes whose regulation in tumors interfere with the p53- and Bcl2- mediated cell cycle arrest and cell death program were identified, notably Birc5, KLf4, Stat1, Ddit3, Lats2 therefore providing a selective advantage for tumor growth. There was considerable agreement among the regulation of c-Myc target genes in mouse and human lung malignancies. Notably, targeting new lung cancer genes i.e. Prc1, Klt4, Ect2, Hey-1, Gas-1, Bnip-2, calpain-2 and Anp32a may enable mechanism based therapies.

The c-Myc transcription factor regulates a wide set of genes in response to mitogenic stimuli. Its targeted overexpression in alveolar epithelial cells led to malignant transformation and development of papillary lung adenocarcinomas (PLAC) in mice. In the first part of the study cell cycle and apoptosis networks were investigated in papillary adenocarcinomas. Now regulation of genes coding for DNA and RNA metabolism and processing, ribosomal biogenesis and protein biosynthesis, nuclear and cellular transport, glycolysis, lipid metabolism and chromatin remodeling is reported. The c-Myc target genes Tyms, Tk, Srm, Fasn and the c-Myc-responsive genes Hells, Rpo1-3, Gpi1, Impdh were found to be significantly induced. Their regulation in PLAC could contribute to an accelerated cell proliferation and growth. Notably, increased expression of some orthologues in human lung carcinomas was observed as well. In addition, bioinformatics to search for c-Myc-binding sites in promoters of regulated genes were applied thereby identifying new c-Myc targets; these included, amongst others, Uck2, Smarcc1, Npm3, Nol5, Lamr1, next to the well established c-Myc targets Srm, Shmt1, Npm1, Slc19a1. These newly identified lung cancer genes are interesting targets for the development of novel antineoplastic therapies.

To better understand the molecular basis of c-Myc transforming capacity genome-wide expression analysis of lung adenocarcinomas in a transgenic mouse model was performed. In part 1and 2 of the study regulatory gene networks associated with cell cycle and apoptosis as well as tumor growth and metabolism was reported.

Here it was focussed on regulated genes involved in extracellular signaling pathways, e.g. activation of EGFR- mediated mitogenic, IGFR- mediated survival and Wnt-signaling as well as disruption of Fas-mediated death and anti-mitogenic Tgf-β-signaling. Additionally, altered gene transcription associated with cell adhesion, cell-cell junctions, regulation of cytoskeleton, proangiogenic signaling and invasion provided a molecular rationale for c-Myc invasive and angiogenic capacities. An identification of novel c-Myc target genes associated with lung tumors (e.g., Rbp1, Reck) along with known c-Myc-responsive or c-Myc -target genes ( e.g., Akap12, Cav1, Hoxa5, Lox, Muc1) and genes linked to lung cancer (e.g., Rassf5, Hgfac, Sema3f, Ptprb), provide highly interesting targets for mechanism based therapies.

### Materials and methods

### Maintainance of the transgenic mouse line

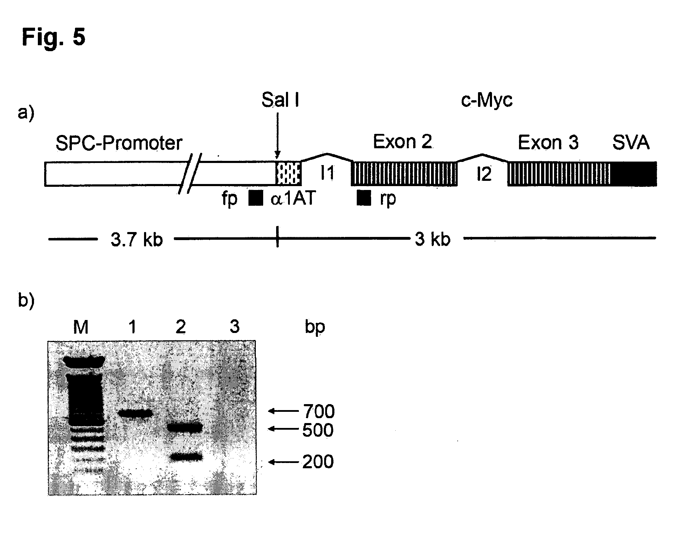

SPC/myc transgenic mice (Ehrhardt et al., 2001) were maintained as hemizygotes in the CD2F1-(DBA/2xBalb/C) background and identified by PCR of DNA extracted from tail biopsies (Hogan et al., 1994) (Fig. 5a, b). PCR was carried out using Platinum PCR SuperMix (InVitrogen) with the primer pair for the c-Myc- transgene: 5'-CAGGGCCAAGGGCCCTTGGGGGCTCTCACAG, 3'-GGACAGGGGCGGGGTGGGAAGCAGCTCG:

### Sample collection and preparation

Mice were anasthesized by an overdose of CO₂ at the age of 9 - 13 months. The tumors were inspected macroscopically, separated from the surrounding lung tissue of SPC/myc transgenic mice and frozen immediately in liquid nitrogen. The tumors were divided into groups according to size 1 mm, 5 mm and >10 mm. Small tumors were pooled in three groups of 3, 3 and 4 separate tumors because of the low yield of RNA. Normal lung from four non transgenic mice of about the same age were used as a control.

### Histology

For histopathology tissue specimen of 5 transgenic animals and 2 non-transgenic controls at the age of 9 to 13 months were investigated. The specimens were rinsed in PBS and fixed in 4 % PBS-buffered formaldehyde, and processed for paraffin embedding as usual. 5 µm thick serial sections were stained with hematoxylin and eosin (H and E), hematoxylin only (H) and PAS for light microscopic evaluation.

### Isolation of RNA, production of c-RNA, array hybridization and scanning.

The cRNA-samples were prepared following the Affymetrix Gene Chip® Expression Analysis Technical Manual (Santa Clara, CA). 10 µg of biotinylated fragmented cRNA was hybridized onto the Murine Genome U74Av2 Array (MG-U74Av2) - a GeneChip® expression oligonucleotide probe array from Affymetrix which contains 12 488 probe sets, that represents annotated sequences in the Mouse UniGene database as well as EST clones. The procedures for isolation of RNA, production of c-RNA, array hybridization and scanning were done according to Affymetrix's manual and as described (Borlak et al., 2005). Each hybridization image was scaled to all probes set intensity of 250 for comparison between chips.

### Gene expression data analysis

The data analysis was perfomed basically as described earlier (Borlak et al., 2005). For single arrays a statistical expression algorithm within the Affymetrix® Microarray Suite software (version 5) yielded gene expression values as numeric values (signal intensities) and detection calls ("Present " or "Absent") produced with different algorithms. A comparison between two arrays (tumor and control lung) resulted in the signal logarithm ratio (log2ratio) and a change call ("Increase" or "Decrease") for expression level of each gene. Data from replicate samples were evaluated and compared using statistical analyses with the Affymetrix® Data Mining Tool 2 (DMT-2). To summarize the expression level (the signal values) for each transcript across the replicates were used the average and standard deviation statistics. Mean fold change values were calculated as the ratio of the average expression levels for each gene between two tissue sets. To determine significance of change in mean gene expression level between comparison groups the unpaired two-sided T-Test was used, with the p-value cutoff determined as 0.05. Additionally to parametric T-test, which uses for analysis numeric expression values (signal intensities), comparison ranking analysis was done to study the concordance between "Increase" or "Decrease" calls for tumors in one set. The results are shown as % of concordance between all comparisons, e.g. 16 analyses (4 tumors versus 4 controls) for tumors of median size and 12 (3 tumors versus 4 controls) for tumors of large size. Three pools of small tumors (1 mm) were compared with the 4 individual controls, resulting in 12 comparisons. The group of differently regulated genes in tumors was restricted to those genes, which were detected ("Present" call) in all samples of a tumor sets for the up regulated genes and in all control lungs for the down regulated genes. Further criteria were FC≥ 3, p-value in T-test ≤ 0,05 and 100% of "Increase" call in comparative ranking analysis for one or more sets of tumors versus control lungs for up regulated genes and FC≤- 3, p-value in T-test ≤ 0,05 and 100% of "Decrease" call accordingly for down regulated genes.

### Gene expression studies by RT-PCR

The primer 3 software (Rozen S and Skaletsky HJ, 2000) was used to design the primers. The amplification fragments spanning an intron were chosen to distinguish between amplification from contaminating DNA.

Total RNA was isolated with the Qiagen RNA purification kit according to the manufacturers instructions. Reverse transcription was carried out using Omniscript (Qiagen), Oligo-dT primers (InVitrogen) and RNasin (Promega) followed by PCR amplification (see above) with the following primer pairs: Ccnb1, fp: CAGTTGTGTGCCCAAGAAGA; rp: TCCATTCACCGTTGTCAAGA (57°, 32 cycles). Cdc2a, ,fp:CTCGGCTCGTTACTCCACTCGAGCATCAAGAAAGAGGTCAAAGG;rp: CCATTTTGCCAGAGATTCGT (56°C, 34 cycles). Stk6:fp:GCCCACTAGGAAAAGGGAAG. rp: CGTTTGCCAACTCAGTGATG (56°C, 34 cycles). Cdk4, fp: AACTGATCGGGACATCAAGG, rp:CACGGGTGTTGCGTATGTA (58°C, 30 cycles). Nek6, fp:TTGAGATGATGGATGCCAAA, rp: AGCTGTGATGAACACGTTGG (56°C, 32 cycles). Prc1, fp: CATGATGCCGAGATTGTACG, rp: CAGCCGATGTAATTCCCACT (57°C, 32 cycles). Birc5, fp: GAATCCTGCGTTTGAGTCGT, rp:CAGGGGAGTGCTTTCTATGC (56°C, 38 cycles). Ddit3, fp:CTGCCTTTCACCTTGGAGAC, rp: GGGCACTGACCACTCTGTTT (58°C, 34 cycles). Satb1 fp: GTGATGGCTCAGTTGCTGAA, rp:CATAGCCCGAAGGTTTACCA (57°C, 32 cycles). Hey1 fp:GAGACCATCGAGGTGGAAAA, rp: ACCCCAAACTCCGATAGTCC (58°C, 32 cycles); β-actin fp:GGCATTGTTACCAACTGGGACG, rp: CTCTTTGATGTCACGCACGATTTC (65°C, 23 cycles). β-actin was used as a housekeeping gene because of the equal expression in all lung samples. PCR reactions were done using Taq Platinum PCR Super-Mix Kit (Invitrogen), and amplification products were separated on 1% agarose gels. A semiquantitative measurement of band intensities was done using Kodak 1D Image Analysis Software. The gene expression values in each samples were normalized to the beta-actin expression and then used to calculate mean expression values for the sets of tumors of various sizes and control lungs. The mean fold changes was computed as a ratio between mean gene expression values for tumor sets and control non transgenic lung as a baseline.

### Western blotting

Frozen lung tumors of about 0.5 - 1 cm from three different SPC/c-Myc transgenic mice and non transgenic lung from three control mice were thawed on ice. Protein extracts were prepared by sonications of the samples in 500 µl 2D-loading buffer with the following incubation with benzonase according to (Molloy et al., 1999) and stored at - 80°C. 75 µg (c-Myc) or 100 µg (Hspa9a, Nek6) of total protein extracts were separated on 10% (Hspa9a), or 12.0 % SDS-polyacrylamide gel (c-Myc, Nek6) and blotted onto PVDF membranes in 25 mM Tris and 190 mM Glycin at 4°C for 2 h (10%, 12% gel) at 350 mA. Specific antibodies to detect the selected proteins were anti-c-Myc rabbit polyclonal (1: 500), anti-GRP75 (Hspa9a) goat polyclonal (1:200) purchased from Santa Cruz and anti-NEK6 rabbit polyclonal (1: 100) purchased from Abgent. Antigen-antibody complexes were visualized using the ECL detection system as recommended by the manufacturer NEN Life Science Products (PerkinElmer Life Science, Rodgau-Juegesheim, Germany) and recorded with Kodak IS 440 CF (Kodak, Biostep GmbH, Jahnsdorf, Germany).

### Bioinformatic search for potential c-Myc-binding sites in 5'-UTR region of the differently expressed genes in tumors.

In order to identify c-Myc targets among genes differently regulated in lung adenocarcinoma were applied positional weight matrices (PWMs) that is the most widely used method for recognition of transcription factor binding sites (Quandt et al., 1995) in a bioinformatic analysis of putative promoter sequences of deregulated genes. For that purpose TRANSFAC® Professional rel. 8.3 database was used which contains the largest collection of weight matrices for eukaryotic transcription factors (Wingender et al., 2001) (http://www.biobase.de/). The UCSC Mouse Genome Browser was used to extract the putative promoter regions of the corresponding genes (www.genome.ucsc.edu/cgi-bin/hgGateway). The beginning of the first exon was considered to be a tentative TSS (transcription start site), and 2000 bp upstream and 100 bp downstream of TSS were extracted. For the analysis of the extracted sequences a search profile which included five PWMs for c-Myc transcription factor with accession numbers M01034, M00322, M00118, M00123, M00615 was applied. The MATCH™ algorithm calculating scores for the matches by using the so-called information vector (Kel et al., 2003) was employed. The matrix similarity cut-offs for the matrices were set to different values (1.0, 0.96, 0.93, 0.98, 0.995 respectively), so that the rate of false positive matches for each matrix was less than 1 per 10,000 bp.

Maintenance of the transgenic mouse line, sample collection and preparation, isolation of RNA, production of c-RNA, array hybridization and scanning, gene expression data analysis, bioinformatic search for c-Myc-binding sites in 5'-UTR region of differently expressed genes in tumors, gene expression studies by RT-PCR and Western blotting were perfomed as described in the first part of the study.

RT- PCRs were performed with the following primer pairs (fp;rp):
Shmt1, fp.GCAACTCTGAACCAGTGCAA, rp:TGAGGATCCAGATGGTAGGC (56°C, 36 cycles). Satb1, fp:GTGATGGCTCAGTTGCTGAA, rp:CATAGCCCGAAGGTTTACCA (57°C, 32 cycles); Arg1, fp:
AAGCTGGTCTGCTGGAAAAA, rp: CTGGTTGTCAGGGGAGTGTT,(55°C, 36 cycles); Fasn, fp: TCTGCAGAGAAGCGAGCATA, rp: GTCATTGGCCTCCTCAAAAA (55°C, 31 cycles); Srm, fp: GTCCAGTGCGAGATTGATGA, rp:
GCAGAAGTGCCTCATCTCCT (57°C, 32 cycles); Hk2, fp:
GGTGGAGATGGAGAACCAGA, rp: TCATTCACCACAGCCACAAT (55°C, 32 cycles); Tk1, fp: CCAGATCGCCCAGTACAAGT, rp: GAAGCACTCCATGCACACAG
(59°C, 34 cycles); Impdh2, fp: GGAAGTGGTTCCATCTGCAT, rp:
TGGCTGCTGAGATGTTTGTC (57°C, 32 cycles); Uck2, fp:
CAGATCCCCGTGTACGACTT, rp: GTCGGCACCTCTAGGAATGA (59°C, 32 cycles); Smarcc1, fp: TGATCCAAGTCGCTCAGTTG, rp:
TCACAGCACACACATCTCCA (57°C, 32 cycles); Npm3, fp:
GGCTGCTTTAGCGTTCTTGA, rp: AAGGTGACAGGTGGTTGGAG (57°C, 33 cycles); Slc19a1, fp: ATGTGCATGTCCTGTGGAGA, rp:
AGGGAAGACGCAATCTGAAA (55°C, 37 cycles); β-actin was used as a housekeeping gene, because its expression was found to be unchanged in lung tumors of SPC/c-Myc mice (microarray analyses) compared with non-transgenic lungs: fp:GGCATTGTTACCAACTGGGACG, rp:
CTCTTTGATGTCACGCACGATTTC (65°C, 23 cycles).

Western blotting was done using specific antibodies for selected proteins: anti-Arg1 (1:100), anti-cShtm (1:100), anti-Hxkll goat polyclonal (1:100) and anti- Fasn rabbit polyclonal (1:100 ) antibodies puchased from Santa Cruz Biotechnology, Inc. Maintenance of the transgenic mouse line, sample collection and preparation, isolation of RNA, production of c-RNA, array hybridization and scanning, gene expression data analysis, bioinformatics search for potential c-Myc-binding sites in 5'-UTR region of the differently expressed genes in tumors, gene expression studies by RT-PCR and Western blot were performed as described.

RT- PCRs were performed with the following primer pairs:
Ros1, fp: CAGTGGCACACGGTACAATC; rp: CTCCGTGAAAGTCCAGCTTC (59°, 36 cycles). Ect2, fp: AGGACCTTCCATTCGAACCT. rp:
GACTCGGGTGTGTTGGAGAT (58°C, 34 cycles). Traf4: fp:
CGGCTTCGACTACAAGTTCC. rp: TAGGGCAGGGGACTACATTG (59°C, 34 cycles). Hgfac, fp: GCTTCCTGGGAAATGGTACA, rp:
CCTCTTGCCACAGGTAGGAC (58°C, 36 cycles). Adam19, fp:
TTTACCGCTCCCTGAACATC, rp: AGCAGTGTGCAGAATCATGG (57°C, 32 cycles). Bmp6, fp: TTCTTCAAGGTGAGCGAGGT, rp:
CTCGGGATTCATAAGGTGGA (57°C, 32 cycles). Acvrl1, fp:
CCACAACGTGTCTCTGATGC, rp: ACACTCTACCAGCGCAACCT (59°C, 32 cycles). Igfbp5, fp: CTACTCCCCCAAGGTCTTCC, rp:
TTGTCCACACACCAGCAGAT (57°C, 29 cycles). Sema3f, fp:
GCTTCCAGCCACACCTAGAG, rp: TACAGGTGTGTTCGGTTCCA (57°C, 34 cycles). Cav fp: GGGAACAGGGCAACATCTAC, rp:
GTGCAGGAAGGAGAGAATGG (59°C, 32 cycles). Cdh5 fp:
CAATGACAACTTCCCCGTCT, rp: TGTTTTTGCCTGAAGTGCTG (57°C, 29 cycles). β-actin was used as a housekeeping gene, because its expression was found to be unchanged in lung tumours of SPC/c-Myc mice (microarray analyses) compared with controls (non-transgenic lungs. The following primer pair was used:
fp:GGCATTGTTACCAACTGGGACG, rp: CTCTTTGATGTCACGCACGATTTC (65°C, 23 cycles).

Western blotting was done using specific antibodies for selected proteins: anti-Traf4 (1:100), anti-hepsin (1:100), anti-Alk1(=Acvrl1) (1:100 ) goat polyclonal antibodies and anti-Igfbp6 (1:100 ) rabbit polyclonal antibody puchased from Santa Cruz Biotechnology, Inc., anti-claudin2 (1:500) rabbit polyclonal antibody puchased from Zymed Laboratories Inc., and Adam19 (1:1000) rabbit polyclonal antibody puchased from Cedarlane Laboratories.

The characteristics of the invention being disclosed in the preceding description, the subsequent drawings and claims can be of importance both singularly and in arbitrary combination for the implementation of the invention in its different embodiments.

The foregoing description of preferred embodiments of the invention has been presented for the purposes of illustration and description. It is not intended to be exhaustive or to limit the invention to the precise form described, and many modifications and variations are possible in light of the teaching above. The embodiments were chosen and described in order to best explain the principles of the invention and its practical applications to thereby enable others skilled in the art to best utilize the invention in various embodiments and with various modifications as are suited to the particular use contemplated invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

### Titles and legends to the figures

### Fig. 1:

Histology of lung tumors of SPC/c-Myc-transgenic mice. Normal subpleural parenchyma of nontransgenic controls (a). Invasive papillary lung adenocarcinoma (PLAC) with maximal diameter of 220 µm besides foci of dispersed BAC: Real papilla with their own stroma (b). Advanced PLAC with folded papillary structures of secondary and tertiary degree (arrow = mitosos) (c). Liver metastasis of PLAC (d)

### Fig. 2:

Differently expressed genes in lung adenocarcinoma of c-Myc -transgenic mice: known responsiveness / interaction with c-Myc; proportion (%) of genes containing potential c-Myc-binding sites in promoter region
1 - known c-Myc-targets and their relatives
2 - known c-Myc-targets and their relatives containing potential c-Myc binding sites
3 - known c-Myc -responsive genes and their relatives containing potential c-Myc binding sites
4 - known c-Myc -responsive genes and relatives
5 - new c-Myc -responsive genes containing potential c-Myc binding sites
6 - new c-Myc -responsive genes

### Fig. 3:

RT-PCRs for selected genes: lanes 1-4: control lung ; 5-7: pools of small size tumors; 8-12: middle size tumors;13-15: large size tumors

### Fig. 4:

Western blot analysis for selected genes: C1-C-3 - control non-transgenic lung; T1-T3 - papillary lung adenocarcinomas of SPC/c-Myc transgenic mice

Fig. 5 Structure of the transgene (Ehrhardt et al., 2001): SPC-Promoter, human surfactant protein promoter; α1AT, first exon of the non coding alpha 1 antitrypsin gene; I1, intron 1 of the alpha 1 antitrypsin gene fused to the first intron of the c-myc protooncogene; I2, intron 2 of the c-myc protooncogene; SVA, SV40 Poly A signal. Primer binding sites within the transgene are indicated (black boxes); fp, forward primer; rp, reverse primer **(a).** PCR analysis of SP-C/myc from tail biopsies to identify transgenic mice. Lane 1-2: transgenic mice; Lane 2: amplified fragment of the transgene was digested with Sal 1 to obtain fragments of about 200 and 500 Bp; Lane 3: non transgenic mouse; M, molecular weight standard **(b).**

### Fig.6:

Up regulated genes involved in cell growth in c-Myc- induced lung adenocarcinoma: known responsiveness / binding to c-Myc; presence of c-Myc-binding sites in promoter region
1 - known c-Myc-targets and their relatives containing potential c-Myc binding sites
2 - known c-Myc-targets and their relatives
3 - known c-Myc -responsive genes and their relatives containing potential c-Myc binding sites
4 - known c-Myc -responsive genes and relatives
5 - new c-Myc -responsive genes containing potential c-Myc binding sites
6 - new c-Myc -responsive genes

### Fig.7:

RT-PCRs for selected genes: lanes 1-4: control lung ; 5-7: pools of small-sized tumors; 8-12: middle-sized tumors;13-15: large-sized tumors

### Fig.8:

Western analysis for selected genes: C1-C-3 - control non-transgenic lung; T1-T2-lung adenocarcinomas of SPC/c-Myc transgenic mice

### Fig. 9:

RT-PCRs for selected genes: lanes 1-4: control lung ; 5-7: pools of small size tumors; 8-12: middle size tumors;13-15: large size tumors

### Fig. 10:

Western analysis for selected genes: C1-C-3 - control non-transgenic lung; T1-T2 lung adenocarcinomas of SPC/c-Myc transgenic mice

### Fig. 11:

Overview of pathways with involved genes deregulated in c-Myc induced lung adenocarcinoma

Targeted overexpression of c-Myc to alveolar epithelium of mice induced multifocal non-invasive bronchioloalveolar carcinoma (BAC) and solitary invasive papillary lung adenocarcinoma (PLAC). Initially, we reported regulation of genes in papillary adenocarcinomas (Meier et al., part1-3). Here we report genes specifically regulated in BAC. Strikingly, only a few genes were commonly regulated in both subtypes of c-Myc-induced adenocarcinomas and included genes involved in cell cycle progression, e.g. Ccnb1, Cdc2a , Ect2, Shtm1 (induced), and lgf binding protein 2 with growth-modulating activity (repressed). The common regulationof these genes in BAC and PLACs is suggestive for their primary role in c-Myc-induced lung carcinogenesis. Notable, the majority of regulated genes differed for BAC and PLAC. Thus, activation of genes involved in angiogenesis like Angpt1, Calcrl, Edg3, and negative regulators of cell proliferation, e.g. Rasa1, Ptpns1, Mapk14, was found specifically in BAC. Repression of genes with growth - inhibitory functions like Tgfbr3, Lats2, Akap12 and induction of genes enhancing cell growth like Arg1, Hk2, Fasn, the proteases Adam19, Hgfac and hepsin were characteristic for PLAC. In conclusion, an identification of genes attributable to BAC and PLAC enables molecular finger printing of the two lung adenocarcinoma subtypes.

Lung cancer is a leading cause of death and a major public health problem throughout the world. Adenocarcinoma of the lung represents approximately 40-50% of all lung cancer cases and is the most prevalent form of lung cancer in younger males, women of all ages, in never smokers and in former smokers (Ramalingam et al., 1998). Histologically adenocarcinomas differ and are subdivided into distinct subtypes on the basis of predominant cell morphology and growth pattern, i.e. - acinar, papillary, bronchioalveolar and mucinous adenocarcinomas (Brambilla et al., 2001). Furthermore, lung carcinogenesis is a multistep process, characterized by the stepwise accumulation of genetic and epigenetic abnormalities, resulting in the selection of clonal cells with uncontrolled growth capacities and increased motility (Chung et al., 1995). The molecular imprinting of malignant growth has been shown to involve an imbalance in activation of protooncogenes, which drives cells to multiply and migrate and inactivation of genes coding for growth-inhibitory molecules. An identification of molecular lesions such as, hyperexpression of protooncogenes or silencing of tumor suppressors, are of great importance for proper diagnosis, prognosis and appropriate therapy of cancer. Notably, the c-Myc transcription factor is a well studied protooncogene with a central role in carcinogenesis (Bernard and Eilers, 2006). It exerts its effects through regulation of expression of genes involved in cell cycle, growth and apoptosis. Though c-Myc is part of the normal cell proliferation machinery, its hyperactivation leads to malignant transformation. We were particularly interested to investigate the role of c-Myc in lung cancer and studied the consequences of targeted over expression of c-Myc in alveolar epithelium of mice. Notably, lung tumors induced by c-Myc were heterogenous and could be classified as either multicentric non-invasive bronchioloalveolar carcinoma (BAC) or solitary invasive papillary lung adenocarcinoma (PLAC) (Part1). Thus, this mouse model mimics subtypes of lung adenocarcinoma, i.e. non-invasive BAC and invasive PLACs. This allowed us to study the pivotal role of c-Myc in lung carcinogenesis in different pathologies. By gene expression chip analysis of solitary invasive tumors, which we identified as invariably PLACs, novel c-Myc target genes associated with invasive lung tumors were identified (Meier et al., part 1-3). Here we report an identification of genes specifically regulated in non-invasive bronchioloalveolar carcinomas. Strikingly, regulation of genes differed mostly amongst BAC and PLAC when compared with control non-transgenic lung. Nonetheless, regulators of cell proliferation and adhesion were similarly activated in these lung carcinomas. Likely, they play a key role in c-Myc induced malignant transformation of lung. Certain genes were specifically activated in BAC and these included genes coding for angiogenesis and/or growth inhibition, whereas for PLAC suppression of a great number of genes coding for growth inhibitors and tumor suppressors was a characteristic feature. Besides, activation of genes involved in cell growth and proteolysis were specificat observed in PLAC and therefore may contribute to a more aggressive phenotype as reported herein. Therefore, oncogenomics enabled an identification of genes specifically associated with either BAC or PLAC adenocarcinomas.

### RESULTS

### Histology of lung tumors.

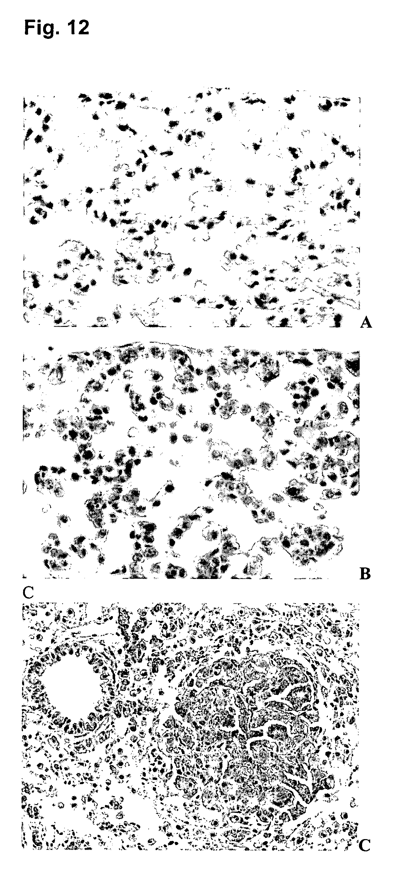

According to the WHO classification for histopathology of lung adenocarcinomas (Brambilla et al., 2001) lung tumors induced by targeted over expression c-MYC were classified as BAC and PLACs. In the first part of our studies (see Meier et al., 2006, Part1) a description of the tumor pathology is given. Unlike PLACs which are solitary tumors and thus could easily be isolated from lung tissue of transgenic mice, BAC growth was dispersed and multifocal. We specifically studied mice at the age of 8 months as they didn't contain macroscopically visible solid tumors (PLACs). This was confirmed by histopathology (Fig.12). Note, the healthy lungs of non-transgenic animals served as a control. Furthermore, transgenic males and females were examined separately to explore sex differences in the development of lung tumors. Indeed, females developed tumors more quickly than males, because 75% of c-Myc transgenic lung was occupied by BAC in females and 60% in males at this age).

### Oncogenomics of BAC

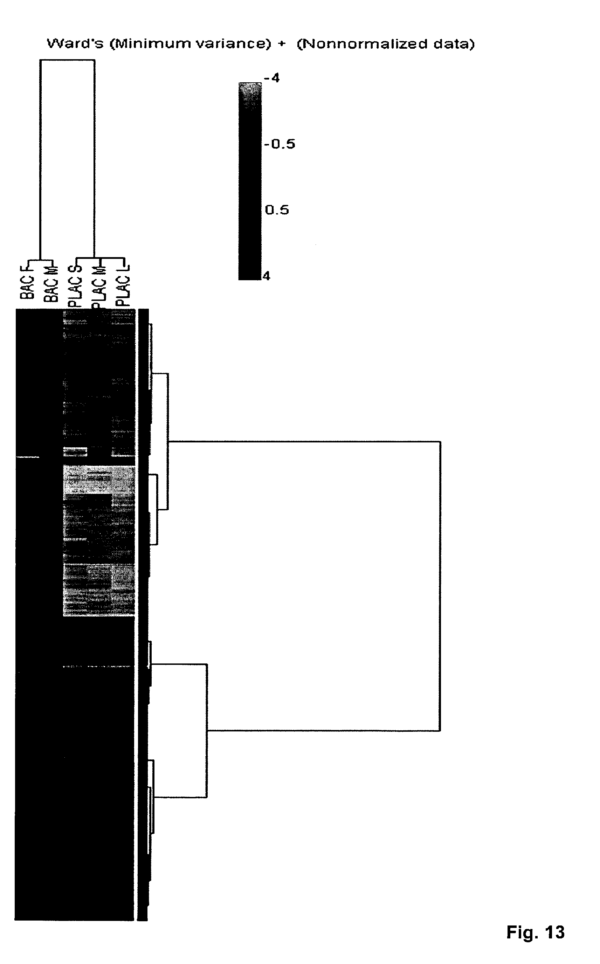

To study genetic events underlying the development of BAC we investigated genome wide transcript expression in the lungs of c-Myc -transgenic males and females at the age of 8 months. BAC growth was confirmed by histopathology. By use of the murine genome array 430 2.0 we identified 246 genes and ESTs differently expressed in transgenic lungs as compared with healthy non- transgenic lung (see Methods for the significant expression change criteria). Note 243 genes were up regulated but 3 genes were repressed. Gene expression changes were more prominent with females resulting in 241 significantly regulated genes. In males the same genes were regulated (Fig.13) but only 5 genes reached the threshold for significance testing (Table 8). The lower gene expression values observed with males agreed well the lesser occupancy of BAC growth in the lungs of transgenic mice, e.g. 75% in females versus 60% in males which indicated a sex specific difference in tumor growth. We previously reported oncogenomic of PLACs using MGU74Av2 microarray (Affymetrix) and yielded 162 genes which were significantly activated and 301 genes which were repressed in these tumors (see Meier et al., 2006 Part1). To identify differences in the regulation of gene transcription in these two subtypes of lung adenocarcinomas we compared expression of significantly regulated genes identified in BAC with that in PLAC and vice versa. As we employed the latest MG430 2.0 microarray representing a greater number of mouse genes (∼14000) than MGU74Av2 (∼6000) used in our previous analysis, only ∼ 2/3 of regulated genes identified in BAC were represented on MGU74Av2 and could therefore be compared with PLAC. Though the thresholds for significantly regulated genes in PLAC were defined more stringent and less mouse genes are represented on the MGU74Av2 array, we identified much more significantly regulated genes in PLAC than in BAC. Altogether, 595 genes significantly regulated in BAC and/or PLAC were taken into comparison. To obtain a comprehensive comparison of gene expression in BAC and PLAC, a hierarchical clustering algorithm was applied to the sum of genes differently expressed in BAC and/or PLAC (Fig.13). Fold changes of gene expression in BAC, in females and males, and PLAC, in small-, middle-and large- sized tumors were used for the comparison (Fig.13). Strikingly, the gene expression profiles of BAC and PLAC were quite different, with only few genes being similarly regulated in these subtypes of lung adenocarcinoma when compared with control non-transgenic lung. Table 8 gives an account of genes differently expressed in BAC and/or in PLAC where they are divided into groups according to the specific regulation in BAC and PLAC. Only selected genes with inferred importance for tumor biology are given accompanied by fold changes of gene expression level and results of statistical analyses. Importantly, we identified only few genes which were similarly up regulated in BAC and PLAC. These genes included positive regulators of cell cycle like c-Myc itself, cyclin b1, Cdc2a and the proto-oncogene Ect2, the enzyme Shtm1 favouring DNA biosynthesis, the Hgf receptor c-Met, the adhesion molecule CD44, and the lipase related Pnliprp1 amongst others. Repression of Igfbp2 involved in modulation of survival signalling was also identified in both subtypes of tumors. In contrast, expression of most genes significantly induced in BAC was diminished in PLAC. Mostly, these genes were only slightly induced or not regulated or even repressed in PLAC when compared with non-transgenic control lung. These included, amongst others, genes coding for angiogenesis like Hif1, Angpt1, Calcrl, Edg3, as well as negative regulators of growth, e.g., Tgfbr3, Rasa1, Ptpns, Sgpl1 and the pro-apoptic Mapk14 (=p38MAPK). Among those genes with opposite regulation in their expression in BAC and PLAC we found the adhesion molecules like integrin alpha 8, Cd38 and cytoskeletal proteins utrofin and vinculin as notable examples. Numerous genes which we previously found to be regulated in PLAC (Part1-Part3) were not changed in BAC. For example, genes up regulated in PLACs which code for proteins in cell division and growth, e.g. Cks1, Cks2, Nek6, kinesins Kif11, Kif 22 Kif4, arginase1, spermidine synthase, Impdh2, Uck2, hexokinase 2, lactate dehydrogenase, fatty acid synthase, were expressed in BAC similar to control lungs as were several translation initiation factors and cell and nuclear transporters. Besides, they included several proteases like Hgf activator, hepsin, Adam19, Thop1 and genes involved in pro-growth signaling like proto-oncogene Ros1, autocrine growth factor amphiregulin, Arhu, kininogen which therefore may contribute to the advanced tumor phenotype. Notably, among numerous genes repressed in PLAC and not changed in BAC we identified the growth inhibitory and tumor suppressors, e.g., Gas1, Septin4, Bmp6, Acvrl1, Akap12, Bnip2, Cav1, Capn2, Ddit3 , Hey1, Igfbp6, Lats2, Lox , Ptprd, Sema 3f, several serpins and others (Part 1, 3). As was noted above, approximately one third of regulated genes identified in BAC have no reference to PLAC, because these genes were not represented on the MG U74Av2 as yet. These genes up regulated in BAC included, amongst others, mitotic kinase Pbk (PDZ binding kinase), Pdgfd, Tmem23, Eaf1, Amd1 and dCMP deaminase with important roles in cell proliferation and growth, Akt3 (v-akt murine thymoma viral oncogene homolog) involved in survival signaling, Sgk3 closely related to Akt and adducin3 mediating actin driven cell movement. Other genes like the mitotic spindle checkpoint Bub1b, the stress response protein Stch, Tlk1 b involved in repair of genomic damage, the transcription factor Klf6 thought to be a tumor suppressor were induced in BAC likely as a part of a feed-back mechanism controlling cell growth and proliferation.

### DISCUSSION

### 1. Genes activated in either c-Myc-induced subtype of lung adenocarcinoma code for essential regulators of cell proliferation

Comparison of gene expression profiles of c-Myc- induced BAC and PLAC revealed that only a limited number of genes were induced in both subtypes of lung adenocarcinoma when compared with healthy lung of non-transgenic mice (Table8.1a, b). Notably, some of these genes were expressed at higher level in PLAC than in BAC (Table8.1a) like the c-Myc transgene itself which was induced 3-5-fold in lungs with BAC and 23- to 58- fold in solid PLAC. For comparison, high transgene expression in tumor cells were also observed in EGF-transgenic liver tumors (Tonjes et al., 1995) and c-Raf-transgenic lung tumors (Kerkhoff et al., 2000), though the nature of this fact remains unclear. Obviously, as tumor growth progresses, expression of the transgenic oncogene increases. Likely, expression of the oncogenic transgene may initially be kept under control, for instance, by recruitment of transcriptional repressors. In advanced stages of tumor growth the ability to suppress activation of the transgene may become less efficient because of impaired control mechanisms or/and autocrine stimulation by oncogene, e.g. c-Myc, of its own transcription through activation of appropriate signaling circuits giving rise to high level of transgene expression. Most genes whose expression was coherent with c-Myc expression coded for proteins essential for cell cycle progression. Thus, genes significantly induced in BAC and still stronger upregulated in PLAC were cyclin b1 (Ccnb1) and its kinase Cdc2a forming the complex which initiates the M phase of cell cycle. Notably, cyclin b1 is the known target for c-Myc, and Cdc2a is a known responsive gene for c-Myc (Born et al., 1994); (Guo et al., 2000). Besides, others included Ect2, a protooncogene involved in cytokinesis and regulation of cytoskeleton thereby contributing to cell transformation (Saito et al., 2004), (Westwick et al., 1998). Shmt1, a key enzyme in the folate metabolism, was shown to be a metabolic switch that, when activated, enables preferential synthesis of DNA precursors rather then DNA methylation cofactor (Herbig et al., 2002). Importantly, Shmt1 and Ccnb1 transcripts were not detected in any of the non-transgenic lungs, i.e., were uniquely expressed in lung tumors and represent, therefore, good candidates for both lung tumor markers and therapeutic targets. Evidence for increased expression of Shmt1 protein in PLAC was already provided in our previous report (see Meier et al., 2006 Part2). Repression of insulin-like growth factor binding protein lgfbp2 which prevent binding of insulin-like growth factor1 and 2 to their receptor (Carrick et al., 2005) could, therefore, enforce survival signalling, though its effect on proliferation can be opposite (Fisher et al., 2005); (Russo et al., 2005) and is, likely, context- dependent. We additionaly identified genes moderately increased in BAC and significantly induced in PLAC (Table8, 1 a) which are associated with cell cycle progression and proliferation. These were the cell cycle regulators Cks2, Ccnb2, Aurka and Cdc20 and nuclear antigen Ki67 widely used as a proliferation marker (Urruticoechea et al., 2005). Other included Top2a, an enzyme that controls and alters the supercoiling of DNA during replication and transcription, phosphoserine aminotranspherase Psat1 found to be expressed at the highest level in S-phase of cell cycle (Baek et al., 2003), folate transporter Slc19a1 (Shen et al., 2005) and Birc5 with dual role in inhibition of apoptosis and stimulation of cytokinesis (Wang et al., 2005a). Identification of the above named genes point to molecular events induced by c-Myc to enhance cell proliferation in PLAC as compared with BAC. Besides, the PDZ-binding kinase (Pbk), which is a novel mitotic kinase up regulated in a variety of highly proliferative malignant cells, fetal tissues and hematologic malignancies (Simons-Evelyn et al., 2001), (Nandi et al., 2004), and dCMP deaminase (Dctd) involved in pyrimidine biosynthesis were significantly induced in BAC (Table8, 1 c) and may well represent new targets of c-Myc to stimulate cell division. Among those genes whose expression was significantly increased in BAC and similarly changed in PLAC (Table8, 1 b) we identified two receptors for growth factors, i.e. the c-Met proto-oncogene (Met) and the fibroblast growth factor receptor 2 (Fgfr2), the hyaluronic acid receptor Cd44 and syndecan1 (Sdc1) with roles in cell proliferation, cell migration and cell-matrix interactions in addition to the lipase-related Pnilprp1. The pivotal role of c-Met, a tyrosine kinase receptor for hepatocyte growth factor (HGF), in oncogenesis, tumor progression and invasiveness is well recognized (Peruzzi and Bottaro, 2006). CD44, a receptor for hyaluronic acid, can interact with other ligands such as MMPs, collagens, osteopontin and therefore participates in a wide variety of cellular conditions including cytoskeletal modification and tumor metastasis (Bourguignon et al., 2004), (Bourguignon et al., 2006); (Pelletier et al., 2006). Sdc1 was shown to mediate HGF binding and promote Met signaling in myeloma (Derksen et al., 2002) as well as stimulate breast carcinoma growth and angiogenesis in vivo (Maeda et al., 2006) The role of Pnliprp1 in oncogenesis is unknown but strong up regulation of the related enzyme lipoprotein lipase (Lpl) was found in our study of Egf-induced hepatocarcinoma in mice (Borlak et al., 2005) and in poor risk B-cell chronic lymphocytic leukemia (Heintel et al., 2005). This suggests that overexpression of members of a gene family of lipases may be of great importance for inappropriate cell proliferation through as yet unestablished mechanism.. Other genes with this expression pattern included Na ⁺/K ⁺/Cl ⁻-cotransporter Slc12a2 which was demonstrated to activate ERK-siganiling cascade (Panet et al., 2006) and Narg1, a component of the protein N-alpha-acetyltransferase complex which was recently found to play an essential role in cell survival (Arnesen et al., 2006) and was over expressed in papillary thyroid carcinoma (Fluge et al., 2002).

Increased expression of some genes with growth-, metastasis- and angiogenesis supressor functions like Ppp2r5c, Gja1, Rrm1 and Nedd 4 may be part of an anti-tumor response in lung cancer.

Thus, the comparison of genes differently expressed in BAC and PLAC proved to be instrumental in identifying particular genes with similar regulation in different subtypes of c-Myc-induced lung adenocarcinomas which suggest their primary role in c-Myc-trigged lung carcinogenesis.. Tracing individual genes specific for tumor pathologies enables an identification of disease candidate genes.

### Genes up regulated in BAC contributing to cell growth

Several new genes which may prove to be essential in lung cancer development were identified in BAC by use of the MG 430 2.0 array (Table8.1c). We identified regulation of the thymoma viral proto-oncogene 3 (Akt3), the platelet-derived growth factor, D (Pdgfd), the sphingomyelin synthase 1 (Tmem23=Sms1), the Adducin 3 (Add3), the S-adenosylmethionine decarboxylase 1 (Amd1), and the ELL associated factor 1 (Eaf1). It is well established that the AKT family plays a central role in carcinogenesis, (Testa and Bellacosa, 2001) and facilites growth factor-mediated cell survival and blocking of apoptic cell death. Note, up regulation of the Akt 3 isoform was found in breast and prostate cancer (Nakatani et al., 1999). Furthermore, PDGFs disregulation contributes to numerous proliferative disorders, with Pdgfd being tumorigenic in mice and was found to be specifically over expressed in lung cancer (LaRochelle et al., 2002). Likewise, Tmem23 stimulates cell growth by synthesis of pro-survival signalling molecule diacylglycerol and depletion of the pro-apoptic mediator ceramid at the same time (Yamaoka et al., 2004). Amd1 provides a substrate for subsequent synthesis of polyamines, which are required for cell proliferation. Up regulation of Amd1 was found in endometrial cancers (Kabbarah et al., 2003). In addition, Eaf1 has a direct role in facilitating of overal rate of RNA polymerase II transcription (Kong et al., 2005). Finally, the adducin family of cytoskeletal proteins are known to mediate actin driven cell movements, and expression of this gene is strikingly restricted to the epithelial cell layer (Akai and Storey, 2002).

### 2. Genes with different regulation in BAC and PLAC (see also Table 8).

### 2.1 Genes significantly enhanced in BAC and repressed in PLAC included those coding for angiogenesis, growth inhibitory response and adhesion

Among genes whose expression was strongly induced in BAC but repressed in PLAC (Table8.2.1) we identified endothelial-specific growth factors angiopoietin 1 (Angpt1), (Zadeh et al., 2004), and the G-protein-coupled receptors, Edg3 (endothelial differentiation, sphingolipid G-protein-coupled receptor, 3) and Calcrl (calcitonin receptor-like receptor) which likely contribute to angiogenesis through increase of endothelial cell proliferation and survival (Fieber et al., 2006)_{[Sh1]}. Besides, genes whose expression was significantly increased in BAC and only slightly in PLAC (Table8.1b) included hypoxia-inducible factor1 Hif1a. Hif1 plays an essential role in cellular and systemic homeostatic response to hypoxia, directly activating transcription of genes involved in energy metabolism, angiogenesis, vasomotor control, apoptosis, proliferation and matrix production, and can also be induced by hypoxia-independent factors like growth factor receptor (e.g. Egfr) signaling (Yudoh et al., 2005). Notably, Hif1 a mediates angiogenic response through transcriptional activation of Vegf and other proangiogenic factors (Xu et al., 2005), (Fischer et al., 2005). Hif1 a is over expressed in many human tumors and in metastases, and is closely associated with a more aggressive tumor phenotype (Zhang et al., 2005). Its increased expression in tumor cells promoted tumor growth in vivo and is associated with bad prognosis in patients with NSCLC (Swinson and O'Byrne, 2006). Hif1 a was also shown to promote nonhypoxia-mediated proliferation in colon cancer cells and xenografts (Dang et al., 2006). Thus, Hif1a is a transcription factor that can directly transactivate genes important for the enhanced growth and metabolism. Activation of proangiogenic genes in BAC argues for the angiogenic capacity of c-Myc.

Furthermore, among genes activated in BAC but not in PLAC (Table8.2.1) we observed regulation of transcripts coding for anti-growth capacities, e.g. the receptor for TGFß family members Tgfbr3 (Copland et al., 2003), the negative regulator of WNT-signaling cascade Fzd6 (Golan et al., 2004), the metalloproteinase inhibitor Timp2 (Seo et al., 2006), the suppressor of RAS function Rasa1 (Vogel et al., 1988), Ptpns1 (Oshima et al., 2002) as well as pro-apoptic Mapk14(=p38MAP (Lenassi M and Plementitas A, 2006) and Sgpl1 (Reiss et al., 2004) amongst others. Activation of the growth-inhibitory genes, may represent a part of a feed-back regulatory response to an inappropriate activation of proliferative signaling induced by constitutive c-Myc over expression. This feedback mechanism was absent in PLAC to foster aggressive tumor growth. Notably, genes oppositely regulated in BAC and PLAC, i.e., induced in BAC and repressed in PLAC, included cytoskeleton coding genes Utrn, Vcl and adhesion molecules Itga8, Cd38. Opposite regulation of these genes may be related to different growth pattern and invasive behaviour of BAC and PLAC. For example, Itga8 was shown to inhibit proliferation and migration of mesangial cells (Bieritz et al., 2003)

Further, genes specifically regulated in BAC, but not in PLAC included the docking protein Gab1 utilized for Egfr- and c-Met signaling (Meng et al., 2005), the proto-oncogene Yes1 involved in integrin signaling (Klinghoffer et al., 1999), and the protease Adam 17 shown to mediate activation of Egfr in vivo in tumor development by nude mice (Borrell-Pages et al., 2003). Likewise, in BAC the apoptosis inhibitors Birc 4 (Tenev et al., 2005) and Api 5 (Kim et al., 2000), the proliferation-associated transferrin receptor 1 (Tfrc1) (Wang et al., 2005b) and the G- proteins Gna12 and Gna13 which interact with cadherins to release transcriptional activator β-catenin (Meigs et al., 2001) were significantly upregulated. It was also reported that Gna13 regulates cell growth and differentiation through the monomeric Rho GTPase (Grabocka and Wedegaertner, 2005). Activation of the genes discussed herein represents unique features of BAC oncogenomics.

### 2.2 Genes induced or repressed in PLAC but not regulated in BAC may contribute to a more aggressive and/or invasive phenotype of PLAC

We identified genes whose expression was significantly regulated in PLAC but unchanged in BAC (Fig.13). A description of genes regulated in PLAC was reported in Meier et al. (Part 1-3). Here we focus on a selected group of genes to demonstrate their distinct and characteristic expression pattern in BAC or PLAC (Table 8, 2.2). Genes whose expression was significantly up regulated in PLAC included those involved in various aspects of cell growth like cell cycle progression, e.g. Cks1, Nek6, polyamine synthesis, e.g. Arg1 and Srm1, nucleotide synthesis, e.g. Impdh2, Uck2 and Rrm2, glycolysis, e.g. Hk2, Ldh, lipid metabolism, e.g. Fasn, transcription, e.g. Rpo1-3, protein synthesis e.g. Eif2b, nuclear transport e.g. Nol5a, and pro-growth signalling, e.g. Ros1 proto-oncogene, amphiregulin, Arhu and kininogen. Activation of such genes involved in cell proliferation and growth together with induction of several proteases, like Adam19, Hgf activator, hepsin, Thop1 and matrix metalloproteinase regulator lipocalin may well represent the molecular switches for more aggressive growth of PLAC. Another specific feature of PLACs was down regulation of numerous genes which, amongst others, included many growth-inhibitory and tumor suppressor genes (see Results and Table 8 and Meier et al. 2006 Part 1, 3). This suggests that suppression of these genes in PLAC was not the result of direct action of c-Myc, but rather represents secondary effect of genetic or epigenetic perturbations upon c-Myc over expression in advanced stages of tumor growth. Genomic instability and chromosomal abnormalities are characteristic for c-Myc over expressing cells (Felsher and Bishop, 1999) and may be due to the accelerated cell cycle and abrogation of cell cycle checkpoints targeted by c-Myc.

### Conclusion

In conclusion, we investigated the oncogenomics of distinct subtypes of lung adenocarcinoma in a c-Myc transgenic mouse model. The lung tumors arise from the same progenitor cells, e.g. alveolar epithelium. Few solitary PLACs were observed in mice at much later time points than multi-centric BAC which we found dispersed throughout the whole lung. Furthermore, genome wide expression profiling revealed more gene expression changes in PLAC than in BAC with a number of genes linked to cell cycle progression being stronger induced in PLAC than in BAC therefore indicating enhanced cell proliferation in PLAC. Besides, genes activated or repressed in PLAC could be linked to a more aggressive phenotype to enable invasive and metastatic growth. Collectively, these observations allowed us to hypothesize that BAC is an earlier stage and PLAC an advanced stage of the lung adenocarcinoma induced by c-Myc. Similar suggestion concerning BAC and PLAC classification in humans were already proposed (Shimosato et al., 1982). Importantly, by genome wide expression profiling of different pathologies of c-Myc-induced lung tumors, e.g. non-invasive BAC and invasive PLAC, genes could be identified which represent, most likely, the molecular basis for malignant growth induced by c-Myc. Genes specifically regulated in BAC or PLAC can therefore be held responsible for the more aggressive phenotype observed with PLACs.

### Methods

### Maintainance of the transgenic mouse line and sample collection

SPC/Myc transgenic mice (Ehrhardt et al., 2001) were maintained in the C57BI/6 background and identified as described previously (Meier et al., 2006, Part1). To study the transcriptome of BAC total RNA was isolated from the lungs of the transgenic mice at the age of 8 months. Normal lungs of non-transgenic animals served as a control. Mice were anesthetized by an overdose of CO₂ and the lung were isolated, one part of each lung being shock frozen in dry ice and stored at-80 ° for further gene expression studies and the other fixed in 4% formaldehyde and processed for histological investigation as described previously (Meier et al., 2006, Part1). 16 c-Myc transgenic males and 16 females were studied separately and compared with equivalent group of non-transgenic animals. For microarray analyses, equal amount of total RNA isolated from 4 individual mice were pooled, i.e. 4 pools of RNA, each pool combining RNAs from 4 mice, were analysed with microarrays per each animal group.

### Isolation and microarray analysis of PLACs was described previously (Meier et al., 2006). Transcriptome analyses

Total RNA was isolated using the RNeasy total RNA isolation Mini Kit (Qiagen), pooled as described above, and a second clean-up of pooled RNA was done with the same RNeasy Kit. 8 mkg of total RNA were used to produce cRNA using One-Cycle Target Labeling and Control Reagents (Affymetrix) according to the Affymetrix Gene Chip® Expression Analysis Technical Manual (Santa Clara, CA, 2005). Purified cRNA was quantified and checked for quality using the NanoDrop ND-1000 and the Agilent 2100 Bioanalyzer, then cleaved into fragments of 35-200 bases by metal-induced hydrolysis.

10 µg of biotinylated fragmented cRNA were hybridized onto the GeneChip® Mouse Genome 430 2.0 array from Affymetrix, washed and stained according to the manufacturer's recommendation. The arrays were scanned using the GeneChip® Scanner 3000. Scanned images were analyzed with the GeneChip® Operating Software (GCOS), each image being scaled to the same all probe set intensity for proper comparison between chips. Default parameters provided in the Affymetrix data analysis software package were applied for analysis.

### Data analysis

### Identification of significantly regulated genes

Gene expression data analysis for a single microarrays and a comparison between two arrays were performed using GCOS software. It applies two different algorithms to produce for each gene on the array numeric expression value and detection call (Present, Absent) and, by comparison of two samples (=arrays), additionally a signal logarithm ratio and a change call ("Increase", "Decrease" or "No Change") .Statistical evaluation of replicate data was done with the Affymetrix® Data Mining Tool 3.1 (DMT). Fold change values were calculated as the ratio of the average expression levels for each gene between two tissue sets, i.e. BAC and normal lung, for males and females separately. To determine significance of gene expression changes in BAC compared with normal lung the unpaired two-sided T-Test with the p-value=0.05 as a threshold of significance was done using numeric gene expression values. Additionally, comparison ranking analysis was done to determine the concordance between change calls ("Increase" or "Decrease") for regulated genes in BAC derived from different mice. The results are shown in Table 8 as % of change calls in 16 analyses (each of 4 tumor pools versus each of 4 normal lung pools). The thresholds for significantly up/down regulated genes in BAC were defined as a mean FC>= 2,5/<=-2,5, a p-value in T-test <=0,05 and number of change calls >=87,5% (i.e. more than 14 out of 16) in comparative ranking in the group of transgenic females or males. The criteria for genes significantly up/down regulated in PLAC were defined more stringent and represented FC≥ 3 or <-3, p-value in T-test ≤ 0,05 and 100% of "Increase"/"Decrease" calls in comparative ranking in at least one group of tumors of different size, i.e. small- (n=3), middle- (n=4) and large-sized tumors (n=3), in comparison to normal lung (n=4) (Meier et al., 2006. Part1). Besides; both in BAC and PLAC analyses, we restricted our consideration for those up or down regulated genes, which were detected ("Present" call) correspondingly in all tumor or in all control lung replicates. In Table 8 the statistical values for all 10 PLACs are reported, whereas by hierarchical clustering the data for PLACs of different sizes were analysed separately (see below).

### Hierarchical gene cluster analysis,

In order to get an overall view of similarity and differences in regulation of gene expression in c-Myc-induced BAC and PLAC hierarchical gene cluster analysis implemented in ArrayTrack software (Tong et al., 2004) was done. Ward fusion algorithm with Euclidean distance was applied to the log transformed (with the base 2) ratios of gene expression values. The list of analysed genes combined all 597 genes significantly deregulated in BAC, males or females, and in PLAC, in small- middle- and large-sized tumors.

### REFERENCES

1. Akai J, Storey K. (2002). Expression of gamma-adducin is associated with regions of morphogenetic cell movement in the chick embryo. Mech Dev 119 Suppl 1: S191-S195.
2. Arnesen T, Gromyko D, Pendino F, Ryningen A, Varhaug JE and Lillehaug JR. (2006). Induction of apoptosis in human cells by RNAi-mediated knockdown of hARD1 and NATH, components of the protein N-alpha-acetyltransferase complex. Oncogene 25: 4350-4360.
3. Baek JY, Jun DY, Taub D and Kim YH. (2003). Characterization of human phosphoserine aminotransferase involved in the phosphorylated pathway of L-serine biosynthesis. Biochem J 373: 191-200.
4. Bernard S, Eilers M. (2006). Control of cell proliferation and growth by Myc proteins. Results Probl Cell Differ 42: 329-342.
5. Bieritz B, Spessotto P, Colombatti A, Jahn A, Prols F and Hartner A. (2003). Role of alpha8 integrin in mesangial cell adhesion, migration, and proliferation. Kidney Int 64: 119-127.
6. Borlak J, Meier T, Halter R, Spanel R and Spanel-Borowski K. (2005). Epidermal growth factor-induced hepatocellular carcinoma: gene expression profiles in precursor lesions, early stage and solitary tumours. Oncogene 24: 1809-1819.
7. Born TL, Frost JA, Schonthal A, Prendergast GC and Feramisco JR. (1994). c-Myc cooperates with activated Ras to induce the cdc2 promoter. Mol Cell Biol 14: 5710-5718.
8. Borrell-Pages M, Rojo F, Albanell J, Baselga J and Arribas J. (2003). TACE is required for the activation of the EGFR by TGF-alpha in tumors. EMBO J 22: 1114-1124.
9. Bourguignon LY, Gilad E, Brightman A, Diedrich F and Singleton P. (2006). Hyaluronan-CD44 interaction with leukemia-associated RhoGEF and epidermal growth factor receptor promotes Rho/Ras co-activation, phospholipase C epsilon-Ca2+ signaling, and cytoskeleton modification in head and neck squamous cell carcinoma cells. J Biol Chem 281: 14026-14040.
10. Bourguignon LY, Singleton PA, Diedrich F, Stern R and Gilad E. (2004). CD44 interaction with Na+-H+ exchanger (NHE1) creates acidic microenvironments leading to hyaluronidase-2 and cathepsin B activation and breast tumor cell invasion. J Biol Chem 279: 26991-27007.
11. Brambilla E, Travis WD, Colby TV, Corrin B and Shimosato Y. (2001). The new World Health Organization classification of lung tumours. Eur Respir J 18: 1059-1068.
12. Carrick FE, Hinds MG, McNeil KA, Wallace JC, Forbes BE and Norton RS. (2005). Interaction of insulin-like growth factor (IGF)-I and -II with IGF binding protein-2: mapping the binding surfaces by nuclear magnetic resonance. J Mol Endocrinol 34: 685-698.
13. Chung GT, Sundaresan V, Hasleton P, Rudd R, Taylor R and Rabbitts PH. (1995). Sequential molecular genetic changes in lung cancer development. Oncogene 11: 2591-2598.
14. Copland JA, Luxon BA, Ajani L, Maity T, Campagnaro E, Guo H et al. (2003). Genomic profiling identifies alterations in TGFbeta signaling through loss of TGFbeta receptor expression in human renal cell carcinogenesis and progression. Oncogene 22: 8053-8062.
15. Dang DT, Chen F, Gardner LB, Cummins JM, Rago C, Bunz F et al. (2006). Hypoxia-inducible factor-1 alpha promotes nonhypoxia-mediated proliferation in colon cancer cells and xenografts. Cancer Res 66: 1684-1936.
16. Derksen PW, Keehnen RM, Evers LM, van Oers MH, Spaargaren M and Pals ST. (2002). Cell surface proteoglycan syndecan-1 mediates hepatocyte growth factor binding and promotes Met signaling in multiple myeloma. Blood 99: 1405-1410.
17. Ehrhardt A, Bartels T, Geick A, Klocke R, Paul D and Halter R. (2001). Development of pulmonary bronchiolo-alveolar adenocarcinomas in transgenic mice overexpressing murine c-myc and epidermal growth factor in alveolar type II pneumocytes. Br J Cancer 84: 813-818.
18. Felsher DW, Bishop JM. (1999). Transient excess of MYC activity can elicit genomic instability and tumorigenesis. Proc Natl Acad Sci U S A 96: 3940-3944.
19. Fieber CB, Eldridge J, Taha TA, Obeid LM and Muise-Helmericks RC. (2006). Modulation of total Akt kinase by increased expression of a single isoform: requirement of the sphingosine-1-phosphate receptor, Edg3/S1 P3, for the VEGF-dependent expression of Akt3 in primary endothelial cells. Exp Cell Res 312: 1164-1173.
20. Fischer I, Gagner JP, Law M, Newcomb EW and Zagzag D. (2005). Angiogenesis in gliomas: biology and molecular pathophysiology. Brain Pathol 15: 297-310.
21. Fisher MC, Meyer C, Garber G and Dealy CN. (2005). Role of IGFBP2, IGF-I and IGF-II in regulating long bone growth. Bone 37: 741-750.
22. Fluge O, Bruland O, Akslen LA, Varhaug JE and Lillehaug JR. (2002). NATH, a novel gene overexpressed in papillary thyroid carcinomas. Oncogene 21: 5056-5068.
23. Golan T, Yaniv A, Bafico A, Liu G and Gazit A. (2004). The human Frizzled 6 (HFz6) acts as a negative regulator of the canonical Wnt. beta-catenin signaling cascade. J Biol Chem 279: 14879-14888.
24. Grabocka E, Wedegaertner PB. (2005). Functional consequences of G alpha 13 mutations that disrupt interaction with p115RhoGEF. Oncogene 24: 2155-2165.
25. Guo QM, Malek RL, Kim S, Chiao C, He M, Ruffy M et al. (2000). Identification of c-myc responsive genes using rat cDNA microarray. Cancer Res 60: 5922-5928.
26. Heintel D, Kienle D, Shehata M, Krober A, Kroemer E, Schwarzinger I et al. (2005). High expression of lipoprotein lipase in poor risk B-cell chronic lymphocytic leukemia. Leukemia 19: 1216-1223.
27. Herbig K, Chiang EP, Lee LR, Hills J, Shane B and Stover PJ. (2002). Cytoplasmic serine hydroxymethyltransferase mediates competition between folate-dependent deoxyribonucleotide and S-adenosylmethionine biosyntheses. J Biol Chem 277: 38381-38389.
28. Kabbarah O, Pinto K, Mutch DG and Goodfellow PJ. (2003). Expression profiling of mouse endometrial cancers microdissected from ethanol-fixed, paraffin-embedded tissues. Am J Pathol 162: 755-762.
29. Kerkhoff E, Fedorov LM, Siefken R, Walter AO, Papadopoulos T and Rapp UR. (2000). Lung-targeted expression of the c-Raf-1 kinase in transgenic mice exposes a novel oncogenic character of the wild-type protein. Cell Growth Differ 11: 185-190.
30. Kim JW, Cho HS, Kim JH, Hur SY, Kim TE, Lee JM et al. (2000). AAC-11 overexpression induces invasion and protects cervical cancer cells from apoptosis. Lab Invest 80: 587-594.
31. Klinghoffer RA, Sachsenmaier C, Cooper JA and Soriano P. (1999). Src family kinases are required for integrin but not PDGFR signal transduction. EMSO J 18: 2459-2471.
32. Kong SE, Banks CA, Shilatifard A, Conaway JW and Conaway RC. (2005). ELL-associated factors 1 and 2 are positive regulators of RNA polymerase II elongation factor ELL. Proc Natl Acad Sci U S A 102: 10094-10098.
33. LaRochelle WJ, Jeffers M, Corvalan JR, Jia XC, Feng X, Vanegas S et al. (2002). Platelet-derived growth factor D: tumorigenicity in mice and dysregulated expression in human cancer. Cancer Res 62: 2468-2473.
34. Lenassi M, Plementitas A. (2006). The role of p38 MAP kinase in cancer cell apoptosis. Radiol Oncol 40: 51-56.
35. Maeda T, Desouky J and Friedl A. (2006). Syndecan-1 expression by stromal fibroblasts promotes breast carcinoma growth in vivo and stimulates tumor angiogenesis. Oncogene 25: 1408-1412.
36. Meigs TE, Fields TA, McKee DD and Casey PJ. (2001). Interaction of Galpha 12 and Galpha 13 with the cytoplasmic domain of cadherin provides a mechanism for beta - catenin release. Proc Natl Acad Sci U S A 98: 519-524.
37. Meng S, Chen Z, Munoz-Antonia T and Wu J. (2005). Participation of both Gab1 and Gab2 in the activation of the ERK/MAPK pathway by epidermal growth factor. Biochem J 391: 143-151.
38. Nakatani K, Thompson DA, Barthel A, Sakaue H, Liu W, Weigel RJ et al. (1999). Upregulation of Akt3 in estrogen receptor-deficient breast cancers and androgen-independent prostate cancer lines. J Biol Chem 274: 21528-21532.
39. Nandi A, Tidwell M, Karp J and Rapoport AP. (2004). Protein expression of PDZ-binding kinase is up-regulated in hematologic malignancies and strongly down-regulated during terminal differentiation of HL-60 leukemic cells. Blood Cells Mol Dis 32: 240-245.
40. Oshima K, Ruhul Amin AR, Suzuki A, Hamaguchi M and Matsuda S. (2002). SHPS-1, a multifunctional transmembrane glycoprotein. FEBS Lett 519: 1-7.
41. Panet R, Eliash M and Atlan H. (2006). Na+/K+/Cl- cotransporter activates MAP-kinase cascade downstream to protein kinase C, and upstream to MEK. J Cell Physiol 206: 578-585.
42. Pelletier L, Guillaumot P, Freche B, Luquain C, Christiansen D, Brugiere S et al. (2006). Gamma-secretase-dependent proteolysis of CD44 promotes neoplastic transformation of rat fibroblastic cells. Cancer Res 66: 3681-3687.
43. Peruzzi B, Bottaro DP. (2006). Targeting the c-Met signaling pathway in cancer. Clin Cancer Res 12: 3657-3660.
44. Ramalingam S, Pawlish K, Gadgeel S, Demers R and Kalemkerian GP. (1998). Lung cancer in young patients: analysis of a Surveillance, Epidemiology, and End Results database. J Clin Oncol 16: 651-657.
45. Reiss U, Oskouian B, Zhou J, Gupta V, Sooriyakumaran P, Kelly S et al. (2004). Sphingosine-phosphate lyase enhances stress-induced ceramide generation and apoptosis. J Biol Chem 279: 1281-1290.
46. Russo VC, Schutt BS, Andaloro E, Ymer SI, Hoeflich A, Ranke MB et al. (2005). Insulin-like growth factor binding protein-2 binding to extracellular matrix plays a critical role in neuroblastoma cell proliferation, migration, and invasion. Endocrinology 146: 4445-4455.
47. Saito S, Liu XF, Kamijo K, Raziuddin R, Tatsumoto T, Okamoto I et al. (2004). Deregulation and mislocalization of the cytokinesis regulator ECT2 activate the Rho signaling pathways leading to malignant transformation. J Biol Chem 279: 7169-7179.
48. Seo DW, Li H, Qu CK, Oh J, Kim YS, Diaz T et al. (2006). Shp-1 mediates the antiproliferative activity of tissue inhibitor of metalloproteinase-2 in human microvascular endothelial cells. J Biol Chem 281: 3711-3721.
49. Shen M, Rothman N, Berndt SI, He X, Yeager M, Welch R et al. (2005). Polymorphisms in folate metabolic genes and lung cancer risk in Xuan Wei, China. Lung Cancer 49: 299-309.
50. Shimosato Y, Kodama T and Kameya T. (1982). Morphogenesis of Lung Cancer. Shimosato Y, Melamed M and Nettesheim P (eds). CRC Press: Boca Raton, pp. 65-89.
51. Simons-Evelyn M, Bailey-Dell K, Toretsky JA, Ross DD, Fenton R, Kalvakolanu D et al. (2001). PBK/TOPK is a novel mitotic kinase which is upregulated in Burkitt's lymphoma and other highly proliferative malignant cells. Blood Cells Mol Dis 27: 825-829.
52. Swinson DE, O'Byrne KJ. (2006). Interactions between hypoxia and epidermal growth factor receptor in non-small-cell lung cancer. Clin Lung Cancer 7: 250-256.
53. Tenev T, Zachariou A, Wilson R, Ditzel M and Meier P. (2005). IAPs are functionally non-equivalent and regulate effector caspases through distinct mechanisms. Nat Cell Biol 7: 70-77.
54. Testa JR, Bellacosa A. (2001). AKT plays a central role in tumorigenesis. Proc Natl Acad Sci U S A 98: 10983-10985.
55. Tong W, Harris S, Cao X, Fang H, Shi L, Sun H et al. (2004). Development of public toxicogenomics software for microarray data management and analysis. Mutat Res 549: 241-253.
56. Tonjes RR, Lohler J, O'Sullivan JF, Kay GF, Schmidt GH, Dalemans W et al. (1995). Autocrine mitogen IgEGF cooperates with c-myc or with the Hcs locus during hepatocarcinogenesis in transgenic mice. Oncogene 10: 765-768.
57. Urruticoechea A, Smith IE and Dowsett M. (2005). Proliferation marker Ki-67 in early breast cancer. J Clin Oncol 23: 7212-7220.
58. Vogel US, Dixon RA, Schaber MD, Diehl RE, Marshall MS, Scolnick EM et al. (1988). Cloning of bovine GAP and its interaction with oncogenic ras p21. Nature 335: 90-93.
59. Wang J, Chen G and Pantopoulos K. (2005b). Inhibition of transferrin receptor 1 transcription by a cell density response element. Biochem J 392: 383-388.
60. Wang J, Chen G and Pantopoulos K. (2005a). Inhibition of transferrin receptor 1 transcription by a cell density response element. Biochem J 392: 383-388.
61. Westwick JK, Lee RJ, Lambert QT, Symons M, Pestell RG, Der CJ et al. (1998). Transforming potential of Dbl family proteins correlates with transcription from the cyclin D1 promoter but not with activation of Jun NH2-terminal kinase, p38/Mpk2, serum response factor, or c-Jun. J Biol Chem 273: 16739-16747.
62. Xu Q, Briggs J, Park S, Niu G, Kortylewski M, Zhang S et al. (2005). Targeting Stat3 blocks both HIF-1 and VEGF expression induced by multiple oncogenic growth signaling pathways. Oncogene 24: 5552-5560.
63. Yamaoka S, Miyaji M, Kitano T, Umehara H and Okazaki T. (2004). Expression cloning of a human cDNA restoring sphingomyelin synthesis and cell growth in sphingomyelin synthase-defective lymphoid cells. J Biol Chem 279: 18688-18693.
64. Yudoh K, Nakamura H, Masuko-Hongo K, Kato T and Nishioka K. (2005). Catabolic stress induces expression of hypoxia-inducible factor (HIF)-1 alpha in articular chondrocytes: involvement of HIF-1 alpha in the pathogenesis of osteoarthritis. Arthritis Res Ther 7: R904-R914.
65. Zadeh G, Koushan K, Pillo L, Shannon P and Guha A. (2004). Role of Ang1 and its interaction with VEGF-A in astrocytomas. J Neuropathol Exp Neurol 63: 978-989.
66. Zhang Q, Tang X, Lu QY, Zhang ZF, Brown J and Le AD. (2005). Resveratrol inhibits hypoxia-induced accumulation of hypoxia-inducible factor-1 alpha and VEGF expression in human tongue squamous cell carcinoma and hepatoma cells. Mol Cancer Ther 4: 1465-1474.

### LEGENDS TO THE FIGURES

Fig.12. Histology of lung tumors of SPC/c-Myc - transgenic lungs Normal subpleural parenchyma of non transgenic animals (a). Dispersed and immediately subpleural densely packed BAC: hyperchromatic low columnar lining cells of the alveoli (b). Initial invasive papillary lung adenocarcinoma (PLAC) besides foci of dispersed BAC (c)

Fig.13. Hierarchical gene clustering different gene expression regulation in BAC and PLAC.
BAC F - BAC, females
BAC M - BAC, males
PLAC S- small-sized PLACs
PLAC M- middlel-sized PLACs
PLAC L- large-sized PLACs

**Table1. Statistic of genes differently regulated in SPC/c-Myc- induced lung tumors**

| | | | |
|---|---|---|---|
| Number of genes expressed in 4 control lung samples : 5269 | | | |
| Number of genes expressed in 10 lung tumor samples : 4706 | | | |
| **UP-regulated genes in tumors** | | Up regulated genes which promoters contain c-Myc binding sites | |
| S >70 & FC>3 & 100% P,I & p <0,05 in one or more sets of tumors | | | |
| all up regulated genes in tumors | **162** | **67** | **41,4%** |
| known direct c-Myc-targets (**T**) | 32 | 20 | 62,5% |
| relatives of known direct c-Myc-targets (**rT**) | 15 | 5 | 33,3% |
| known c- Myc- responsive genes ( **R** ) | 16 | 8 | 50% |
| relatives of known c-Myc-responsive genes ( **rR**) | 9 | 2 | 22,2% |
| new c-Myc -responsive genes | 90 | 32 | 35,6% |
| **DOWN-regulated genes in tumors** 100% P & S>70 in control lungs & FC<-3 & p<0,05 & 100%D in one or more sets of tumors | | Down regulated genes which promoters contain c-Myc binding sites | |
| all down regulated genes in tumors | **301** | **52** | **17,2%** |
| known direct c-Myc-targets (**T**) | 5 | 1 | 20% |
| relatives of known direct c-Myc-targets (**rT**) | 16 | 2 | 12,5% |
| known c- Myc- responsive genes (**R**) | 24 | 5 | 20,8% |
| relatives of known c-Myc-responsive genes ( **rR**) | 17 | 4 | 23,5% |
| new c-Myc responsive genes | 239 | 40 | 16,7% |

| | | | |
|---|---|---|---|
| S - expression signal intensity FC- mean fold change of expression values in tumors compared with the control lungs P- "Present" detection call in Affymetrix microarray analysis I -"Increase" change call in Affymetrix microarray analysis D- "Decrease" change call in Affymetrix microarray analysis p- p-value in T-test between sets of tumors and control lungs sets of tumors : tumors of small, middle and large size | | | |

**Table2 Gene expression signatures in c-Myc-induced lung papillary adenocarcinoma: genes involved in cell proliferation, growth and apoptosis: (a) - up regulated genes; (b) down regulated genes**

| ACC | Gene Symbol | Gene Title | | N | FC | | | % **Increase/Decrease**** | | | **p-value in T-test compared to control lung** | | |
|---|---|---|---|---|---|---|---|---|---|---|---|---|---|
| | | | | | size of tumors | | | size of tumors | | | size of tumors | | |
| | | | | | small | middle | large | small | middle | large | small | middle | large |
| **(a) Up regulated genes in C-Myc induced lung tumors** | | | | | | | | | | | | | |
| ***Cell proliferation:*** | | | | | | | | | | | | | |
| *Cell cycle promoting genes* | | | | | | | | | | | | | |
| G1/S | | | | | | | | | | | | | |
| **AA791962** | **Cdk4** | **cyclin-dependent kinase 4** | **T, R** | **2** | **2,7** | **2,1** | **2,9** | **100** | **100** | **100** | **0,00** | **0,10** | **0,02** |
| AI849928 | Ccnd1 | *cyc*/*inD1* | **R** | **2** | 1,6 | 1,5 | 1,4 | 100 | 56 | 58 | 0,00 | 0,05 | 0,04 |
| X72310 | Tfdp1 | transcription factor Dp 1 | **R** | | 3,3 | 2,5 | 3,1 | 100 | 88 | 100 | 0,01 | 0,06 | 0,02 |
| G2/M | | | | | | | | | | | | | |
| | | | | | | | | | | | | | |
| X64713 | Ccnb1 | **Cyclin B1** | **T,R** | | 22.2 | 9.4 | *12.8* | 100 | 88 | 66 | 0,03 | 0,16 | 0,33 |
| X66032 | Ccnb2 | cyclin B2 | **rT** | | 7,6 | 5,1 | *4,2* | 100 | 69 | 58 | 0,03 | 0,11 | 0,24 |
| M38724 | Cdc2a | cell division cycle 2 homolog A (S. pombe) | **R** | | 18,0 | 10,6 | *10,5* | 100 | 50 | 33 | 0,01 | 0,06 | 0,19 |
| | | | | | | | | | | | | | |
| AB025409 | Cks1 | CDC28 protein kinase 1 | **rT,rR** | | 9,1 | 6,6 | 7,0 | 100 | 100 | 100 | 0,01 | 0,03 | 0,02 |
| AA681998 | Cks2 | CDC28 protein kinase regulatory subunit 2 | **T,R** | | 10,1 | 6,0 | *5,8* | 100 | 100 | 100 | 0,04 | 0,08 | 0,24 |
| | | | | | | | | | | | | | |
| AW061324 | Cdc20 | cell division cycle 20 homolog (S. cerevisiae) | | | 8,3 | 5,1 | *4,5* | 100 | 94 | 91 | 0,05 | 0,14 | 0,19 |
| | | | | | | | | | | | | | |
| U80932 | Stk6 | **serine/threonine kinase 6** | | | 6,4 | 3,4 | *4,2* | 100 | 75 | 66 | 0,01 | 0,08 | 0,30 |
| AI846534 | Nek6 | NIMA (never in mitosis gene a)-related expressed kinase 6 | | | 3,1 | 3,2 | 3,5 | 100 | 100 | 100 | 0,00 | 0,01 | 0,01 |
| | | | | | | | | | | | | | |
| AA856349 | Prc1 | **protein regulator of cytokinesis 1** | | 1 | 6,0 | 3,0 | *4,2* | 100 | 44 | 66 | 0,00 | 0,04 | 0,23 |
| L11316 | Ect2 | ect2 oncogene | | | 10,3 | 4,6 | *4,4* | 100 | 94 | 100 | 0,02 | 0,12 | 0,20 |
| X82786 | Mki67 | antigen identified by monoclonal antibody Ki 67 | | | 21,6 | 8,0 | *11,6* | 100 | 75 | 66 | 0,05 | 0,12 | 0,26 |
| AJ223293 | Kif11 | **kinesin family member 11** | **T** | **l** | 4,3 | 2,9 | *3,3* | 100 | 50 | 33 | 0,02 | 0,14 | 0,30 |
| D12646 | Kif4 | kinesin family member 4 | **rT** | | 3,2 | 2,3 | *2,3* | 100 | 69 | 58 | 0,05 | 0,05 | 0,22 |
| AI131895 | Kif22 | kinesin family member 22 | **rT** | | 8,1 | 5,0 | *5,2* | 100 | 75 | 91 | 0,01 | 0,05 | 0,18 |
| *Inhibitors of antiproliferative signaling* | | | | | | | | | | | | | |
| AB013819 | Birc5 | **baculoviral IAP repeat-containing** 5 | | | 8,6 | 4,5 | *6,0* | 100 | 50 | 66 | 0,01 | 0,15 | 0,32 |
| D17666 | Hspa9a | heat shock protein, A | **T** | **Z** | 2,5 | 2,5 | 3,3 | 100 | 88 | 100 | 0,01 | 0,05 | 0,02 |
| ***Apoptosis*** | | | | | | | | | | | | | |
| **AF041377** | **Cideb** | **cell death-inducing DNA fragmentation factor, alpha subunit-like effector B** | | | **11,2** | **14,9** | **11,6** | **100** | **100** | **100** | **0,01** | **0,06** | **0,10** |
| AB021961 | Trp53 | transformation related protein 53 | **T,R** | **l** | 2,9 | 1,9 | 3,1 | 100 | 44 | 100 | 0,01 | 0,13 | 0,03 |
| AF076482 | Pglyrp | peptidoglycan recognition protein | | | 1,9 | 3,3 | 3,1 | 83 | 100 | 100 | 0,03 | 0,04 | 0,04 |
| *Transcriptions factors* | | | | | | | | | | | | | |
| M32057 | Zfp239 | **zinc finger protein 239** | | | 4,6 | 3,7 | 6,4 | 91 | 44 | 100 | 0,00 | 0,21 | 0,03 |
| AF049702 | Elf5 | E74-like factor 5 | | **l** | 5,6 | 6,1 | 6,1 | 100 | 100 | 100 | 0,00 | 0,02 | 0,03 |
| AB008174 | Tcf2 | transcription factor 2 | **rR** | | 3,0 | 2,8 | 3,2 | 100 | 100 | 100 | 0,00 | 0,00 | 0,00 |
| M62362 | Cebpa | *CCAAT*/*enhancer binding protein (C*/*EBP), alpha* | ***T, R*** | *Z* | *3,0* | 2,6 | 3,2 | 100 | 100 | 100 | 0,00 | 0,00 | 0,01 |
| **(b) Down regulated genes in c-Myc induced lung tumors** | | | | | | | | | | | | | |
| *Inhibitors of growth:* | | | | | | | | | | | | | |
| *Cell cycle control* | | | | | | | | | | | | | |
| U19597 | Cdkn2d | cyclin-dependent kinase inhibitor 2D (p19, inhibits CDK4) | | | *-1,9* | | -4,1 | 75 | | 83 | 0,08 | | 0,03 |
| AI849416 | Lats2 | large tumor suppressor 2 | | | -3,3 | -3,3 | -5,8 | 100 | 100 | 100 | 0,00 | 0,00 | 0,00 |
| *Apoptosis:* | | | | | | | | | | | | | |
| *p53-mediated pathway* | | | | | | | | | | | | | |
| U20344 | KIf4 | Kruppel-like factor 4 (gut) | **R** | | -8,8 | -8,7 | -13,2 | 100 | 100 | 100 | 0,01 | 0,01 | 0,01 |
| AJ243895 | Hey1 | hairy/enhancer-of-split related with YRPW motif 1 | | | -4,9 | -3,7 | -5,1 | 100 | 93 | 100 | 0,00 | 0,00 | 0,00 |
| U06924 | Stat1 | signal transducer and activator of transcription 1 | | | -2,6 | -3,2 | -4,8 | 100 | 100 | 100 | 0,01 | 0,01 | 0,01 |
| X65128 | Gas1 | growth arrest specific 1 | **R** | | -7,3 | -5,8 | -5,7 | 100 | 100 | 100 | 0,00 | 0,00 | 0,00 |
| *negative regulators of Bcl-2- mediated anti-apoptic pathway* | | | | | | | | | | | | | |
| AF035207 | Bnip2 | BCL2/adenovirus E1B 19kDa-interacting protein 1, NIP2 | | | -2,4 | -3,0 | -3,4 | 100 | 100 | 100 | 0,00 | 0,00 | 0,00 |
| D38117 | Capn2 | calpain 2 | | | -3,0 | -3,0 | -3,8 | 100 | 100 | 100 | 0,02 | 0,03 | 0,02 |
| U73478 | Anp32a | acidic (leucine-rich) nuclear phosphoprotein 32 family, member A Lats2 (s. cell cycle) | | | -1,7 | -3,6 | -3.8 | 66 | 100 | 100 | 0,29 | 0,00 | 0,01 |
| **X67083** | **Ddit3** | **DNA-damage inducible transcript 3** | **R** | | **-2,3** | **-3,5** | **-6,2** | **100** | **100** | **100** | **0,03** | **0,01** | **0,01** |
| *Transcription factors* | | | | | | | | | | | | | |
| **X74134** | **Nr2f1** | ***nuclear receptor subfamily* 2, *group F, member*** 1 | ***R*** | | **-3,1** | **-3,1** | **-3,1** | **100** | **87** | **100** | **0,00** | **0,00** | **0,00** |
| AF010133 | Madh6 | MAD homolog 6 (Drosophila) | | | -4,8 | -5,8 | -6,9 | 100 | 100 | 100 | 0,01 | 0,01 | 0,01 |
| **L35949** | **Foxf1a** | **forkhead box F1a** | | 1 | **-9,3** | **-12,6** | **-19,4** | **100** | **100** | **100** | **0,00** | **0,00** | **0,00** |
| AW121328 | Tbx3 | T-box 3 | | 2 | -10,6 | -10,6 | -10,6 | 100 | 100 | 100 | 0,00 | 0,00 | 0,00 |
| **AF035717** | **Tcf21** | **transcription factor 21** | | | **-9,4** | **-12,7** | **-31,6** | **100** | **100** | **100** | **0,01** | **0,01** | **0,01** |
| **X55123** | **Gata3** | **GATA binding protein 3** | | | **-5,7** | **-5,7** | **-5,7** | **100** | **93** | **100** | **0,01** | **0,01** | **0,01** |
| X94127 | Sox2 | SRY-box containing gene 2 | | | -16,4 | 1,0 | -6,1 | 100 | 62 | 100 | 0,00 | 0,99 | 0,01 |
| AF041847 | Crap | cardiac responsive adriamycin protein | | | -19,6 | -27,6 | -27,6 | 100 | 100 | 100 | 0,03 | 0,03 | 0,03 |
| **J03776** | **Trim30** | **tripartite motif protein 30** | | | **-3,4** | **-3,4** | **-3,4** | **100** | **93** | **100** | **0,02** | **0,01** | **0,01** |
| AI853712 | KIf7 | Kruppel-like factor 7 (ubiquitous) | | 2 | -3,1 | -2,6 | -3,4 | 100 | 93 | 100 | 0,00 | 0,01 | 0,01 |
| **D49473** | **Sox17** | **SRY-box containing gene 17** | | | **-5,8** | **-6,9** | **-8,6** | **100** | **100** | **100** | **0,01** | **0,01** | **0,00** |
| L35032 | Sox18 | SRY-box containing gene 18 | | | -5,5 | -6,6 | -6,3 | 100 | 100 | 100 | 0,00 | 0,00 | 0,00 |
| **U33629** | **Meis1** | **myeloid ecotropic viral integration site 1** | | | **-4,6** | **-4,6** | **-4,6** | **100** | **100** | **100** | **0,00** | **0,00** | **0,00** |
| **Y12293** | **Foxf2** | **forkhead box F2** | | | **-5,7** | **-5,7** | **-5,7** | **100** | **100** | **100** | **0,00** | **0,00** | **0,00** |

| | | | | | | | | | | | | | |
|---|---|---|---|---|---|---|---|---|---|---|---|---|---|
| T- known c-Myc-target R- known c-Myc responsive gene rT- relative of known c-Myc-target rR- relative of known c-Myc-responsive gene N- number of potential c-Myc binding sites in promotor sequence ¹- % Increase/Decrease - concordance of change calls in the pairwise comparisons (each tumor to each normal lung sample), by which genes are up or down regulated respectively | | | | | | | | | | | | | |

Bold gene cell indicates that gene was not detected in all control lungs (for up regulated genes).

Bold row indicates that gene was not detected in all control lungs and was expressed in all tumor samples (for up regulated genes) and inversely for down regulated genes.

**Table3. Fold changes of gene expression in lung tumors determined in RT-PCR and microarray analysis**

| Gene | Method | Mean FC | | |
|---|---|---|---|---|
| | | size of tumors | | |
| | | small | middle | large |
| Cdk4 | RT-PCR | 3.0 | 2.0 | 1.4 |
| | Affymetrix | 2.7 | 2.1 | 2.9 |
| Stk6 | RT-PCR | 6.6 | 3.1 | 4.1 |
| | Affymetrix | 6.4 | 3.4 | 4.2 |
| Nek6 | RT-PCR | 3.0 | 2.6 | 2.9 |
| | Affymetrix | 3.1 | 3.2 | 3.5 |
| Ccnb1 | RT-PCR | 9.5 | 4.0 | 5.4 |
| | Affymetrix | 22.2 | 9.4 | 12.8 |
| Cdc2a | RT-PCR | 13.3 | 6.5 | 8.3 |
| | Affymetrix | 18.0 | 10.6 | 10.5 |
| Prc1 | RT-PCR | 8.0 | 5.1 | 5.5 |
| | Affymetrix | 6.0 | 3.0 | 4.2 |
| Birc5 | RT-PCR | 10.1 | 4.8 | 4.9 |
| | Affymetrix | 8.6 | 4.5 | 6.0 |
| Hey1 | RT-PCR | A | -1.9 | A |
| | Affymetrix | A | -3.7 | A |
| Ddit3 | RT-PCR | -4.0 | -3.5 | -5.9 |
| | Affymetrix | A | A | A |

| | | | | |
|---|---|---|---|---|
| A - "Absent" - no expression was detected | | | | |

**Table 4 Gene expression signature in c-Myc-induced lung papillary adenocarcinoma: differently expressed genes* involved in stimulation of cell proliferation and growth**

| ACC | Gene Symbol | Gene Title | | N | FC | | | % **Increase/Decrease**** | | | **p-value in T-test compared to control lung** | | |
|---|---|---|---|---|---|---|---|---|---|---|---|---|---|
| | | | | | size of tumors | | | size of tumors | | | size of tumors | | |
| | | | | | small | middle | large | small | middle | large | small | middle | large |
| *Cell growth:* | | | | | | | | | | | | | |
| *metabolism, glycolysis* | | | | | | | | | | | | | |
| **U51805** | **Arg1** | **arginase 1, liver** | | | **6,1** | **10,8** | ***14,8*** | **94** | **100** | **100** | **0,01** | **0,09** | **0,27** |
| X13135 | Fasn | fatty acid synthase | **T, R** | **2** | 3,1 | 2,9 | *3,1* | 100 | 100 | 100 | 0,00 | 0,03 | 0,09 |
| AB033887 | Facl4 | fatty acid-Coenzyme A ligase, long chain 4 | r**R** | | *2,7* | 3,2 | 3,2 | 91 | 88 | 100 | 0,09 | 0,02 | 0,00 |
| Z67748 | Srm | spermidine synthase | **T, R** | **5** | 4,6 | 3,4 | 5,3 | 100 | 88 | 100 | 0,00 | 0,15 | 0,05 |
| **AA913994** | **Shmt1** | **serine hydroxymethyl transferase 1 (soluble)** | **T, R** | **4** | **7,5** | **7,3** | **9,4** | **100** | **100** | **100** | **0,01** | **0,04** | **0,04** |
| AI841389 | Eno1 | enolase 1, alpha non-neuron | **T, R** | | *2,3* | 2,3 | 3,5 | 100 | 100 | 100 | 0,00 | 0,01 | 0,05 |
| M17516 | Ldh1 | lactate dehydrogenase 1, A chain | **T, R** | | *3,4* | 3,3 | 3,7 | 100 | 100 | 100 | 0,01 | 0,01 | 0,03 |
| Y11666 | Hk2 | hexokinase 2 | **R** | | *2,1* | 3,6 | 4,8 | 100 | 100 | 100 | 0,01 | 0,03 | 0,02 |
| J05277 | Hk1 | hexokinase 1 | | **2** | *2,8* | 2,0 | 3,2 | 100 | 75 | 100 | 0,01 | 0,13 | 0,00 |
| M32599 | Gapd | glyceraldehyde-3-phosphate dehydrogenase | | **1** | *3,0* | 2,9 | 3,5 | 100 | 100 | 100 | 0,01 | 0,00 | 0,04 |
| AF058956 | Suclg2 | succinate-Coenzyme A ligase, GDP-forming, beta subunit | | | *2,2* | 2,2 | 3,0 | 100 | 94 | 100 | 0,00 | 0,01 | 0,02 |
| M14220 | Gpi1 | glucose phosphate isomerase 1 | **R** | | 4,2 | 2,0 | 2,7 | 100 | 50 | 33 | 0,05 | 0,29 | 0,36 |
| L31777 | Tpi | triosephosphate isomerase | | **1** | *2,2* | 2,8 | 3,4 | 100 | 100 | 100 | 0,01 | 0,00 | 0,01 |
| AW123026 | Gnpnat1 | glucosamine-phosphate N-acetyltransferase 1 | | **1** | *2,8* | 2,7 | 3,5 | 100 | 94 | 100 | 0,04 | 0,04 | 0,02 |
| AB030316 | Pign | ***phosphatidylinositol* glycan, class N** | | | *2,8* | 1,8 | 3,2 | 50 | 50 | 100 | 0,03 | 0,29 | 0,00 |
| *nucleotide biosynthesis,* | | | | | | | | | | | | | |
| *DNA metabolism* | | | | | | | | | | | | | |
| M13352 | Tyms | thymidylate synthase | **T, R** | | 3,7 | 2,2 | *2,3* | 100 | 63 | 75 | 0,00 | 0,12 | 0,20 |
| X60980 | Tk1 | **thymidine kinase 1** | **T, R** | | 4,2 | 2,5 | 2,9 | 100 | 19 | 33 | 0,00 | 0,05 | 0,22 |
| U20892 | Gart | phosphoribosylglycinamide formyltransferase | **R** | **1** | 3,0 | 2,6 | 3,4 | 100 | 94 | 100 | 0,01 | 0,06 | 0,04 |
| M33934 | Impdh2 | inosine 5'-phosphate dehydrogenase 2 | **R** | **2** | 4,5 | 3,5 | 4,5 | 100 | 100 | 100 | 0,01 | 0,03 | 0,02 |
| A1850362 | Uck2-pending | uridine-cytidine kinase 2 | | **1** | 4,4 | 2,8 | 4,5 | 100 | 100 | 100 | 0,00 | 0,06 | 0,02 |
| U60318 | Mre11a | **meiotic recombination 11 homolog A (S. cerevisiae)** | | | 3,6 | 2,5 | 3,2 | 100 | 50 | 100 | 0,02 | 0,02 | 0,01 |
| U01915 | Top2a | **topoisomerase (DNA) II alpha** | | | 10,6 | 4,8 | *7,2* | 100 | 50 | 33 | 0,05 | 0,07 | 0,27 |
| L32836 | Ahcy | S-adenosylhomocysteine hydrolase | **R** | | 3,1 | 2,3 | *3,0* | 100 | 88 | 100 | 0,00 | 0,03 | 0,07 |
| AI841645 | Ard1 | N-acetyltransferase ARD1 homolog (S. cerevisiae) | | | 3,3 | 2,7 | *2,9* | 100 | 100 | 100 | 0,00 | 0,03 | 0,02 |
| X67668 | Hmgb2 | high mobility group box 2 | | | 4,3 | 2,4 | *2,7* | 100 | 75 | 75 | 0,01 | 0,14 | 0,24 |
| D90374 | Apex1 | apurinic/apyrimidinic endonuclease 1 | **T,R** | **2** | 6,6 | 4,0 | 5,9 | 100 | 100 | 100 | 0,00 | 0,06 | 0,00 |
| X66323 | Xrcc5 | X-ray repair complementing defective repair in Chinese hamster cells 5 | | | 3,5 | 3,0 | 3,3 | 100 | 100 | 100 | 0,00 | 0,00 | 0,02 |
| K02927 | Rrm1 | **ribonucleotide reductase M1** | | | 3,8 | 2,2 | *3,2* | 100 | 25 | 33 | 0,00 | 0,12 | 0,31 |
| M14223 | Rrm2 | ribonucleotide reductase M2 | | | 5,8 | 2,7 | 3,8 | 100 | 6 | 66 | 0,00 | 0,06 | 0,24 |
| AW122092 | Rfc4 | replication factor C (activator 1) 4 | **R** | | 4,7 | 3,1 | 3,6 | 100 | 75 | 100 | 0,01 | 0,05 | 0,03 |
| M38700 | G22p1 | thyroid autoantigen | **T** | | 4,1 | 3,2 | 4,0 | 100 | 94 | 100 | 0,00 | 0,09 | 0,00 |
| U25691 | Hells | **helicase, lymphoid specific** | **R** | **1** | 3,3 | 1,6 | *3,1* | 100 | 75 | 100 | 0,00 | 0,01 | 0,10 |
| *Chromatin* | *structure* | | | | | | | | | | | | |
| U85614 | Smarcc1 | SWI/SNF related, matrix associated, actin dependent regulator of chromatin, subfamily c, member 1 | | **1** | 3,0 | 2,2 | *2,6* | 100 | 81 | 100 | 0,02 | 0,02 | 0,00 |
| AI851599 | H1fx | H1 histone family, member X | | | 4,8 | *5,8* | 6,4 | 100 | 8 | 24 | 0,03 | 0,10 | 0,14 |
| M35153 | Lmnb1 | lamin B1 | | | 3,8 | 2,1 | 2,7 | 100 | 56 | 91 | 0,01 | 0,07 | 0,07 |
| *RNA processing,* | | | | | | | | | | | | | |
| *ribosomal biogenesis* | | | | | | | | | | | | | |
| AI853173 | Rpo1-3 | RNA polymerase 1-3 | **rR** | | 3,2 | 2,7 | 3,0 | 100 | 81 | 100 | 0,00 | 0,04 | 0,00 |
| AI838709 | Spnr | spermatid perinuclear RNA binding protein | | | 6,1 | 7,1 | 5,3 | 100 | 100 | 100 | 0,01 | 0,02 | 0,02 |
| AW121447 | Nol5a | nucleolar protein 5A | **R** | **2** | 5,4 | 3,7 | 5,2 | 100 | 81 | 100 | 0,01 | 0,11 | 0,02 |
| AA656775 | Rrs1 | RRS1 ribosome biogenesis regulator homolog (S. cerevisiae) | **R** | **3** | 4,2 | 2,9 | 4,6 | 100 | 75 | 100 | 0,00 | 0,08 | 0,03 |
| X07699 | Ncl | nucleolin | **T, R** | **4** | 3,5 | 2,7 | 3,5 | 100 | 81 | 100 | 0,01 | 0,03 | 0,02 |
| M33212 | Npm1 | nucleophosmin 1 | **T,R** | **3** | 3,7 | 3,1 | 4,0 | 100 | 100 | 100 | 0,00 | 0,02 | 0,01 |
| **U64450** | **Npm3** | **nucleoplasmin 3** | | **2** | **6,0** | **5,5** | **7,4** | **100** | **94** | **100** | **0,01** | **0,05** | **0,04** |
| AI183202 | Hnrpa1 | heterogeneous nuclear ribonucleoprotein A1 | **T,** R | **2** | 2,7 | 2,1 | 3,0 | 100 | 75 | 100 | 0,00 | 0,04 | 0,02 |
| Z22593 | Fbl | fibrillarin | **R** | **1** | 3,2 | 2,8 | 3,6 | 100 | 94 | 100 | 0,00 | 0,02 | 0,01 |
| AF053232 | Nol5 | nucleolar protein 5 | | **1** | 7,7 | 4,8 | 6,6 | 100 | 100 | 100 | 0,00 | 0,04 | 0,02 |
| AW060597 | Snrpg | small nuclear ribonucleoprotein polypeptide G | **rT** | | *2,7* | 2,3 | 3,2 | 100 | 81 | 100 | 0,02 | 0,10 | 0,04 |
| AA684508 | Rnu22 | RNA, U22 small nucleolar | **rT** | | 4,8 | 3,1 | 3,8 | 100 | 69 | 83 | 0,00 | 0,03 | 0,02 |
| AI853113 | Cpsf5 | cleavage and polyadenylation specific factor 5* | **rT** | | *2,9* | 2,6 | 3,1 | 100 | 88 | 100 | 0,01 | 0,02 | 0,03 |
| AA656757 | Pabpc4 | poly A binding protein, cytoplasmic 4 | **T** | **2** | 5,2 | 4,0 | 5,8 | 100 | 94 | 100 | 0,00 | 0,04 | 0,03 |
| AI851198 | Nola1 | nucleolar protein family A, member 1 (H/ACA small nucleolar RNPs) | | **1** | 4,6 | 3,6 | 4,9 | 100 | 88 | 100 | 0,01 | 0,07 | 0,02 |
| AI852665 | Mki67ip | Mki67 (FHA domain) interacting nucleolar phosphoprotein | | | 5,2 | 3,3 | 3,6 | 100 | 88 | 100 | 0,01 | 0,11 | 0,01 |
| AA674812 | Ppan | peter pan homolog (Drosophila) | | **1** | 4,0 | 2,6 | 3,7 | 100 | 75 | 100 | 0,01 | 0,06 | 0,00 |
| AI852608 | Rnac-pending | RNA cyclase homolog | | **2** | 3,6 | 2,7 | 4,0 | 100 | 88 | 100 | 0,02 | 0,04 | 0,00 |
| AI845664 | Grwd1 | **glutamate-rich WD repeat containing 1** | | | *3,8* | 2,1 | 3,2 | 91 | 44 | 100 | 0,00 | 0,16 | 0,02 |
| *protein synthesis and metabolism* | | | | | | | | | | | | | |
| AW122030 | Psat1 | phosphoserine aminotransferase 1 | **T, R** | | 7,2 | 5,3 | 5,5 | 100 | 100 | 100 | 0,02 | 0,03 | 0,03 |
| U12403 | Rpl10a | ribosomal protein L10A | **T, R** | **1** | *2,6* | 2,2 | 3,2 | 100 | 81 | 100 | 0,00 | 0,05 | 0,03 |
| X51528 | Rpl13a | ribosomal protein L13a | **T, R** | | 6,3 | 4,3 | 5,0 | 100 | 100 | 100 | 0,00 | 0,02 | 0,04 |
| X05021 | Rpl27a | ribosomal protein L27a | **T, R** | **1** | *2,9* | 2,4 | 3,3 | 100 | 75 | 100 | 0,00 | 0,05 | 0,02 |
| AW060951 | Bzw2 | basic leucine zipper and W2 domains 2 | **rT** | **2** | 5,4 | 4,1 | 6,2 | 100 | 81 | 100 | 0,00 | 0,10 | 0,00 |
| AI839363 | Eif3s6 | eukaryotic translation initiation factor 3, subunit 6 | **T, R** | **1** | 4,1 | 3,2 | 4,4 | 100 | 75 | 100 | 0,00 | 0,09 | 0,01 |
| AV170770 | Cct5 | chaperonin subunit 5 (epsilon) | **T, R** | **1** | 4,1 | 3,4 | 3,6 | 100 | 75 | 100 | 0,00 | 0,05 | 0,00 |
| AW122851 | Fkbp11 | FK506 binding protein 11 | **rT** | **1** | 7,9 | 7,5 | *9,1* | 100 | 75 | 100 | 0,00 | 0,12 | 0,05 |
| AI840579 | Srr | serine racemase | | | 4,5 | 3,6 | 5,0 | 100 | 100 | 100 | 0,00 | 0,04 | 0,02 |
| AW124889 | Pycs | pyrroline-5-carboxylate synthetase (glutamate gamma-semialdehyde synthetase) | | **1** | 3,6 | 2,0 | 4,4 | 100 | 50 | 100 | 0,00 | 0,04 | 0,01 |
| AW046590 | Pcbd | 6-pyruvoyl-tetrahydropterin synthase/dimerization cofactor of hepatocyte nuclear factor 1 alpha (TCF1) | | | 5,0 | 4,3 | 5,0 | 100 | 100 | 100 | 0,01 | 0,01 | 0,03 |
| AI840436 | Mrps5 | mitochondrial ribosomal protein S5 | | | *2,4* | 2,2 | 3,3 | 100 | 50 | 100 | 0,09 | 0,01 | 0,00 |
| X06406 | Lamr1 | laminin receptor 1 (ribosomal protein SA) | | **2** | *2,7* | 2,2 | 3,1 | 83 | 75 | 100 | 0,01 | 0,04 | 0,01 |
| AW124432 | Mrpl12 | mitochondrial ribosomal protein L12 | | | 4,0 | 3,5 | 3,2 | 100 | 88 | 100 | 0,00 | 0,07 | 0,01 |
| AW045418 | Rpl44 | ribosomal protein L44 | | **1** | *2,6* | 2,1 | 3,1 | 100 | 75 | 100 | 0,00 | 0,05 | 0,01 |
| AW120719 | Eif2b1 | eukaryotic translation initiation factor 2B, subunit 1 (alpha) | | | 4,3 | 3,2 | 3,3 | 100 | 94 | 100 | 0,00 | 0,03 | 0,00 |
| transport | | | | | | | | | | | | | |
| AW125446 | Golph2 | golgi phosphoprotein 2 | **rT** | | 4,1 | 5,1 | 4,6 | 100 | 100 | 100 | 0,01 | 0,00 | 0,04 |
| AW122428 | Timm10 | **translocase of inner mitochondrial membrane 10 homolog (yeast)** | **rR** | **1** | 5,7 | 3,3 | 4,2 | 100 | 50 | 100 | 0,01 | 0,14 | 0,03 |
| AA655369 | Timm8a | translocase of inner mitochondrial membrane 8 homolog a (yeast) | **rR** | **2** | 5,8 | 4,5 | 5,7 | 100 | 100 | 100 | 0,00 | 0,07 | 0,01 |
| AF043249 | Tomm40 | **translocase of outer mitochondrial membrane 40 homolog (yeast)** | | | *2,7* | 2,3 | 3,2 | 100 | 38 | 100 | 0,00 | 0,00 | 0,00 |
| A1846308 | Sfxn1 | sideroflexin 1 | **R** | **1** | *2,9* | 2,4 | 3,1 | 100 | 88 | 100 | 0,00 | 0,05 | 0,00 |
| L23755 | Slc19a1 | **solute carrier family 19 (sodium/hydrogen exchanger), member 1** | **T** | **1** | 10,4 | 6,6 | 11,1 | 100 | 75 | 100 | 0,02 | 0,04 | 0,03 |
| AI846682 | Slc15a2 | solute carrier family 15 (H+/peptide transporter), member 2 | | | *2,1* | 2,7 | 3,6 | 66 | 88 | 100 | 0,21 | 0,02 | 0,03 |
| AI594427 | Slc4a7 | solute carrier family 4, sodium bicarbonate cotransporter, member 7 | | | *2,5* | 2,3 | 3,1 | 100 | 88 | 100 | 0,00 | 0,04 | 0,00 |
| AF020195 | Slc4a4 | solute carrier family 4 (anion exchanger), member 4 | | | *2,1* | 4,2 | 4,3 | 91 | 100 | 100 | 0,01 | 0,09 | 0,05 |
| U88623 | Aqp4 | *aquaporin 4* | ***rR*** | | 7,1 | 5,0 | *4,9* | 100 | 75 | 66 | 0,02 | 0,08 | 0,25 |
| AI846319 | Rangnrf-pending | **RAN guanine nucleotide release factor** | | **1** | 4,2 | 3,6 | *5,7* | 100 | 50 | 91 | 0,00 | 0,05 | 0,00 |
| D55720 | Kpna2 | karyopherin (importin) alpha 2 | **rT** | | 3,2 | 1,8 | *1,7* | 100 | 56 | 41 | 0,03 | 0,17 | 0,36 |
| AI847564 | Kpnb3 | karyopherin (importin) beta 3 | | **1** | 3,9 | 2,7 | 3,8 | 100 | 94 | 100 | 0,02 | 0,03 | 0,01 |
| AW212243 | Ipo4 | importin 4 | | | 3,6 | 2,8 | 3,7 | 100 | 81 | 100 | 0,00 | 0,01 | 0,00 |
| X61399 | Mlp | MARCKS-like protein | | **1** | 3,3 | 2,5 | 4,6 | 100 | 94 | 100 | 0,01 | 0,02 | 0,03 |
| M60348 | Abcb1b | ATP-binding cassette, sub-family B (MDR/TAP), member 1 B | **rT** | **1** | *2,0* | 1,6 | 3,2 | 83 | 50 | 100 | 0,02 | 0,28 | 0,00 |
| AF099988 | Stk39 | serine/threonine kinase 39, STE20/SPS1 homolog (yeast) | | **1** | 3,3 | 3,3 | 4,3 | 100 | 100 | 100 | 0,01 | 0,01 | 0,02 |
| *Chromatin remodelling* | | | | | | | | | | | | | |
| U05252 | Satb1 | special AT-rich sequence binding protein 1 | | | -5,4 | -5,6 | -7,0 | 100 | 100 | 100 | 0,01 | 0,01 | 0,00 |
| U73478 | Anp32a | acidic (leucine-rich) nuclear phosphoprotein 32 family, member A | | | *-1,7* | -3,6 | -3,8 | 66 | 100 | 100 | 0,29 | 0,00 | 0,01 |
| X05862 | Hist1h2bc | histone 1, H2bc | **rT** | | *-3,0* | -3,0 | -4,9 | 100 | 100 | 100 | 0,00 | 0,00 | 0,00 |

| | | | | | | | | | | | | | |
|---|---|---|---|---|---|---|---|---|---|---|---|---|---|
| *Criteria for significant expression changes were as follows: a gene should be expressed ("Present" call) in all tumor replicates of one set or in all control non-transgenic lungs for up- and down-regulated genes respectively and have a mean fold change (FC)≥ 3, p-value in T-test ≤ 0,05 and 100% "Increase" call in comparative ranking analysis for up regulated genes and FC≤- 3, p-value in T-test ≤ 0,05 and 100% "Decrease" for down regulated genes T- known c-Myc-target R- known c-Myc responsive gene rT- relative of known c-Myc-target rR- relative of known c-Myc-responsive gene N- number of potential c-Myc binding sites in promotor sequence **- % Increase/Decrease - concordance of change calls in the pairwise comparisons (each tumor to each normal lung sample), by which genes are up or down regulated respectively | | | | | | | | | | | | | |

Bold gene cell indicates that gene was not detected in all control lungs (for up regulated genes).

Bold row indicates that gene was not detected in all control lungs and was expressed in all tumor samples (for up regulated genes) and inversely for down regulated genes

**Table 5. Fold changes of gene expression in lung tumors determined by RT-PCR and microarray analysis**

| Gene | Method | Mean FC | | |
|---|---|---|---|---|
| | | size of tumors | | |
| | | small | middle | large |
| Shtm1 | RT-PCR | 3.1 | 2.4 | 2.7 |
| | Affymetrix | 7.5 | 7.3 | 9.4 |
| Npm3 | RT-PCR | 3.8 | 2.5 | 2.8 |
| | Affymetrix | 6.0 | 5.5 | 7.4 |
| Tk1 | RT-PCR | 8.7 | 3.9 | 4.4 |
| | Affymetrix | 4.2 | 2.5 | 2.9 |
| Uck2 | RT-PCR | 3.1 | 1.9 | 2.4 |
| | Affymetrix | 4.4 | 2.8 | 4.5 |
| Impdh2 | RT-PCR | 2.8 | 2.1 | 2.4 |
| | Affymetrix | 4.5 | 3.5 | 4.5 |
| Srm1 | RT-PCR | 5.5 | 3.5 | 4.1 |
| | Affymetrix | 4.6 | 3.4 | 5.3 |
| Arg1 | RT-PCR | 4.3 | 4.0 | 4.6 |
| | Affymetrix | 6.1 | 10.8 | 14.8 |
| Fasn | RT-PCR | 1.9 | 1.5 | 1.7 |
| | Affymetrix | 3.1 | 2.9 | 3.1 |
| Hk2 | RT-PCR | 1.5 | 2.1 | 2.4 |
| | Affymetrix | 2.1 | 3.6 | 4.8 |
| Slc19a1 | RT-PCR | 4.3 | 3.8 | 4.7 |
| | Affymetrix | 10.4 | 6.6 | 11.1 |
| Smarcc1 | RT-PCR | 1,8 | 1,4 | 1,7 |
| | Affymetrix | 3.0 | 2.2 | 2.6 |
| Satb1 | RT-PCR | A | A | A |
| | Affymetrix | -5.4 | -5.6 | -7.0 |

| | | | | |
|---|---|---|---|---|
| A - "Absent" - no expression was detected | | | | |

**Table 6 Gene expression profiles in c-Myc-induced lung papillary adenocarcinoma: differently expressed genes involved in extracellular control of cell proliferation, adhesion, invasion and angiogenesis: (a) - up regulated genes; (b) down regulated genes**

| ACC | Gene Symbol | Gene Title | | N | **FC** | | | **%Increase*/Decrease** | | | **p-value in T-test compared to control lung** | | |
|---|---|---|---|---|---|---|---|---|---|---|---|---|---|
| | | | | | size of tumors | | | size of tumors | | | size of tumors | | |
| | | | | | small | middle | large | small | middle | large | small | middle | large |
| **(a) Up regulated genes in C-Myc induced lung tumors** | | | | | | | | | | | | | |
| **Activation of growth factors signaling through tyrosine** | | | | | | | | | | | | | |
| **knase receptors** | | | | | | | | | | | | | |
| L41352 | **Areg** | amphiregulin | | | **5,1** | 7,7 | ***8,3*** | 100 | 100 | 66 | 0,01 | 0,07 | 0,18 |
| AI467390 | **Map2k1** | mitogen activated protein kinase kinase 1* | rT | 1 | **2,7** | 2,7 | **3,6** | 100 | 100 | 100 | 0,00 | 0,01 | 0,01 |
| L00039 | **Myc** | myelocytomatosis oncogene | | | **51,0** | 23,4 | **58,6** | 100 | 100 | 100 | 0,01 | 0,05 | 0,04 |
| U15443 | **Ros1** | **Ros1 proto-oncogene** | | | **9,3** | 13,3 | ***21,9*** | 100 | 75 | 100 | 0,02 | 0,24 | 0,19 |
| Activation of Wnt/ beta-catenin signaling | | | | | | | | | | | | | |
| AW121294 | **Arhu** | ras homolog gene family, member U | | **1** | **4,0** | 4,2 | ***3,8*** | 100 | 44 | 66 | 0,00 | 0,21 | 0,18 |
| AI646638 | **Frat2** | **frequently rearranged in advanced T-cell lymphomas 2** | | | **5,0** | 4,6 | **5,9** | 100 | 75 | 100 | 0,02 | 0,04 | 0,00 |
| **Decrease of death receptor signaling** | | | | | | | | | | | | | |
| **AV109962** | **Traf4** | **Tnf receptor associated factor 4** | | **1** | **4,3** | **5,0** | **4,5** | **100** | 100 | **100** | **0,01** | **0,00** | **0,03** |
| **Cell junctions** | | | | | | | | | | | | | |
| AF072128 | **Cldn2** | **claudin 2** | | | ***20,7*** | 24,0 | **45,3** | 33 | 50 | 100 | 0,12 | 0,06 | 0,03 |
| X63099 | **Gjb3** | gap junction membrane channel protein beta 3 | | **1** | 8,7 | 7,5 | **8,8** | 100 | 100 | 100 | 0,00 | 0,01 | 0,02 |
| M63801 | **Gja1** | gap junction membrane channel protein alpha 1 | | **2** | **3,2** | 1,8 | ***2,9*** | 100 | 75 | 100 | 0,00 | 0,03 | 0,05 |
| **Adhesion, cytoskeleton regulation** | | | | | | | | | | | | | |
| M22832 | Krt1-18 | keratin complex 1, acidic, gene 18 | | | ***2,9*** | 3,2 | **3,6** | 100 | 100 | 100 | 0,00 | 0,01 | 0,00 |
| AF011450 | **Col15a1** | procollagen, type XV | | | ***1,*7** | 5,1 | **4,6** | 50 | 94 | 100 | 0,04 | 0,06 | 0,03 |
| AA170696 | **Icam1** | intercellular adhesion molecule* | T | | **3,3** | 1,8 | ***2,8*** | 100 | 56 | 100 | 0,04 | 0,18 | 0,01 |
| **AW228162** | **Dsc2** | **desmocollin 2** | rR | | 4,1 | 4,1 | 4,4 | 100 | 94 | **100** | **0,00** | **0,00** | **0,00** |
| AI152659 | **Dsg2** | desmoglein 2 | | 1 | ***3,0*** | 3,5 | **3,5** | 100 | 100 | 100 | 0,01 | 0,02 | 0,00 |
| L11316 | **Ect2** | ect2 oncogene | | | **10,3** | 4,6 | ***4,4*** | 100 | 94 | 100 | 0,02 | 0,12 | 0,20 |
| **(b) Down regulated genes in C-Myc induced lung tumors** | | | | | | | | | | | | | |
| **TNF-signaling** | | | | | | | | | | | | | |
| M83649 | **Tnfrsf6** | tumor necrosis factor receptor superfamily, member 6 | R | 1 | **-4,2** | -2,6 | -3,6 | 100 | 87 | 91 | 0,02 | 0,02 | 0,01 |
| Y00208 | **Hoxa5** | *homeo* box *A5* | *R* | | **-6,1** | -7,4 | **-7,7** | 100 | 100 | 100 | 0,00 | 0,00 | 0,00 |
| **Proteolysis, invasion, angiogenesis** | | | | | | | | | | | | | |
| M84683 | **Muc1** | mucin 1, transmembrane* | T | | **3,0** | 2,5 | ***2,1*** | 100 | 100 | 91 | 0,00 | 0,01 | 0,08 |
| X81627 | **Lcn2** | lipocalin 2 | | | **4,8** | 7,3 | ***4,7*** | 100 | 100 | 100 | 0,00 | 0,02 | 0,08 |
| **AI786089** | **Kng** | **kininogen** | | | **22,4** | **37,1** | ***12,6*** | **100** | 100 | **100** | **0,02** | **0,03** | **0,09** |
| **AI042655** | **Hgfac** | **hepatocyte growth factor activator** | | | **4,7** | **9,6** | **8,7** | **100** | 100 | **100** | **0,01** | **0,01** | **0,01** |
| AA608277 | **Adora2b** | adenosine A2b receptor | | | **5,8** | 4,1 | ***2,4*** | 100 | 75 | 33 | 0.00 | 0,10 | 0,44 |
| AW230369 | **Spint1** | serine protease inhibitor, Kunitz type 1 | | | **3,0** | 2,7 | ***2,3*** | 100 | 100 | 100 | 0,00 | 0,00 | 0,02 |
| AA726223 | **Adam19** | a disintegrin and metalloproteinase domain 19 (meltrin beta) | | **1** | **3,2** | 3,3 | 3,1 | 100 | 100 | 100 | 0,00 | 0,02 | 0,02 |
| AF030065 | **Hpn** | hepsin | | | **5,0** | 4,7 | **5,3** | 100 | 100 | 100 | 0,00 | 0,00 | 0,00 |
| **AW047185** | **Thop1** | **thimet oligopeptidase 1** | **R** | | **4,0** | **2,8** | **4,2** | **100** | 69 | **100** | **0,00** | **0,01** | **0,02** |
| **TGF-beta- signaling** | | | | | | | | | | | | | |
| **AI850533** | **Bmp6** | **bone morphogenetic protein 6** | | 1 | **-15,5** | **-34,8** | **-22,8** | **100** | 100 | **100** | **0,00** | **0,00** | **0,00** |
| AJ009862 | **Tgfb1** | Tgf-beta ? | | | **-2,9** | ? | **-7,2** | 75 | ? | 83 | 0,04 | | 0,01 |
| **AA717826** | **Dpt** | **dermatopontin** | | | **-5,0** | **-6,7** | **-16,6** | **100** | **100** | **100** | **0,02** | **0,01** | **0,01** |
| L48015 | **Acvrl1** | activin A receptor, type II-like 1 | | | **-5,9** | -6,7 | **-7,6** | 100 | 100 | 100 | 0,00 | 0,00 | 0,00 |
| X77952 | **Eng** | endoglin | | | **-6,7** | -6,9 | **-7,6** | 100 | 100 | 100 | 0,00 | 0,00 | 0,00 |
| D76432 | **Zfhx1a** | zinc finger homeobox 1a | | | **-6,8** | -6,8 | **-8,5** | 100 | 100 | 100 | 0,00 | 0,00 | 0,00 |
| AV138783 | **Gadd45b** | growth arrest and DNA-damage-inducible 45 beta | **rR** | **1** | -1,5 | | **-3,5** | 58 | | 100 | 0,01 | 0,01 | 0,02 |
| ***negative regulation* of IGF-1- mediated survival pathway** | | | | | | | | | | | | | |
| L12447 | **Igfbp5** | insulin-like growth factor binding protein 5 | | | **-6,5** | *-1,9* | **-4,9** | 100 | 75 | 100 | 0,00 | 0,25 | 0,00 |
| X81584 | **Igfbp6** | insulin-like growth factor binding protein 6 | | | **-8,8** | -8,8 | **-18,9** | 100 | 100 | 100 | 0,00 | 0,00 | 0,00 |
| **X76066** | **Igfbp4** | **insulin-like growth factor binding protein 4** | | | **-2,8** | **-3,3** | **-5,3** | **100** | **100** | **100** | **0,03** | **0,03** | **0,01** |
| U88327 | **Socs2** | suppressor of cytokine signaling 2 | | | **-3,7** | -1,7 | *-2,3* | 100 | 87 | 100 | 0,01 | 0,05 | 0,04 |
| U57524 | **Nfkbia** | nuclear factor of kappa light chain gene enhancer in B-cells inhibitor, alpha | | | **-3,1** | -2,1 | -2,8 | 100 | 93 | 100 | 0,00 | 0,01 | 0,00 |
| ***negative regulation of* Wnt/β- catenin signaling** | | | | | | | | | | | | | |
| AI019193 | Tcf7 | transcription factor 7, T-cell specific* | **T** | **1** | **-4,9** | -5,8 | -8,5 | 100 | 100 | 100 | 0,01 | 0,00 | 0,00 |
| AB023419 | **Sox7** | SRY-box containing gene 7 | | | **-4,4** | -5,8 | -6,3 | 100 | 100 | 100 | 0,00 | 0,00 | 0,00 |
| ***negative regulation of* Ras/Raf/MEK/ERK-signaling** | | | | | | | | | | | | | |
| X61940 | **Dusp1** | dual specificity phosphatase 1 | **R** | **1** | **-3,7** | *-1,7* | *-2,3* | 100 | 93 | 100 | 0,00 | 0,17 | 0,11 |
| D83484 | **Ptpre** | protein tyrosine phosphatase, receptor type, E | | | *-3,0* | -4,5 | **-4,5** | 100 | 100 | 100 | 0,00 | 0,00 | 0,00 |
| AB018194 | **Ptpns1** | protein tyrosine phosphatase, non-receptor type substrate 1 | | | *-2,0* | -3,9 | **-4,7** | 75 | 100 | 100 | 0,01 | 0,00 | 0,00 |
| AF053959 | **Rassf5** | Ras association (RalGDS/AF-6) domain family 5 | | | *-2,8* | -3,2 | -3,7 | 100 | 100 | 100 | 0,00 | 0,00 | 0,00 |
| **Scaffolding proteins, chaperones** | | | | | | | | | | | | | |
| AB020886 | **Akap12** | A kinase (PRKA) anchor protein (gravin) 12 | **R** | **1** | **-5,8** | -9,0 | **-11,9** | 100 | 100 | 100 | 0,00 | 0,00 | 0,00 |
| **AF033275** | **Akap2** | **A kinase (PRKA) anchor protein** | **2 rR** | | **-3,3** | **-3,3** | ***-4,8*** | **100** | **93** | **91** | **0,01** | **0,01** | **0,01** |
| AI747654 | **Cav** | caveolin, caveolae protein | **R** | **1** | **-4,0** | -5,6 | -6,5 | 100 | 100 | 100 | 0,00 | 0,00 | 0,00 |
| AW120711 | **Dnajb9** | DnaJ (Hsp40) homolog, subfamily B, member 9 | ***rR*** | **1** | **-3,0** | -2,8 | *-2,1* | 100 | 100 | 83 | 0,01 | 0,00 | 0,00 |
| AF040252 | **Fkbp7** | FK506 binding protein 7 | | **1** | **-3,6** | -3,2 | **-4,1** | 100 | 100 | 100 | 0,00 | 0,00 | 0,00 |
| **Anti-angiogenic, anti-invasive** | | | | | | | | | | | | | |
| **AF080090** | **Sema3f** | **sema domain, immunoglobulin domain (Ig), short basic domain, secreted, (semaphorin) 3 F** | | | **-4,9** | **-5,4** | **-5,6** | **100** | **100** | **100** | **0,01** | **0,00** | **0,00** |
| **X70854** | **Fbln1** | **fibulin 1 !!!** | **rR** | | **-3,8** | **-3,1** | **-10,1** | **100** | **100** | **100** | **0,00** | **0,00** | **0,00** |
| **D21165** | **Vsnl1** | **visinin-like 1** | | | **-7,1** | **-7,1** | **-7,1** | **100** | **87** | **100** | **0,00** | **0,00** | **0,00** |
| AB006960 | **Reck** | reversion-inducing-cysteine-rich protein with kazal motifs | | **1** | **-4,1** | -5,9 | **-5,2** | 100 | 100 | 100 | 0,00 | 0,00 | 0,00 |
| D38117 | **Capn2** | calpain 2 | | | **-3,0** | -3,0 | **-3,8** | 100 | 100 | 100 | 0,02 | 0,03 | 0,02 |
| **Adhesion, ECM** | | | | | | | | | | | | | |
| AI853217 | **Cdh5** | cadherin 5 | | | **-7,2** | -9,9 | **-10,0** | 100 | 100 | 100 | 0,00 | 0,00 | 0,00 |
| A1844853 | **Spock2** | sparc/osteonectin, cwcv and kazal-like domains proteoglycan 2 | | | **-6,6** | -11,8 | **-22,3** | 100 | 100 | 100 | 0,00 | 0,00 | 0,00 |
| X14432 | **Thbd** | thrombomodulin | | **1** | **-16,0** | -27,1 | **-55,8** | 100 | 100 | 100 | 0,00 | 0,00 | 0,00 |
| X80764 | **Tie1** | tyrosine kinase receptor 1 | | | **-8,4** | -12,7 | **-12,2** | 100 | 100 | 100 | 0,00 | 0,00 | 0,00 |
| **X65493** | **Icam2** | **intercellular adhesion molecule 2** | | | **-12,0** | **-30,6** | **-48,1** | **100** | **100** | **100** | **0,00** | **0,00** | **0,00** |
| D13664 | **Osf2-pending** | osteoblast specific factor 2 (fasciclin I-like | **R** | | **-3,7** | -3,5 | **-5,4** | 100 | 100 | 100 | 0,00 | 0,00 | 0,00 |
| X71426 | **Tek** | endothelial-specific receptor tyrosine kinase | | | **-12,6** | -16,9 | **-26,3** | 100 | 100 | 100 | 0,00 | 0,00 | 0,00 |
| AW061260 | **Nes** | nestin | | | **-3,1** | -4,3 | **-4,9** | 100 | 100 | 100 | 0,00 | 0,00 | 0,00 |
| AA754887 | **Cd97** | CD97 antigen* | **T** | | **-8,4** | -9,5 | **-9,7** | 100 | 100 | 100 | 0,00 | 0,00 | 0,00 |
| AI843063 | **Vwf** | Von Willebrand factor homolog | | | **-8,4** | -7,7 | **-16,9** | 100 | 100 | 100 | 0,00 | 0,00 | 0,00 |
| AA880988 | **Xlkd1** | extra cellular link domain-containing 1 | | | **-12,2** | -11,7 | **-28,2** | 100 | 100 | 100 | 0,02 | 0,02 | 0,02 |
| U66166 | **Sparcl1** | SPARC-like 1 (mast9, hevin) | | | *-2,9* | -3,2 | **-5,4** | 100 | 100 | 100 | 0,00 | 0,00 | 0,00 |
| M60474 | **Marcks** | myristoylated alanine rich protein kinase C substrate | | | **-5,8** | -4,1 | *-5,0* | 100 | 93 | 91 | 0,02 | 0,01 | 0,01 |
| AI854794 | **Tenc1** | tensin like C1 domain-containing phosphatase | | | **-5,6** | -5,1 | **-6,8** | 100 | 100 | 100 | 0,03 | 0,03 | 0,02 |
| L23769 | **Mfap2** | microfibrillar-associated protein 2* | **rT** | | **-5,2** | -5,5 | **-5,8** | 100 | 100 | 100 | 0,01 | 0,00 | 0,01 |
| **U30292** | **Col13a1** | **procollagen, type XIII, alpha 1** | **rR** | | **-6,2** | **-7,3** | **-16,7** | **100** | **100** | **100** | **0,01** | **0,01** | **0,01** |
| **D86917** | **Pcdha6** | **protocadherin alpha 6** | | | **-7,6** | **-8,6** | **-9,0** | **100** | **100** | **100** | **0,00** | **0,00** | **0,00** |
| U69176 | **Lama4** | laminin, alpha 4 | | | **-3,0** | -3,1 | *-2,9* | 100 | 100 | 100 | 0,00 | 0,00 | 0,00 |
| **X84014** | **Lama3** | **laminin, alpha 3** | | | **-3,5** | **-4,8** | -5,2 | **100** | **100** | **100** | **0,01** | **0,00** | **0,01** |
| AF041409 | **Itga8** | integrin alpha 8* | **rT** | | **-7,5** | -10,6 | **-8,8** | 100 | 100 | 100 | 0,02 | 0,02 | 0,02 |
| **D86916** | **Pcdha4** | **protocadherin alpha 4** | | | **-9,9** | **-9,9** | **-9,9** | **100** | **100** | **100** | **0,00** | **0,00** | **0,00** |
| X75285 | **Fbln2** | fibulin 2 | **R** | | **-3,7** | -3,3 | *-2,2* | 100 | 100 | 75 | 0,00 | 0,00 | 0,00 |
| **M77123** | **Vtn** | **vitronectin** | | | **-6,6** | **-6,6** | **-6,6** | **100** | **93** | **100** | **0,00** | **0,00** | **0,00** |
| U12884 | **Vcam1** | vascular cell adhesion molecule 1 | | **1** | **-4,7** | -3,7 | *-4,1* | 100 | 93 | 91 | 0,01 | 0,01 | 0,01 |
| **X79199** | **Tna** | **tetranectin (plasminogen binding protein)** | | | **-11,1** | **-11,1** | **-11,1** | **100** | **100** | **100** | **0,00** | **0,00** | **0,00** |
| AW060556 | **Stab1** | stabilin 1 | | | **-3,0** | -3,3 | **-3,4** | 100 | 100 | 100 | 0,01 | 0,00 | 0,01 |
| **Cell junctions** | | | | | | | | | | | | | |
| U82758 | **Cldn5** | claudin 5 | | | **-9,1** | -17,8 | **-45,9** | 100 | 100 | 100 | 0,00 | 0,00 | 0,00 |
| **Growth suppressors** | | | | | | | | | | | | | |
| Z38110 | **Pmp22** | peripheral myelin protein | | | **-6,2** | -9,6 | **-16.3** | 100 | 100 | 100 | 0,00 | 0,00 | 0,00 |
| X58289 | **Ptprb** | protein tyrosine phosphatase, receptor type, B | | | **-14,3** | -21,3 | **-40,9** | 100 | 100 | 100 | 0,00 | 0,00 | 0,00 |
| L09562 | **Ptprg** | protein tyrosine phosphatase, receptor type, G | | | **-5,7** | -3,5 | -4,0 | 100 | 87 | 91 | 0,01 | 0,02 | 0,01 |
| AF099973 | **Slfn2** | schlafen2 ? | | | **-3,3 ?** | | **-3,7** | 75 ? | | 91 | 0,03 | | 0,03 |
| **AV349686** | **Ndr2** | **N-myc downstream regulated 2** | | | **-5,8** | -4,4 | **-4,8** | **100** | **100** | **100** | **0,00** | **0,00** | **0,00** |
| AI882555 | **Ets1** | E26 avian leukemia oncogene 1, 5' domain | | | **-4,2** | -6,1 | **-5,3** | 100 | 100 | 100 | 0,01 | 0,00 | 0,00 |
| D10837 | **Lox** | lysyl oxidase | **R** | **1** | **-3,8** | -2,3 | **-4,9** | 100 | 87 | 100 | 0,02 | 0,03 | 0,01 |
| D11374 | **Sipa1** | signal-induced proliferation associated gene 1 | | | **-3,0** | -3,3 | **-3,9** | 100 | 100 | 100 | 0,00 | 0,00 | 0,00 |
| D76440 | **Ndn** | necdin | | | **-7,6** | -8,4 | **-10,4** | 100 | 100 | 100 | 0,05 | 0,05 | 0,04 |
| AF030001 | **Notch4** | Notch gene homolog 4 (Drosophila)* ? | **T** | | **-5,7** | -6,2 | **-5,8** | 100 | 100 | 100 | 0,02 | 0,02 | 0,03 |
| **X74134** | **Nr2f1** | ***nuclear receptor subfamily* 2, *group F, member 1*** | ***R*** | | **-3,1** | **-3,1** | **-3,1** | **100** | **87** | **100** | **0,00** | **0,00** | **0,00** |
| **Transformation-associated decrease** | | | | | | | | | | | | | |
| Z16406 | **Meox2** | mesenchyme homeobox 2 | | | **-8,6** | -12,6 | **-12,6** | 100 | 100 | 100 | 0,03 | 0,03 | 0,02 |
| X60367 | **Rbp1** | retinol binding protein 1, cellular | | **2** | **-3,3** | -1,3 | -1,5 | 100 | 75 | 66 | 0,01 | 0,72 | 0,56 |
| AI152789 | **Sema7a** | sema domain, immunoglobulin domain (Ig), and GPI membrane anchor, (semaphorin) 7A | | | **-3,9** | -4,5 | -7,5 | 100 | 87 | 83 | 0,03 | 0,02 | 0,02 |
| X85994 | **Sema3c** | sema domain, immunoglobulin domain (Ig), short basic domain, secreted, (semaphorin) 3C | | **1** | **-5,1** | -4,6 | -2,5 | 100 | 93 | 91 | 0,01 | 0,01 | 0,04 |
| Z80941 | **Sema3e** | sema domain, immunoglobulin domain (Ig), short basic domain, secreted, (semaphorin) 3E | | | -2,5 | -4,2 | **-3,5** | 100 | 100 | 100 | 0,00 | 0,00 | 0,01 |
| Z68618 | **Tagln** | transgelin | | | **-3,9** | -4,5 | **-4,9** | 100 | 100 | 100 | 0,00 | 0,00 | 0,00 |
| AI841606 | **Ablim1** | actin-binding LIM protein 1 | | | **-4,4** | -5,6 | **-6,8** | 100 | 100 | 100 | 0,01 | 0,01 | 0,01 |

| | | | | | | | | | | | | | |
|---|---|---|---|---|---|---|---|---|---|---|---|---|---|
| T- known c-Myc-target R- known c-Myc responsive gene rT- relative of known c-Myc-target rR- relative of known c-Myc-responsive gene N- number of potential c-Myc binding sites in promotor sequence *- % Increase/Decrease - concordance of change calls in the pairwise comparisons (each tumor to each normal lung sample), by which genes are up or down regulated respectively | | | | | | | | | | | | | |

Bold gene cell indicates that gene was not detected in all control lungs (for up regulated genes).

Bold row indicates that gene was not detected in all control lungs and was expressed in all tumor samples (for up regulated genes) and inversely for down regulated genes.

**Table 7. Fold changes of gene expression in lung tumors determined by RT-PCR and microarray analysis**

| Gene | Method | Mean FC | | |
|---|---|---|---|---|
| | | size of tumors | | |
| | | small | middle | large |
| Ros1 | RT-PCR | 3,48 | 3,11 | 3,32 |
| | Affymetrix | 9,26 | 13,22 | 21,85 |
| Ect2 | RT-PCR | 3,55 | 2,53 | 3,11 |
| | Affymetrix | 10,25 | 4,61 | 4,44 |
| Traf4 | RT-PCR | 2,27 | 2,19 | 2,06 |
| | Affymetrix | 4,30 | 5,02 | 4,49 |
| Hgfac | RT-PCR | 3,98 | 6,28 | 6,50 |
| | Affymetrix | 4,70 | 9,57 | 8,65 |
| Adam19 | RT-PCR | 2,72 | 2,58 | 2,07 |
| | Affymetrix | 3,17 | 3,33 | 3,14 |
| Bmp6 | RT-PCR | -9,27 | -28,44 | -32,45 |
| | Affymetrix | -15,47 | -34,84 | -22,81 |
| Acvrl1 | RT-PCR | -3,65 | -7,11 | -8,34 |
| | Affymetrix | -5,89 | -6,73 | -7,59 |
| Igfbp5 | RT-PCR | -2,90 | -1,92 | -2,41 |
| | Affymetrix | -6,51 | -1,94 | -4,85 |
| Sema3f | RT-PCR | -2,11 | -2,33 | -2,90 |
| | Affymetrix | -4,90 | -5,40 | -5,60 |
| Cav | RT-PCR | -1,90 | -2,96 | -2,76 |
| | Affymetrix | -4,04 | -5,64 | -6,54 |
| Cdh5 | RT-PCR | -2,74 | -3,80 | -3,80 |
| | Affymetrix | -7,18 | -9,90 | -9,95 |

**Table8. Similar and different gene expression changes in c-Myc - induced bronchioalveolar carcinoma (BAC) and papillary lung adenocarcinoma (PLAC).**

| Representative**Gene Title** | | **Gene Symbol** | **BAC, F** | **BAC, M** | BAC, F | BAC, M | BAC, F | BAC, M | **PLAC** | PLAC | PLAC |
|---|---|---|---|---|---|---|---|---|---|---|---|
| Public ID | | | **FC** | **FC** | P,I/D (%) | P, I/D (%) | T-test | T-test | **FC** | P, I/D (%) | T-test |
| | **1. Genes induced/repressed in BAC and PLAC** | | | | | | | | | | |
| | **1a. stronger induced/repressed in PLAC than in BAC** | | | | | | | | | | |
| BC006728 | **myelocytomatosis oncogene** | **Myc** | **5,02** | **3,18** | 100 | 100 | Up | Up | **42,23** | 100 | Up |
| NM_007629 | **cyclin B1, related sequence 1 /// cyclin B1** | **Ccnb1** | **2,82** | **3,05** | 100 | 88 | Up | Up | **14,27** | 85 | Up |
| NM_007659 | **cell division cycle 2 homolog A (S. pombe)** | **Cdc2a** | **2,97** | **2,96** | 100 | 100 | Up | Up | **12,79** | 60 | Up |
| NM_007900 | **ect2 oncogene** | **Ect2** | **3,53** | **2,19** | 100 | 94 | Up | Up | 6,25 | 97 | Up |
| AF237702 | **serine hydroxymethyl transferase 1 (soluble)** | **Shmt1** | **2,84** | **1,79** | 100 | 50 | Up | Up | **7,98** | 100 | Up |
| AK011784 | **insulin-like growth factor binding protein 2** | **lgfbp2** | **-2,72** | **-2,08** | 100 | 100 | Down | Down | **-12,03** | 100 | Down |
| X82786 | **antigen identified by monoclonal antibody Ki67** | **Mki67** | **1,83** | **2,1** | 94 | 100 | Up | Up | **13,14** | 80 | Up |
| AW061324 | **cell division cycle 20 homolog (S. cerevisiae)** | **Cdc20** | **2,12** | **1,82** | 100 | 69 | Up | Up | **5,87** | 95 | Up |
| X66032 | **cyclin B2** | **Ccnb2** | **1,71** | **1,58** | 100 | 75 | Up | Up | **5,56** | 75 | Up |
| U80932 | **serine/threonine kinase 6** | **Aurka** | **1,69** | **1,52** | 75 | 75 | Up | Up | **4,52** | 80 | Up |
| AB013819 | **baculoviral IAP repeat-containing 5** | **Birc5** | **1,62** | **1,85** | 88 | 94 | Up | Up | **6,18** | 70 | Up |
| AA681998 | **CDC28 protein kinase regulatory subunit 2** | **Cks2** | **1,77** | **1,23** | 94 | 63 | Up | None | **7,15** | 100 | Up |
| L23755 | **solute carrier family 19 (sodium/hydrogen exchanger), member 1** | **SIc19a1** | **1,56** | **1,27** | 100 | 50 | Up | None | **9,07** | 90 | Up |
| U01915 | **topoisomerase (DNA) II alpha** | **Top2a** | **2,24** | **2,21** | 100 | 100 | Up | Up | **7,24** | 60 | Up |
| AW122030 | **phosphoserine aminotransferase 1** | **Psat1** | **1,7** | **1,34** | 100 | 63 | Up | None | **5,95** | 100 | Up |

| | **1b. equally or stronger induced in BAC than in PLAC** | | | | | | | | | | |
|---|---|---|---|---|---|---|---|---|---|---|---|
| NM_008591 | **met proto-oncogene** | **Met** | **11,99** | **5,82** | 100 | 75 | Up | None | **6,16** | 60 | Up |
| NM_010207 | **fibroblast growth factor receptor 2** | **Fgfr2** | **2,75** | **2,16** | 100 | 75 | Up | None | **1,79** | 90 | Up |
| X66083 | **CD44 antigen** | **Cd44** | **3,6** | **2,14** | 100 | 75 | Up | None | 3,28 | 85 | Up |
| BI788645 | **syndecan 1** | **Sdc1** | **3,63** | **2,2** | 100 | 75 | Up | None | 2,79 | 100 | Up |
| NM_018874 | **pancreatic lipase related protein 1** | **Pnliprp1** | **8,4** | **5,9** | 100 | 88 | Up | Up | **4,36** | 42 | Up |
| B1665568 | **SMC4 structural maintenance of chromosomes 4-like 1 (yeast)** | **Smc4I1** | **3,34** | **3,11** | 100 | 81 | Up | Up | **1,75** | 72 | Up |
| BG067031 | **NMDA receptor-regulated gene 1** | **Narg1** | **2,68** | **1,89** | 100 | 75 | Up | Up | **1,89** | 77 | Up |
| BG069505 | **solute carrier family 12, member 2** | **SIc12a2** | **3,25** | **2,67** | 100 | 81 | Up | Up | **1,69** | 92 | Up |
| BB269715 | **hypoxia inducible factor 1, alpha subunit** | **Hif1a** | **5,11** | **5,39** | 88 | 63 | Up | None | **1,51** | 70 | Up |
| NM_012010 | **eukaryotic translation initiation factor 2, subunit 3, structural gene X-linked** | **Eif2s3x** | **4,71** | **3,22** | 100 | 75 | Up | None | **1,67** | 72 | Up |
| AI314055 | **trans-golgi network protein** | **Tgoln1** | **4,49** | **4,17** | 100 | 75 | Up | None | **1,84** | 87 | Up |
| X66091 | **splicing factor, arginine/serine-rich 1 (ASF/SF2)** | **Sfrs1** | **3,53** | **2,47** | 100 | 75 | Up | None | **1,59** | 67 | Up |
| BF136532 | **Protein phosphatase 2, regulatory subunit B (B56), gamma isoform** | **Ppp2r5c** | **3,31** | **3,01** | 100 | 75 | Up | None | **1,87** | 82 | Up |
| BB758819 | **ribonucleotide reductase M1** | **Rrm1** | **3,47** | **1,91** | 100 | 75 | Up | None | **2,95** | 50 | Up |
| M63801 | **gap junction membrane channel protein alpha 1** | **Gja1** | **4,09** | **2,9** | 100 | 75 | Up | None | **2,24** | 92 | Up |
| BG073415 | **neural precursor cell expressed,** | **Nedd4** | **3,7** | **2,76** | 100 | 81 | Up | None | **1,7** | 87 | Up |
| | **developmentally down-regulted gene 4** | | | | | | | | | | |
| BC010204 | **forty-two-three domain containing 1** | **Fyttd1** | **2,57** | **1,79** | 100 | 75 | Up | None | **2,06** | 87 | Up |
| AV045658 | **ring finger protein 4** | **Rnf4** | **3,42** | **2,65** | 100 | 75 | Up | None | **2,16** | 90 | Up |

| | **1c. increased in BAC** | | | | | | | | | | |
|---|---|---|---|---|---|---|---|---|---|---|---|
| AF124142 | **thymoma viral proto-oncogene 3** | **Akt3** | **2,97** | **1,88** | 94 | 69 | Up | None | **-'** | -' | -' |
| NM_009665 | **S-adenosylmethionine decarboxylase 1 *lll* S-adenosylmethionine decarboxylase 2** | **Amd1 *lll* Amd2** | **3,13** | **2,18** | 100 | 81 | Up | None | **-'** | -' | -' |
| BI410363 | **adducin 3 (gamma)** | **Add3** | **3,78** | **2,6** | 100 | 88 | Up | Up | **-'** | -' | -' |
| BG069699 | **dCMP deaminase** | **Dctd** | **3,85** | **2,31** | 94 | 81 | Up | Up | **-'** | -' | -' |
| BE915516 | **ELL associated factor 1** | **Eaf1** | **4,48** | **2,55** | 100 | 69 | Up | None | **-'** | -' | -' |
| NM_023209 | **PDZ binding kinase** | **Pbk** | **3,39** | **3,15** | 100 | 88 | Up | Up | **-'** | -' | -' |
| AF335583 | **platelet-derived growth factor, D polypeptide** | **Pdgfd** | **2,74** | **1,91** | 100 | 75 | Up | None | **-'** | -' | -' |
| BE629162 | **transmembrane protein 23** | **Tmem23** | **2,74** | **1,74** | 94 | 69 | Up | None | **-'** | -' | -' |
| AU045529 | **budding uninhibited by benzimidazoles 1 homolog, beta (S. cerevisiae)** | **Bub1b** | **2,82** | **1,81** | 94 | 25 | Up | None | **-'** | -' | -' |
| BE533039 | **stress 70 protein chaperone, microsome-associated, human homolog** | **Stch** | **3,02** | **2,28** | 100 | 75 | Up | None | **-'** | -' | -' |
| BB749708 | **tousled-like kinase 1** | **Tlk1** | **2,95** | **2,28** | 100 | 69 | Up | None | **-'** | -' | -' |
| NM_011803 | **Kruppel-like factor 6** | **Klf6** | **3,55** | **2,63** | 100 | 81 | Up | Up | **-'** | -' | -' |

| | **2. Genes with different regulation in BAC and PLAC** | | | | | | | | | | |
|---|---|---|---|---|---|---|---|---|---|---|---|
| | **2.1 significantly induced in BAC and not regulated/repressed in PLAC** | | | | | | | | | | |
| NM_009163 | **sphingosine phosphate lyase 1** | **Sgpl1** | **6,59** | **4,6** | 100 | 75 | Up | None | **-1,21** | 37 | None |
| NM_021356 | **growth factor receptor bound protein 2-associated protein 1** | **Gab1** | **2,71** | **1,72** | 94 | 75 | Up | None | **-1,16** | 42 | None |
| BI662324 | **guanine nucleotide binding protein, alpha 13** | **Gna13** | **3,35** | **2,67** | 100 | 88 | Up | Up | **-1,3** | 60 | Down |
| BF302166 | **guanine nucleotide binding protein, alpha 12** | **Gna12** | **2,78** | **1,85** | 94 | 75 | Up | None | **-1,78** | 100 | Down |
| M81483 | **protein phosphatase 3, catalytic subunit, beta isoform** | **Ppp3cb** | **3** | **1,81** | 100 | 81 | Up | Up | **-1,24** | 0 | None |
| C76813 | **a disintegrin and metalloproteinase domain 17** | **Adam17** | **2,74** | **2,18** | 100 | 75 | Up | None | **1,34** | 0 | None |
| NM_009535 | **Yamaguchi sarcoma viral (v-yes) oncogene homolog 1** | **Yes1** | **5,28** | **2,68** | 100 | 75 | Up | None | **-1,1** | 0 | None |
| BI134907 | **catenin (cadherin associated protein), 1, 88kDa** | **betaCtnnb1** | **2,51** | **1,85** | 100 | 81 | Up | None | **1,29** | 37 | Up |
| U36502 | **signal transducer and activator of transcription 5A** | **Stat5a** | **4,32** | **2,32** | 100 | 56 | Up | None | **1,32** | 5 | Up |
| M33324 | **growth hormone receptor** | **Ghr** | **2,62** | **2,43** | 100 | 75 | Up | None | **-2,46** | 100 | Down |
| BE981338 | **GPI-anchored membrane protein 1** | **Gpiap1** | **5,19** | **4,22** | 100 | 75 | Up | None | **1,29** | 57 | Up |
| BB810450 | **transferrin receptor** | **Tfrc** | **5,89** | **5,7** | 100 | 75 | Up | None | **1,18** | 12 | None |
| AF173681 | **thioredoxin interacting protein** | **Txnip** | **2,91** | **2,2** | 100 | 81 | Up | None | **1,16** | 32 | None |
| BM213828 | **karyopherin (importin) alpha 3** | **Kpna3** | **2,72** | **2,32** | 100 | 75 | Up | None | **1,13** | 32 | None |
| AW544889 | **karyopherin (importin) beta 1** | **Kpnb1** | **3,22** | **2,78** | 100 | 75 | Up | None | **1,24** | 32 | Up |
| BG066916 | **ethanolamine kinase 1** | **Etnk1** | **5,88** | **4,68** | 100 | 81 | Up | None | **1** | 12 | None |
| AV118744 | **apoptosis inhibitor 5** | **Api5** | **3,77** | **2,75** | 100 | 75 | Up | None | **1,08** | 7 | None |
| BF134200 | **Baculoviral IAP repeat-containing 4** | **Birc4** | **3,58** | **2,79** | 100 | 75 | Up | Up | **-1,34** | 5 | None |
| NM_008173 | **nuclear receptor subfamily 3, group C, member 1** | **Nr3c1** | **7,29** | **4,93** | 100 | 75 | Up | Up | **-1,86** | 85 | Down |
| NM_018824 | **solute carrier family 23 (nucleobase transporters), member 2** | **Slc23a2** | **4,09** | **2,61** | 94 | 56 | Up | None | **-1,95** | 90 | Down |
| NM_026228 | **solute carrier family 39 (metal ion transporter), member 8** | **Slc39a8** | **4,36** | **2,51** | 100 | 75 | Up | None | **-4,1** | 100 | Down |
| NM_007646 | **CD38 antigen** | **Cd38** | **3,29** | **2,39** | 100 | 81 | Up | Up | **-3,68** | 90 | Down |
| BB623587 | **integrin alpha 8** | **Itga8** | **3,31** | **2,58** | 100 | 75 | Up | None | **-9,72** | 100 | Down |
| X83506 | **utrophin** | **Utrn** | **4,11** | **2,77** | 100 | 75 | Up | None | **-2,86** | 100 | Down |
| NM_009502 | **vinculin** | **Vcl** | **6,14** | **3,88** | 100 | 75 | Up | None | **-2,4** | 100 | Down |
| NM_009640 | **angiopoietin 1** | **Angpt1** | **13,73** | **6,03** | 100 | 75 | Up | None | **-4,65** | 100 | Down |
| AB015595 | **calcitonin receptor-like** | **Calcrl** | **6,08** | **3,78** | 100 | 75 | Up | None | **-26,4** | 100 | Down |
| NM_010101 | **endothelial differentiation, sphingolipid G-protein-coupled receptor, 3** | **Edg3** | **10,76** | **3,48** | 100 | 75 | Up | None | **-1,99** | 72 | Down |
| AF039601 | **transforming growth factor, beta receptor III** | **Tgfbr3** | **4,32** | **3,03** | 94 | 63 | Up | None | -3,93 | 92 | Down |
| NM_008056 | **frizzled homolog 6 (Drosophila)** | **Fzd6** | **4,39** | **2,95** | 100 | 81 | Up | Up | **1,42** | 0 | None |
| BE995678 | **tumor rejection antigen gp96** | **Tra1** | **2,51** | **1,63** | 100 | 75 | Up | None | 1,4 | 52 | Up |
| AK003821 | **anaphase-promoting complex subunit 5** | **Anapc5** | **2,66** | **2,03** | 100 | 81 | Up | Up | **1,37** | 65 | Up |
| NM_011926 | **CEA-related cell adhesion molecule 1 //*l* CEA-related cell adhesion molecule 2** | **Ceacam1 /// Ceacam2** | **6,06** | **3,64** | 88 | 75 | Up | None | **1,23** | 40 | None |
| AK021111 | **serine/threonine kinase 3 (Ste20, yeast homolog)** | **Stk3** | **3,29** | **2,1** | 100 | 81 | Up | Up | **-1,32** | 17 | None |
| AA124924 | **RAS p21 protein activator 1** | **Rasa1** | **4,57** | **2,5** | 100 | 75 | Up | Up | **-1,03** | 12 | None |
| AB015978 | **oncostatin M receptor** | **Osmr** | **3,69** | **1,65** | 100 | 69 | Up | Up | **1,04** | 30 | None |
| M93954 | **tissue inhibitor of metalloproteinase 2** | **Timp2** | **4,55** | **3,34** | 100 | 81 | Up | None | **-2,84** | 100 | Down |
| AB018194 | **protein tyrosine phosphatase, non-receptor type substrate 1** | **Ptpns1** | **2,61** | **1,56** | 94 | 75 | Up | None | **-3,19** | 92 | Down |
| BB400304 | **toll interacting protein** | **Tollip** | **3,74** | **2,69** | 100 | 75 | Up | None | **-1,32** | 62 | Down |
| NM_013657 | **sema domain, immunoglobulin domain** | **Sema3c** | **2,72** | **2,68** | 100 | 75 | Up | None | **-3,74** | 95 | Down |
| | **(Ig), short basic domain, secreted, (semaphorin) 3C** | | | | | | | | | | |

| | **2.2 significantly induced/ repressed in PLAC, not in BAC** | | | | | | | | | | |
|---|---|---|---|---|---|---|---|---|---|---|---|
| AB025409 | **CDC28 protein kinase 1** | **Cks1/Cks1b** | **1,3** | **1,28** | 63 | 44 | None | Up | **7,48** | 100 | Up |
| M33934 | **inosine 5'-phosphate dehydrogenase 2** | **Impdh2** | **1,32** | **1,13** | 75 | 31 | Up | None | **4,09** | 100 | Up |
| AI131895 | **kinesin family member 22** | **Kif22** | **2,41** | **2,12** | 13 | 25 | Up | Up | **5,99** | 87 | Up |
| Al846534 | **NIMA (never in mitosis gene a)-related expressed kinase 6** | **Nek6** | **1,22** | **-1,02** | 6 | 0 | Up | None | **3,25** | 100 | Up |
| AI850362 | **uridine-cytidine kinase 2** | **Uck2** | **1,01** | **-1,36** | 0 | 6 | None | None | **3,78** | 100 | Up |
| AI042655 | **hepatocyte growth factor activator** | **Hgfac** | **-1,07** | **-1,24** | 0 | 6 | None | None | **7,83** | 100 | Up |
| Z67748 | **spermidine synthase** | **Srm** | **1,2** | **-1,06** | 31 | 6 | Up | None | **4,33** | 95 | Up |
| Y11666 | **hexokinase 2** | **Hk2** | **-1,06** | **-1,3** | 13 | 38 | None | Down | **3,47** | 100 | Up |
| L41352 | **amphiregulin** | **Areg** | **-1,83** | **-1,44** | 63 | 63 | None | None | **7,1** | 90 | Up |
| AA726223 | **a disintegrin and metalloproteinase domain 19 (meltrin beta)** | **Adam19** | **1,14** | **-1,11** | 6 | 6 | None | None | **3,23** | 100 | Up |
| AW121294 | **ras homolog gene family, member U** | **Arhu/Rhou** | **-1,07** | **-1,06** | 6 | 13 | None | None | **4** | 67 | Up |
| U51805 | **arginase 1, liver** | **Arg1** | **1,54** | **1,84** | 6 | 6 | None | None | **10,58** | 97 | Up |
| U15443 | **Ros1 proto-oncogene** | **Ros1** | **-1,73** | **1,23** | 0 | 6 | None | None | **14,64** | 90 | Up |
| M17516 | **lactate dehydrogenase 1, A chain** | **Ldh1** | **1,1** | **1,02** | 6 | 0 | None | None | **3,48** | 100 | Up |
| AF030065 | **hepsin** | **Hpn** | **-1,07** | **-1,07** | 0 | 6 | None | None | **4,96** | 100 | Up |
| AV109962 | **Tnf receptor associated factor 4** | **Traf4** | **-1,18** | **1,19** | 0 | 0 | None | None | **4,64** | 100 | Up |
| AI786089 | **kininogen** | **Kng1** | **Absent** | **Absent** | 0 | 0 | | | **25,34** | 100 | Up |
| AI853173 | **RNA polymerase 1-3** | **Rpo1-3** | **1,04** | **-1,04** | 0 | 0 | None | None | **2,94** | 92 | Up |
| X13135 | **fatty acid synthase** | **Fasn** | **1,18** | **-1,02** | 50 | 0 | Up | None | **3,02** | 100 | Up |
| AW121447 | **nucleolar protein 5A** | **Nol5a** | **1,25** | **1,13** | 25 | 38 | None | None | **4,66** | 92 | Up |
| AW047185 | **thimet oligopeptidase 1** | **Thop1** | **1,1** | **-1,06** | 13 | 0 | None | None | **3,58** | 87 | Up |
| X81627 | **lipocalin 2** | **Lcn2** | **-1,41** | **-1,45** | 56 | 56 | None | None | **5,76** | 100 | Up |
| X65128 | **growth arrest specific 1** | **Gas1** | **1,23** | **1,26** | 0 | 0 | Up | None | **-6,15** | 100 | Down |
| X61452 | **septin 4** | **Sept4** | **-1,34** | **-1,14** | 75 | 19 | Down | None | **-3,02** | 100 | Down |
| AI850533 | **bone morphogenetic protein 6** | **Bmp6** | **-1,18** | **-1,1** | 19 | 13 | None | None | **-22,71** | 100 | Down |
| AF035207 | **BCL2/adenovirus E1B 19kDa-interacting protein 1, NIP2** | **Bnip2** | **-1,16** | **-1,01** | 38 | 0 | None | None | **-2,9** | 100 | Down |
| AB020886 | **A kinase (PRKA) anchor protein (gravin) 12** | **Akap12** | **-1,19** | **1,01** | 0 | 0 | None | None | **-8,23** | 100 | Down |
| Z31664 | **activin A receptor, type II-like 1** | **Acvrl1** | **-1,05** | **-1,09** | 13 | 19 | None | None | **-5,54** | 100 | Down |
| AI747654 | **caveolin, caveolae protein** | **Cav/Cav1** | **-1,08** | **-1,02** | 0 | 0 | None | None | **-5,24** | 100 | Down |
| D38117 | **calpain 2** | **Capn2** | **-1,02** | **-1,11** | 0 | 6 | None | None | **-3,21** | 100 | Down |
| X67083 | **DNA-damage inducible transcript 3** | **Ddit3** | **-1,66** | **-1,21** | 88 | 44 | Down | Down | **-3,38** | 100 | Down |
| AJ243895 | **hairy/enhancer-of-split related with YRPW motif 1** | **Hey1** | **-1,09** | **-1,11** | 6 | 0 | None | Down | **-4,35** | 97 | Down |
| X81584 | **insulin-like growth factor binding protein** | **6 Igfbp6** | **-1,15** | **-1,16** | 13 | 13 | Down | None | **-10,5** | 100 | Down |
| AI849416 | **large tumor suppressor 2** | **Lats2** | **1,8** | **2,08** | 0 | 0 | None | None | **-3,77** | 100 | Down |
| D10837 | **lysyl oxidase** | **Lox** | **2,04** | **1,24** | 0 | 0 | Up | None | **-3,15** | 95 | Down |
| AI844911 | **protein tyrosine phosphatase, receptor type, D** | **Ptprd** | **-1,18** | **1,09** | 25 | 6 | None | None | **-5,64** | 100 | Down |
| AF080090 | **sema domain, immunoglobulin domain (Ig), short basic domain, secreted, (semaphorin) 3 F** | **Sema3f** | **-1,19** | **-1,26** | 44 | 63 | None | None | **-5,3** | 100 | Down |
| AF010254 | **serine (or cysteine) proteinase inhibitor, clade G, member 1** | **Serping1** | **-1,06** | **-1,1** | 0 | 6 | None | None | **-5,02** | 100 | Down |

| | | | | | | | | | | | |
|---|---|---|---|---|---|---|---|---|---|---|---|
| FC (fold change) - the ratio between mean gene expression levels in the c-Myc transgenic lungs and control non-transgenic animals P,I/D (%) - number (%) of "Present" and "Increase" / "Decrease" calls in the pairwise comparisons, each tumor sample versus each control lung sample, by which genes were up or down regulated respectively T-test - the significance of gene expression changes acoordingly to T-test (p-value cut-off 0.05) M- males, F- females For analysis of gene expression changes in BAC 4 RNA pools from c-Myc -transgenic lungs were compared with 4 RNA pools from control lungs, each pool combined RNA isolated from 4 different mice, for each gender separately.. For analysis of gene expression changes in PLAC 10 tumor nodes from different mice were compared with 4 lungs of control non-transgenic animals. | | | | | | | | | | | |

## Claims

1. Use of at least one biomarker selected from the group consisting of Satb1, Hspa9a, Hey1, Gas1, Bnip2,Capn2,Anp32a, Ddit3,Ccnb2, Cdkn2d (p19), Prc1, Uck2, Srm, Shmt1, Slc19a1, Npm1, Npm3, Nol5, Lamr1/Rpsa, Arhu(Rhou), Traf4, Adam19, Bmp6, Rbp1, Reck, Ect2
in the diagnosis, prognosis and/or treatment monitoring of cancer, in particular of lung cancer.

2. Use as claimed in claim 1 for monitoring the therapeutic treatment of a patient suffering from lung cancer, in particular the treatment with irinotecan, paclitaxel and/or 5-fluorouracil and/or the treatment with a drug binding to the epithelial cell adhesion molecule (EpCAM), such as an anti EpCAM antibody may be.

3. Use as claimed in one of the claims 1-2, wherein the at least one biomarker is selected from the group consisting of Ccnb2, Slc19a1, Uck2, Srm1, Nol5a, Arhu, Adam19, Ect2, Shmt1
or from the group consisting of Gas1, Bmp6, Bnip2, Capn2, Ddit3 , Hey1.

4. Use as claimed in claim 3 for the diagnosis, prognosis and/or treatment monitoring of PLAC.

5. Use as claimed in one of the claims 1-4, wherein at least one biomarker selected from the group consisting of Ccnb2, SIc19a1, Uck2, Srm1, Nol5a, Arhu, Adam19, Ect2, Shmt1 and at least one biomarker selected from the group consisiting of Gas1, Bmp6, Bnip2, Capn2, Ddit3 , Hey1 are used.

6. A method for diagnosing, prognosing and/or staging cancer and/or monitoring the treatment of cancer, in particular lung cancer, comprising
(a) measuring the level of at least one biomarker selected from the group consisting of Satb1, Hspa9a, Hey1,Gas1, Bnip2,Capn2,Anp32a, Ddit3,Ccnb2, Cdkn2d (p19), Prc1, Uck2, Srm, Shmt1, Slc19a1, Npm1, Npm3, Nol5, Lamr1/Rpsa, Arhu(Rhou), Traf4, Adam19, Bmp6, Rbp1, Reck, Ect2 in a patient or in a sample of a patient suffering from or being susceptible to cancer, and
(b) comparing the level of said at least one biomarker in said patient or in said sample to a reference level of said at least one biomarker, in particular by the use according to one of the claims 1-5.

7. Method as claimed in claim 6 for monitoring the therapeutic treatment of a patient suffering from lung cancer, in particular the treatment with irinotecan, paclitaxel and/or 5-fluorouracil and/or the treatment with a drug binding to the epithelial cell adhesion molecule (EpCAM), such as an anti EpCAM antibody may be.

8. Method as claimed in one of the claims 6-7, wherein the at least one biomarker is selected from the group consisting of Ccnb2, SIc19a1, Uck2, Srm1, Nol5a, Arhu, Adam19, Ect2, Shmt1 or from the group consisting of Gas1, Bmp6, Bnip2, Capn2, Ddit3 , Hey1.

9. Method as claimed in claim 8 for the diagnosis, prognosis, staging and/or treatment monitoring of PLAC.

10. Method as claimed in one of the claims 8-9 to distinguish between different subtypes of lung cancer, such as (but not limited to) lung adenocarcinomas as defined by BAC or PLAC, wherein a significantly increased level of Ccnb2, Slc19a1, Uck2, Srm1, Nol5a, Arhu, Adam19, Ect2, Shmt1 in comparison with the level of a normal individual or a significantly decreased level of Gas1, Bmp6, Bnip2, Capn2, Ddit3 , Hey1 in comparison with the level of a normal individual is indicative of PLAC.

11. Method as claimed in one of the claims 6-7 to distinguish between different subtypes of lung cancer, such as (but not limited to) lung adenocarcinomas as defined by BAC or PLAC, wherein at least one biomarker selected from the group consisting of Ccnb2, SIc19a1, Uck2, Srm1, Nol5a, Arhu, Adam19, Ect2, Shmt1 and at least one biomarker selected from the group consisting of Gas1, Bmp6, Bnip2, Capn2, Ddit3 , Hey1 are measured.

12. Method as claimed in one of the claims 10-11, wherein a significantly increased level of Ccnb2, SIc19a1, Uck2, Srm1, Nol5a, Arhu, Adam19, Ect2, Shmt1 in comparison with the level of a normal individual and a significantly decreased level of Gas1, Bmp6, Bnip2, Capn2, Ddit3 , Hey1 in comparison with the level of a normal individual is indicative of PLAC.

13. Medicament for the treatment of lung carcinoma comprising a composition that decreases the expression or activity of a c-myc modulated gene selected from the group consisting of
Prc1, Klt4, Ect2, Cdc20, Stk6, Nek6, Ect2, Birc5, Hspa9a, Cideb, Pglyrp, Zfp239, Elf5, Arg1, Hk1, Gapd, Suclg2, Tpi, Gnpnat1, Pign, Uck2, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39,
Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn
and/or
increases the expression or activity of a c-myc modulated gene selected from the group consisting of
Satb1, Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1.

14. Antisense composition, wherein the antisense composition comprises a nucleotide sequence complementary to a coding sequence of a c-myc modulated gene selected from the group consisting of
Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Co115a1, Dsg2, Ect2, Lcn2, Kng, Hgfac, Adora2b, Spint1, Adam19, Hpn.

15. SiRNA composition, wherein the siRNA composition reduces the expression of a c-myc modulated gene selected from the group consisting of
Prc1, Kit4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rp144, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn.

16. Antibody composition, wherein the antibody composition comprises a pharmaceutically effective amount of an antibody or fragment thereof that binds to a polypeptide encoded by a gene selected from the group consisting of
Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn.

17. Polypeptide composition, wherein the polypeptide composition comprises a polypeptide coded by the sequence of a c-myc modulated gene selected from the group consisting of
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1.

18. Vaccine composition comprising a polypeptide coded by the sequence of a c-myc modulated gene selected from the group consisting of
Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn.

19. Nucleotide composition comprising a nucleotide sequence of a c-myc modulated gene selected from the group consisting of
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1.

20. Composition according to one of the claims 14-19, wherein the c-myc modulated gene is selected from the group of genes consisting of Prc1, Stk6, Birc5, Cideb, Zfp239, Arg1, Pign, Mre11a, Top2a, Rrm1, Npm3, Grwd1, Tomm40, Rangnrf, Ros1, Frat2, Traf4, Cldn2, Kng, Hgfac, Foxf1a, Tcf21, Gata3, Trim30, Sox17, Meis1, Foxf2, Bmp6, Dpt, Igfbp4, Sema3f, Vsnl1, Icam2, Pcdha6, Lama3, Vtn, Tna, Ndr2.

21. Composition according to one of the claims 14-20, wherein the c-myc modulated gene is selected from a first group consisting of Rbp1, Reck, Areg, Ros1, Arhu, Frat2, Traf4, Myc, Frat2, Cldn2, Gjb3, Gja1, Krt1-18, Co115a1, Dsg2, Ect2, Lcn2, Kng, Hgfac, Adora2b, Spint1, Adam19, Hpn or from a second group consisting of Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1,
or preferably is selected from a first group consisting of Ros1, Frat2, Traf4, Cldn2, Kng, Hgfac or from a second group consisting of Bmp6, Dpt, Igfbp4, Sema3f, Vsnl1, Icam2, Pcdha6, Lama3, Vtn, Tna, Ndr2,
or more preferably is selected from a first group consisting of Traf4, Kng, Hgfac or from a second group consisting of Bmp6, Dpt, Igfbp4, Sema3f, Vsnl1, Icam2, Pcdha6, Lama3, Vtn, Tna, Ndr2.

22. Composition according to one of the claims 14-21, wherein the c-myc modulated gene is a human or murine gene.

23. Medicament according to claim 13, wherein the composition is a composition as claimed in one of the claims 14-22.

24. Primer pair selected from the group of primer pairs as disclosed in the Materials and Methods section.

25. Use of a composition that decreases the expression or activity of a c-myc modulated gene selected from the group consisting of
Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn.
and/or
increases the expression or activity of a c-myc modulated gene selected from the group consisting of
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1
for the preparation of a medicament.

26. Use of an antisense composition for the preparation of a medicament, wherein the antisense composition comprises a nucleotide sequence complementary to a coding sequence of a c-myc modulated gene selected from the group consisting of Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Co115a1, Dsg2, Ect2, Lcn2, Kng, Hgfac, Adora2b, Spint1, Adam19, Hpn.

27. Use of an siRNA composition for the preparation of a medicament, wherein the siRNA composition reduces the expression of a c-myc modulated gene selected from the group consisting of
Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn.

28. Use of an antibody composition for the preparation of a medicament, wherein the antibody composition comprises a pharmaceutically effective amount of an antibody or fragment thereof that binds to a polypeptide encoded by a gene selected from the group consisting of
Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn.

29. Use of a polypeptide composition for the preparation of a vaccine, wherein the polypeptide composition comprises a polypeptide coded by the sequence of a c-myc modulated gene selected from the group consisting of
Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn.

30. Use of a nucleotide composition for the preparation of a medicament, wherein the nucleotide composition comprises a nucleotide sequence of a c-myc modulated gene selected from the group consisting of
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1.

31. Use of a polypeptide composition for the preparation of a medicament, wherein the polypeptide composition comprises a polypeptide coded by the sequence of a c-myc modulated gene selected from the group consisting of
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1.

32. Use according to one of the claims 25-31, wherein the c-myc modulated gene is selected from a first group consisiting of Prc1, Stk6, Birc5, Cideb, Zfp239, Arg1, Pign, Mre11a, Top2a, Rrm1, Npm3, Grwd1, Tomm40, Rangnrf,
Ros1, Frat2, Traf4, Cldn2, Kng, Hgfac or from a second group consisiting of Foxf1a, Tcf21, Gata3, Trim30, Sox17, Meis1, Foxf2, Bmp6, Dpt, Igfbp4, Sema3f, Vsnl1, Icam2, Pcdha6, Lama3, Vtn, Tna, Ndr2,
or preferably is selected from a first group consisiting of Cideb, Arg1, Mre11a, Top2a, Npm3, Traf4, Kng, Hgfac or from a second group consisiting of
Foxf1a, Tcf21, Gata3, Trim30, Sox17, Meis1, Foxf2, Bmp6, Dpt, Igfbp4, Sema3f, Vsnl1, Icam2, Pcdha6, Lama3, Vtn, Tna, Ndr2.

33. Use according to one of the claims 25-32, wherein the c-myc modulated gene is selected from a first group or from a second group according to claim 21.

34. Use according to one of the claims 25-33, wherein the c-myc modulated gene is a human or murine gene.

35. Use according to one of the claims 25-34 for the preparation of a medicament for preventing, treating, or ameliorating lung carcinoma.

36. Use according to one of the claims 25-35 for the preparation of a medicament for preventing, treating, or ameliorating adenocarcinoma, in particular papillary adenocarcinoma, of the lung.

37. Nucleotide composition, wherein the nucleotide composition comprises a nucleotide sequence of a c-myc modulated gene selected from the group consisting of Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rp144, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn.

38. Use of novel c-myc modulated genes and/or gene products of thereof to screen for and to identify drugs against lung carcinoma, wherein at least one or more genes and/or one or more gene products of thereof selected from the group of Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn,
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1
is used.

39. Use according to claim 38, wherein one or more genes selected from the group of Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn, Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2, Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1
and/or their gene products is incubated with a compound to be tested and changes in the expression of said genes and/or derived sequences and/or the function of said gene products are determined.

40. Use according to one of the claims 38-39, wherein a composition according to one of the claims 19 or 37, preferably including a nucleotide sequence of a gene according to claim 32 is used.

41. Use according to one of the claims 38-40 for identifying a compound that inhibits the expression of a gene selected from the group of
Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn,
comprising the steps of : (1) contacting a test cell expressing said gene with a test compound; (2) detecting the expression level of said gene; and (3) determining the compound that suppresses said expression level compared to a normal control level of said gene as an inhibitor of said gene.

42. Use according to one of the claims 38-40 for identifying a compound that enhances the expression or activity of a gene selected from the group of,
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1,
comprising the steps of : (1) contacting a test cell expressing said gene with a test compound; (2) detecting the expression level of said gene; and (3) determining the compound that increases said expression level compared to a normal control level of said gene as an enhancer of said lung adenocarcinoma associated gene.

43. Use as claimed in one of the claims 38-42, comprising the steps of : (1) contacting a test compound with a polypeptide encoded by the selected gene; (2) detecting the binding activity between the polypeptide and the test compound; and (3) selecting a compound that binds to the polypeptide.

44. Use as claimed in one of the claims 38-43, comprising the steps of : (a) contacting a test compound with a polypeptide encoded by the selected gene;
(b) detecting the biological activity of the polypeptide of step (a); and (c) selecting a compound that suppresses the biological activity of the polypeptide encoded by the gene selected from the group consisting of
Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rp144, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn.
in comparison with the biological activity detected in the absence of the test compound, and/or enhances the biological activity of the polypeptide encoded by the polynucleotide selected from the group consisting of
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1,
in comparison with the biological activity detected in the absence of the test compound.

45. Use as claimed in claim 44, wherein said biological activity is cell proliferation.

46. Use according to one of the claims 38-45 comprising the steps of : (1) contacting a test compound with a cell into which a vector comprising the transcriptional regulatory region of one or more of the selected genes and a reporter gene that is expressed under the control of the transcriptional regulatory region has been introduced,; (2) measuring the activity of said reporter gene; and (3) selecting a compound that reduces the expression level of said reporter gene when the selected gene is an up-regulated gene selected from the group consisting of
Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn,
and/or that enhances the expression level of said reporter gene when the selected gene is a down-regulated gene selected from the group consisting of
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1,
,as compared to a control.

47. Use as claimed in claims 38-46, wherein drugs regulate the expression of one or more of said genes and/or the function of one or more of said gene products and/or their derived molecules and are used for the (production of means for) treatment of lung carcinoma.

48. Use as claimed in claims 38-47, wherein DNA and/or or related molecules encoding one or more of said gene products and/or derived structures are used.

49. Use as claimed in claims 38-48, wherein one or more polypeptides, peptides and/or derived molecules having the function of one or more of said gene products, are used.

50. Procedure for identifying, labelling and treating of lung carcinoma, wherein a biological or biotechnological system is contacted with a soluble substance having affinity with at least one of the genes selected from the group of
Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Hpn, and/or mRNA encoded thereby and/or their gene products and/or parts thereof and wherein the soluble substance is linked with a marker.

51. Procedure according to claim 50, wherein the biological or biotechnological system is an organism, a tissue, a cell, a part of a cell, a DNA, a RNA, a cDNA, a mRNA, a cRNA, a protein and/or a peptide and/or a derived structure and/or contains the same and/or wherein at least one complementary oligonucleotide, in particular at least one nucleotide sequence according to claim 14 or at least one primer or at least one primer pair according to the materials and methods section is used as the substance having specific affinity.

52. Procedure according to claim 50-51, wherein the biological or biotechnological system comprises cells of a lung carcinoma and/or an oligonucleotide library is used.

53. Procedure according to claim 50-52, wherein the biological or biotechnological system is isolated or derived from a transgenic mouse, in particular a c-myc mouse as disclosed in the description.

54. Procedure, in particular according to one of the claims 50-53, for diagnozing lung cancer or a predisposition to developing lung cancer in a subject, comprising determining an expression level of a c-myc modulated gene selected from the group consisting of Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn, in a biological sample derived from the subject, wherein an increase of said level compared to a normal control level of said gene indicates that said subject suffers from or is at risk of developing lung cancer.

55. Procedure according to one of the claims 50-54, wherein said increase is at least 200% greater than said normal control level.

56. Procedure according to one of the claims 54-55, further comprising determining an expression level of a c-myc modulated gene selected from the group consisting of Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1,
in a biological sample derived from the subject, and wherein a decrease in said level compared to a normal control level indicates said subject suffers from or is at risk of developing non-small cell lung cancer.

57. Procedure according to claim 56, wherein said decrease is at least 200% lower than said normal control level.

58. Procedure according to one of the claims 54-57, wherein said procedure further comprises determining said level of a plurality of the c-myc modulated genes.

59. Procedure according to one of the claims 50-58, wherein any one method is used select from the group consisting of : (1) detecting the mRNA of the selected genes; (2) detecting the protein encoded by the selected gene; and (3) detecting the biological activity of the protein encoded by the selected gene.

60. Procedure according to one of the claims 50-59, wherein hybridization of a selected gene probe to a gene transcript of a biological sample is determined.

61. Procedure according to one of the claims 50-60, wherein a primer pair as claimed in claim 10 is used as gene probe.

62. Procedure according to one of the claims 60-61, wherein said hybridization step is carried out on a DNA array.

63. Method appropriate for the use according to one of the claims. 38-49 or for the procedure according to one of the claims 50-62 comprising the steps of
- isolating a biological sample from biological material received from an organism, in particular from a human patient, suffering from lung cancer or from an organism to be tested for its susceptibility to lung cancer, and
- determining the level of
Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rp144, Eif2b, Tomm40, Slc15a2, Slc4a7, Slc4a4, Rangnrf, Kpnb3, Ipo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn
and/or
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, Igfbp5, Igfbp6, Igfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1.
or of fragments of thereof or of a selection of thereof in the biological sample by screening the presence of said proteins or of fragments of thereof or of mRNA coding for the same.

64. Method according to claim 63, comprising the steps of
- isolating a biological sample from biological material received from a healthy organism, in particular from a human being and pairwisely comparing the gene expression profiles determined for the isolated biological samples by correlating the levels measured; and/or
- a standard is used for determining the changes of expression, in particular the level of gene expression of at least one of the genes to be screened derived from a gene expression profile for a healthy tissue of the organ, wherein the tumor is formed, is used.

65. Method according to claim 64, wherein
- the isolated biological sample is a tissue, a cell, a cellular compartment, total RNA, total protein or a body fluid; and/or
- the standard comprises values for the expression levels of the targeted genes and/or values for ß-Actin, in particular being determined parallely to the gene expression profile (2); and/or
- the levels of expression of the at least two genes coding for the factors screened are normalized with respect to the levels of expression of the respective genes in healthy tissue wherein the tumor is formed, in particular by using the standard for normalizing.

66. Method according to claim 63-65, wherein the gene expression profiling comprises the steps of
- synthesizing a cDNA library derived of the isolated RNA by RT-PCR,
- synthesizing a cRNA library, derived of the cDNA library by second strand cDNA synthesis and *in vitro* transcription of the double stranded cDNA,
- producing a RNA fragment library derived of the cRNA library by hydrolytic cleavage into RNA fragments,
- performing a hybridization assay by incubating an oligonucleotide array including spatially addressed solid phase bound oligonucleotide sequences coding for the at least two oligonucleotide sequences to be screened or for parts of thereof or for sequences being complementary of the same, with the cRNA fragment library,
- scanning the hybridization pattern of the oligonucleotide array.

67. Method according to one of the claims 63-66, wherein labelled ribonucleotides, such as biotin labelled ribonucleotides may be, are used for the *in vitro* transcription.

68. Method according to one of the claims 63-67, wherein oligonucleotide microarrays are used for performing the hybridization assays.

69. Method according to one of the claims 63-68, wherein antibodies specific for said targets are used.

70. Test kit for identifying and/or determining lung carcinoma comprising two or more detection reagents which bind to one or more genes selected from the group consisting of
Prc1, Klt4, Ect2, Cdc20, Stk6, Nek6, Ect2, Birc5, Hspa9a, Cideb Pglyrp, Zfp239, Elf5, Uck2, Smarcc1, Arg1, Hk1, Gapd, Suclg2, Tpi, Gnpnat1, Pign, Gapd, Mre11a, Top2a, Ard1, Hmgb2, Xrcc5, Rrm1, Rrm2, Smarcc1, Npm3, Nol5, Lamr1, H1fx, Lmnb1, Spnr, Npm3, Nola1, Mki67ip, Ppan, Rnac, Grwd1, Srr, Pycs, Pcbd, Mrps5, Lamr1, Mrpl12, Rpl44, Eif2b, Tomm40, Slc15a2, Slc4a7, Slc4a4, Rangnrf, Kpnb3, lpo4, Mlp, Stk39, Rbp1, Reck, Areg, Ros1, Arhu, Frat2, Traf4, Myc, Frat2, Cldn2, Gjb3, Gja1, Krt1-18, Col15a1, Dsg2, Ect2, Lcn2, Kng, Hgfac, Adora2b, Spint1, Adam19, Hpn,
Cdkn2d, Lats2, Hey1, Stat1, Bnip2, Capn2, Anp32a, Madh6, Foxf1a, Tbx3, Tcf21, Gata3, Sox2, Crap, Trim30, Klf7, Sox17, Sox18, Meis1, Foxf2,
Satb1, Anp32a,
Bmp6, Tgfb1, Dpt, Acvrl1, Eng, Zfhx1a, lgfbp5, lgfbp6, lgfbp4, Socs2, Nfkbia, Sox7, Ptpre, Ptpns1, Rassf5, Fkbp7, Sema3f, Vsnl1, Reck, Capn2, Cdh5, Spock2, Thbd, Tie1, Icam2, Tek, Nes, Vwf, Xlkd1, Sparcl1, Marcks, Tenc1, Pcdha6, Lama4, Lama3, Pcdha4, Vtn, Vcam1, Tna, Stab1, Cldn5, Pmp22, Ptprb, Ptprg, Slfn2, Ndr2, Ets1, Sipa1, Ndn, Meox2, Rbp1, Sema7a, Sema3c, Sema3e, Tagln, Ablim1,
and/or mRNA of thereof and/or polypeptides encoded thereby.
